# EUROPEAN PATENT APPLICATION

(11) **EP 2 327 770 A1**
(43) Date of publication of application: **01.06.2011**
(21) Application number: 10180259.3
(22) Date of filing: 07.11.2006
(51) Int. Cl.: C12N 9/12, C12N 15/82, A01H 5/00, A01H 5/10

(54) **Use of Group I shaggy-like kinases for increasing salt-stress resistance and yield of plants**

(30) Priority: 07.11.2005 EP 05110413; 07.11.2005 EP 05110429; 14.11.2005 US 736194 P; 17.11.2005 EP 05110900; 23.11.2005 US 739686 P; 24.11.2005 EP 05111260; 05.12.2005 US 742287 P
(62) Divisional of application: 06819308.5
(71) Applicant: CropDesign N.V., 9052 Zwijnaarde (BE)
(72) Inventor: Sanz Molinero, Ana Isabel, 9050, Gentbrugge (BE)
(74) Representative: Mistry, Meeta

(57) **Abstract**

The present invention relates generally to the field of molecular biology and concerns a method for improving plant growth characteristics relative to corresponding wild type plants. More specifically, the present invention concerns a method for improving plant growth characteristics comprising modulating expression in a plant of a Group I glycogen synthase kinase (Group I shaggy-like kinase) or a homologue thereof. The present invention also concerns plants having modulated expression of a Group I glycogen synthase kinase (Group I shaggy-like kinase) or a homologue thereof, which plants have improved growth characteristics relative to corresponding wild type plants. The invention also provides constructs useful in the methods of the invention.

## Description

The present invention relates generally to the field of molecular biology and concerns a method for improving plant growth characteristics relative to corresponding wild type plants or other control plants. More specifically, the present invention concerns a method for improving plant growth characteristics comprising modulating expression in a plant of a nucleic acid encoding a Group I glycogen synthase kinase (Group I shaggy-like kinase) or a homologue thereof. The present invention also concerns plants having modulated expression of a nucleic acid encoding a Group I shaggy-like kinase or a homologue thereof, which plants have improved growth characteristics relative to corresponding wild type or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is yield. Yield is normally defined as the measurable produce of economic value from a crop. This may be defined in terms of quantity and/or quality. Yield is directly dependent on several factors, for example, the number and size of the organs, plant architecture (for example, the number of branches), seed production leaf scenescence and more. Root development, nutrient uptake and stress tolerance and early vigour may also be important factors in determining yield. Optimizing one of the abovementioned factors may therefore contribute to increasing crop yield.

Seed yield is a particularly important trait, since the seeds of many plants are important for human and animal nutrition. Crops such as, corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another trait of particular economic interest is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

The ability to increase plant yield would have many applications in areas such as agriculture, including in the production of ornamental plants, arboriculture, horticulture and forestry. Increasing yield may also find use in the production of algae for use in bioreactors (for the biotechnological production of substances such as pharmaceuticals, antibodies or vaccines, or for the bioconversion of organic waste) and other such areas.

### Background

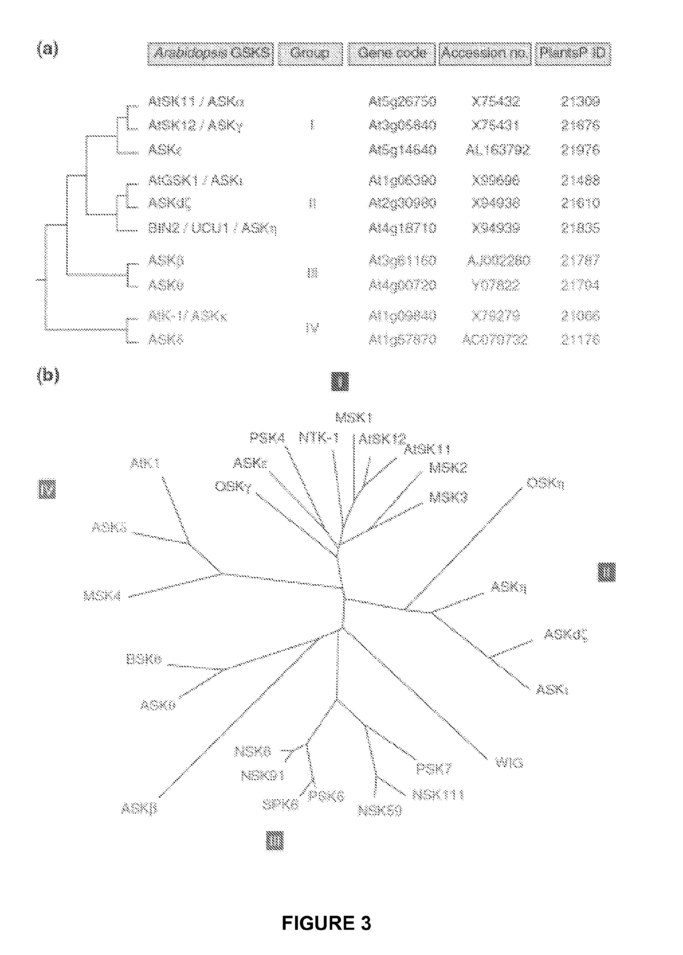

Plant shaggy-like kinases are encoded by a multigene family. The Arabidopsis genome has been found to contain ten shaggy-like kinase-encoding genes which fall into four distinct subfamilies. The protein sequences of distinct family members are highly conserved throughout the kinase domain, however the N- and C- terminal regions differ considerably indicating that the various plant shaggy-like kinases are involved in diverse biological processes, such as hormone signalling, development and stress responses. Based on protein sequence homology, the plant shaggy-like kinases can be classed into four groups (I-IV), with each of the four groups being involved in different processes (see Figure 3). In addition to the full-length cDNA sequences available for Arabidopsis thaliana shaggy-like kinases, full-length cDNA sequences are also available in public databases for shaggy-like kinases from Brassica napus, Medicago sativa, Nicotiana tabacum, oryza sativa and Petunia hybrida and Zea mays, among others. AtGSK1, a gene encoding a group II Arabidopsis shaggy-like kinase, has been reported to complement the salt sensitive phenotype of yeast calcineurin mutants. In seedlings, the production of the same shaggy-like kinase has been shown to be induced by NaCI and abscisic acid. Overproduction of the AtGSK1 gene has been reported to induce salt-stress-responsive genes and anthocyanin accumulation and to alter intracellular cation levels, which result in enhanced salt and drought tolerance.

Given that shaggy-like kinases from different groups are known to be involved in diverse biological processes, it was surprising to find shaggy-like kinases from two different groups to be involved in the same biological process, i.e. in stress responses. It was unexpected to find that a shaggy-like kinase from a group other than Group II was able to confer increased tolerance in plants to abiotic stress.

It has now been found that modulating expression in a plant of a Group I shaggy-like kinase or a homologue thereof gives plants having improved growth characteristics relative to corresponding wild type plants or other control plants.

The present invention therefore provides a method for improving plant growth characteristics comprising modulating expression in a plant of a Group I shaggy-like kinase or a homologue thereof.

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. A "control plant" as used herein refers not only to whole plants, but also to plant parts, including seeds and seed parts.

Advantageously, performance of the methods according to the present invention results in plants having improved growth characteristics, particularly one or more of increased yield, improved growth, improved biomass, improved architecture, improved cell division and improved tolerance to abiotic stress relative to corresponding wild type or other control plants.

The term "increased yield" as defined herein is taken to mean an increase in any one or more of the following, each relative to corresponding wild type or other control plants: (i) increased biomass (weight) of one or more parts of a plant, particularly aboveground (harvestable) parts, or increased root biomass, increased root volume, increased root number, increased root diameter or increased root length (of thick or thin roots), or increased biomass of any other harvestable part; (ii) increased total seed yield, which includes an increase in seed biomass (seed weight) and which may be an increase in the seed weight per plant or on an individual seed basis and/or per hectare or acre; (iii) increased number of flowers (florets) per panicle, which is expressed as a ratio of number of filled seeds over number of primary panicles; (iv) increased seed fill rate (expressed in percentage terms as the proportion of the number of filled seeds over the number of florets); (v) increased number of (filled) seeds; (vi) increased seed size, which may also influence the composition of seeds; (vii) increased seed volume, which may also influence the composition of seeds (including oil, protein and carbohydrate total content and composition); (viii) increased (individual or average) seed area; (ix) increased (individual or average) seed length; (x) increased (individual or average) seed width; (xi) increased (individual or average) seed perimeter; (xii) increased harvest index (HI), which is expressed as a ratio of the yield of harvestable parts, such as seeds, over the total biomass; and (xiii) increased thousand kernel weight (TKW), which is extrapolated from the number of filled seeds counted and their total weight. An increased TKW may result from an increased seed size and/or seed weight. An increased TKW may result from an increase in embryo size and/or endosperm size.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

Since the transgenic plants according to the present invention have increased yield, it is likely that these plants exhibit an increased growth rate (during at least part of their life cycle), relative to the growth rate of control plants at a corresponding stage in their life cycle. The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of rice plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Performance of the methods of the invention gives plants having an increased growth rate. Therefore, according to the present invention, there is provided a method for increasing the growth rate of plants which method comprises modulating expression in a plant of a Group I shaggy-like kinase or a homologue thereof.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

Performance of the methods according to the present invention results in plants having increased tolerance to abiotic stress. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. For example, drought and/or salinisation are manifested primarily as osmotic stress, resulting in the disruption of homeostasis and ion distribution in the cell. Oxidative stress, which frequently accompanies high or low temperature, salinity or drought stress may cause denaturation of functional and structural proteins. As a consequence, these diverse environmental stresses often activate similar cell signaling pathways and cellular responses, such as the production of stress proteins, up-regulation of anti-oxidants, accumulation of compatible solutes and growth arrest.

Since diverse environmental stresses activate similar pathways, the exemplification of the present invention with drought stress should not be seen as a limitation to drought stress, but more as a screen to indicate the involvement of certain polypeptides or a homologues thereof in abiotic stresses in general. Furthermore, the methods of the present invention may be performed under non-stress conditions or under conditions of mild drought to give plants having improved growth characteristics (particularly increased yield) relative to corresponding wild type or other control plants. The term "non-stress" conditions as used herein are preferably those environmental conditions that do not significantly go beyond the everyday climatic and other abiotic conditions that plants may encounter most preferably those conditions that allow optimal growth of plants. Persons skilled in the art are aware of normal soil conditions and climatic conditions for a given location.

A particularly high degree of "cross talk" is reported between drought stress and high-salinity stress (Rabbani et al. (2003) Plant Physiol 133: 1755-1767). Therefore, it would be apparent that a Group I shaggy-like kinase (as defined herein) would, along with its usefulness in conferring salt-tolerance in plants, also find use in protecting the plant against various other abiotic stresses. Furthermore, Rabbani et al. (2003, Plant Physiol 133: 1755-1767) report that similar molecular mechanisms of stress tolerance and responses exist between dicots and monocots. The methods of the invention are therefore advantageously applicable to any plant.

The term "abiotic stress" as defined herein is taken to mean any one or more of: water stress (due to drought or excess water), anaerobic stress, salt stress, temperature stress (due to hot, cold or freezing temperatures), chemical toxicity stress and oxidative stress. According to one aspect of the invention, the abiotic stress is an osmotic stress, selected from water stress, salt stress, oxidative stress and ionic stress. Preferably, the water stress is drought stress. The term salt stress is not restricted to common salt (NaCI), but may be any one or more of: NaCI, KCI, LiCI, MgCI₂, CaCl₂, amongst others.

Increased tolerance to abiotic stress is manifested by increased plant yield in abiotic stress conditions.

Performance of the methods of the invention gives plants having increased tolerance to abiotic stress. Performance of the methods of the invention gives plants grown under non-stress conditions or under mild drought conditions improved growth characteristics (particularly increased yield) relative to corresponding wild type plants or other control plants grown under comparable conditions.

The present invention also provides a method for improving abiotic stress tolerance in plants, comprising increasing activity in a plant of a Group I shaggy-like kinase or a homologue thereof, which Group I shaggy-like kinase has: (i) at least 77% sequence identity to the amino acid sequence represented by SEQ ID NO: 2; and (ii) motif I: R/H/V/N/Q E/G LK G/N and motif II: K Q/N CXXX G/A/S, where X may be any amino acid.

Preferably, where the method of the invention comprises modulating expression in a plant of a nucleic acid encoding a Group I shaggy-like kinase or a homologue thereof, the improved growth characteristic is improved tolerance to abiotic stress.

According to the another preferred feature of the present invention, there is provided a method for improving abiotic stress tolerance in plants, comprising modulating expression (preferably increasing activity and/or expression) in a plant of a Group I shaggy-like kinase or a homologue thereof, which Group I shaggy-like kinase has: (i) at least 77% sequence identity to the amino acid sequence represented by SEQ ID NO: 2; and (ii) motif I: R/H/V/N/Q E/G LK G/N and motif II: K Q/N CXXX G/A/S, where X may be any amino acid.

The methods of the invention are therefore advantageously applicable to any plant.

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising Acacia spp.,Acer spp., Actinidia spp., Aesculus spp., Agathis australis, Albizia amara, Alsophila tricolor, Andropogon spp., Arachis spp, Areca catechu, Astelia fragrans, Astragalus cicer, Baikiaea plurijuga, Betula spp., Brassica spp., Bruguiera gymnorrhiza, Burkea africana, Butea frondosa, Cadaba farinosa, Calliandra spp, Camellia sinensis, Canna indica, Capsicum spp., Cassia spp., Centroema pubescens, Chaenomeles spp., Cinnamomum cassia, Coffea arabica, Colophospermum mopane, Coronillia varia, Cotoneaster serotina, Crataegus spp., Cucumis spp., Cupressus spp., Cyathea dealbata, Cydonia oblonga, Cryptomeria japonica, Cymbopogon spp., Cynthea dealbata, Cydonia oblonga, Dalbergia monetaria, Davallia divaricata, Desmodium spp., Dicksonia squarosa, Diheteropogon amplectens, Dioclea spp, Dolichos spp., Dorycnium rectum, Echinochloa pyramidalis, Ehrartia spp., Eleusine coracana, Eragrestis spp., Erythrina spp., Eucalyptus spp., Euclea schimperi, Eulalia villosa, Fagopyrum spp., Feijoa sellowiana, Fragaria spp., Flemingia spp, Freycinetia banksii, Geranium thunbergii, Ginkgo biloba, Glycine javanica, Gliricidia spp, Gossypium hirsutum, Grevillea spp., Guibourtia coleosperma, Hedysarum spp., Hemarthia altissima, Heteropogon contortus, Hordeum vulgare, Hyparrhenia rufa, Hypericum erectum, Hyperthelia dissoluta, Indigo incarnata, Iris spp., Leptarrhena pyrolifolia, Lespediza spp., Lettuca spp., Leucaena leucocephala, Loudetia simplex, Lotonus bainesii, Lotus spp., Macrotyloma axillare, Malus spp., Manihot esculenta, Medicago sativa, Metasequoia glyptostroboides, Musa sapientum, Nicotianum spp., Onobrychis spp., Ornithopus spp., Oryza spp., Peltophorum africanum, Pennisetum spp., Persea gratissima, Petunia spp., Phaseolus spp., Phoenix canariensis, Phormium cookianum, Photinia spp., Picea glauca, Pinus spp., Pisum sativum, Podocarpus totara, Pogonarthria fleckii, Pogonarthria squarrosa, Populus spp., Prosopis cineraria, Pseudotsuga menziesii, Pterolobium stellatum, Pyrus communis, Quercus spp., Rhaphiolepsis umbellata, Rhopalostylis sapida, Rhus natalensis, Ribes grossularia, Ribes spp., Robinia pseudoacacia, Rosa spp., Rubus spp., Salix spp., Schyzachyrium sanguineum, Sciadopitys verticillata, Sequoia sempervirens, Sequoiadendron giganteum, Sorghum bicolor, Spinacia spp., Sporobolus fimbriatus, Stiburus alopecuroides, Stylosanthos humilis, Tadehagi spp, Taxodium distichum, Themeda triandra, Trifolium spp., Triticum spp., Tsuga heterophylla, Vaccinium spp., Vicia spp., Vitis vinifera, Watsonia pyramidata, Zantedeschia aethiopica, Zea mays, amaranth, artichoke, asparagus, broccoli, Brussels sprouts, cabbage, canola, carrot, cauliflower, celery, collard greens, flax, kale, lentil, oilseed rape, okra, onion, potato, rice, soybean, strawberry, sugar beet, sugar cane, sunflower, tomato, squash, tea and algae, amongst others.

According to a preferred embodiment of the present invention, the plant is a crop plant such as soybean, sunflower, canola, alfalfa, rapeseed, cotton, tomato, potato or tobacco. Further preferably, the plant is a monocotyledonous plant, such as sugarcane. More preferably the plant is a cereal, such as rice, maize, wheat, barley, millet, rye, sorghum or oats.

The term "Group I shaggy-like kinase or homologue thereof" as defined herein refers to a polypeptide having: (i) at least 77% sequence identity to the amino acid sequence represented by SEQ ID NO: 2; and (ii) motif l: R/H/V/N/Q E/G LK G/N and motif II: K Q/N CXXX G/A/S, where X may be any amino acid. A polypeptide meeting the aforementioned requirements allows a Group I shaggy-like kinase to be distinguished from those of other groups.

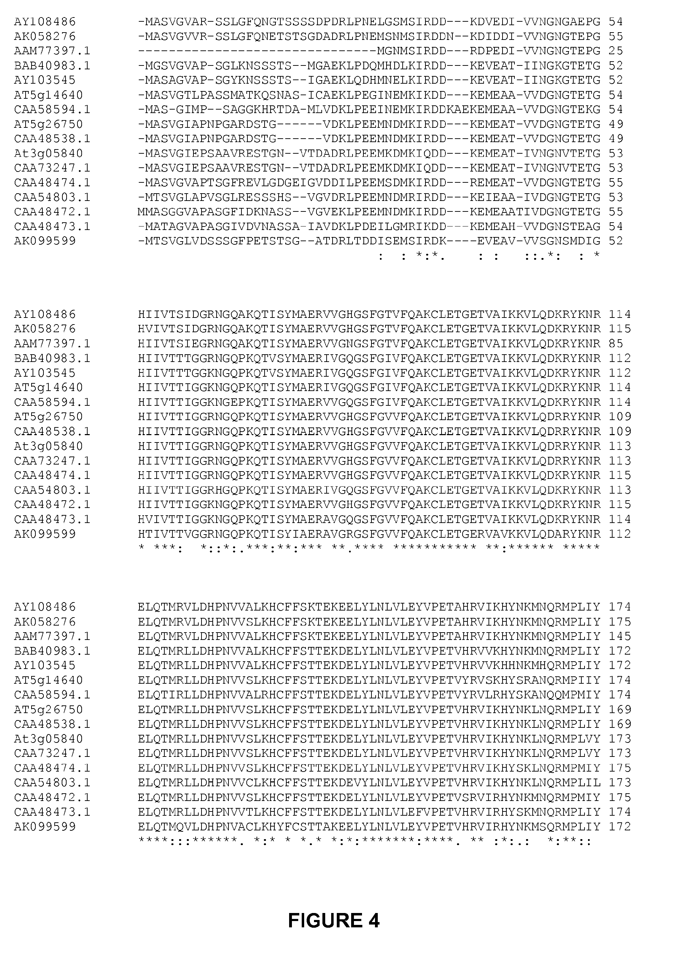

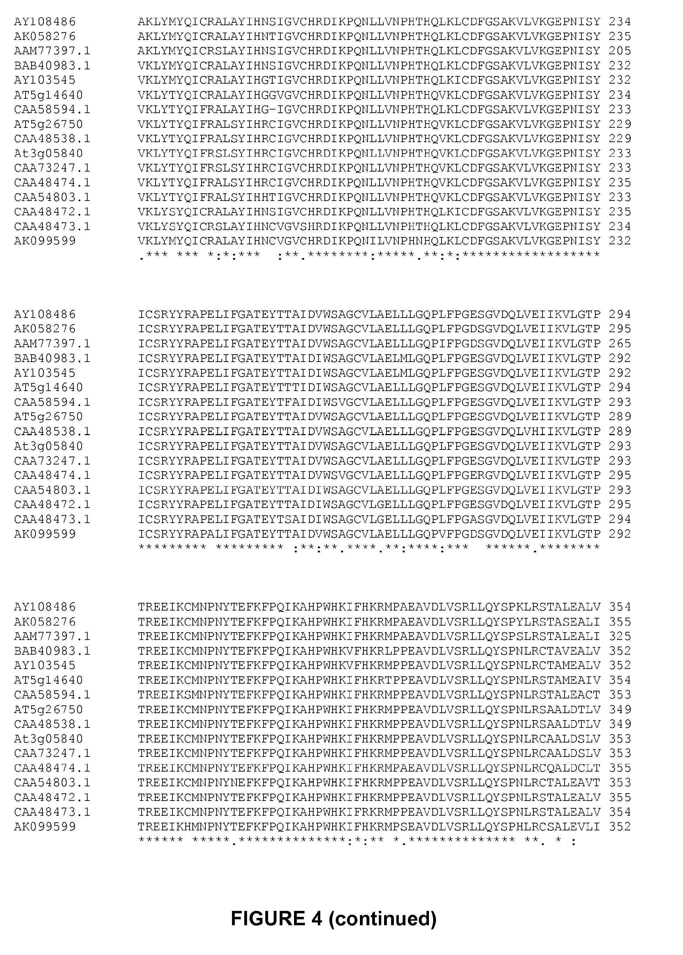

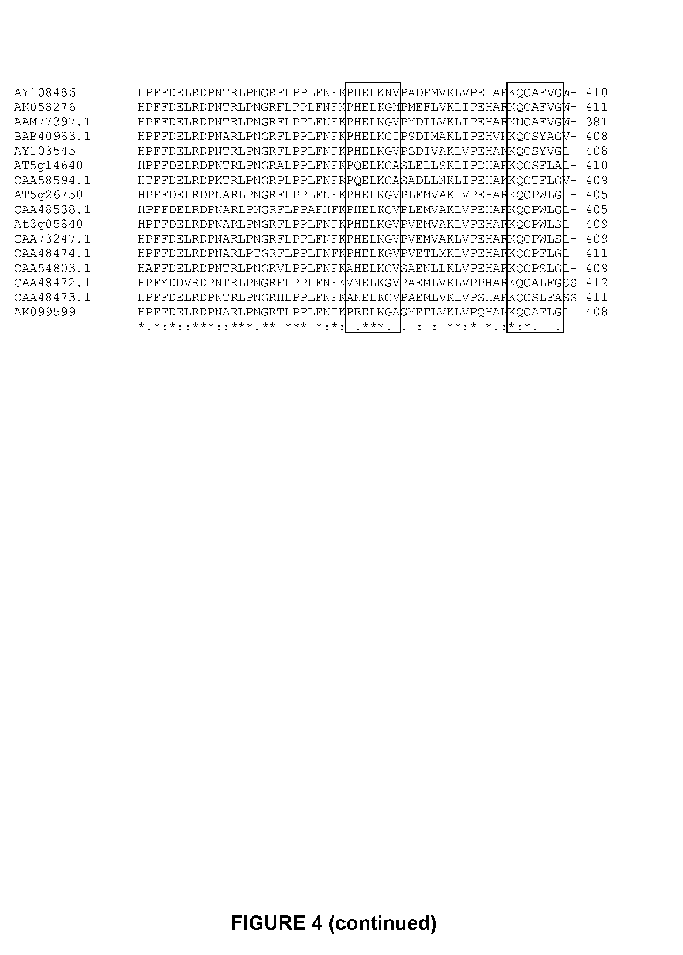

A "Group I shaggy-like kinase or a homologue thereof" falling within the above definition may readily be identified using routine techniques well known to persons skilled in the art. For example, a polypeptide having at least 77% identity to the amino acid represented by SEQ ID NO: 2 may readily be established by sequence alignment. Methods for the alignment of sequences for comparison are well known in the art, such methods include GAP, BESTFIT, BLAST, FASTA and TFASTA. GAP uses the algorithm of Needleman and Wunsch (J. Mol. Biol. 48: 443-453, 1970) to find the alignment of two complete sequences that maximises the number of matches and minimises the number of gaps. The BLAST algorithm calculates percent sequence identity and performs a statistical analysis of the similarity between the two sequences. The software for performing BLAST analysis is publicly available through the National Centre for Biotechnology Information. A shaggy-like kinase or a homologue thereof having at least 77% identity to the amino acid represented by SEQ ID NO: 2 may readily be identified by aligning a query amino acid sequence with known Group I shaggy-like kinase sequences (see for example the alignment shown in Figure 4) using, for example, the VNTI AlignX multiple alignment program, based on a modified clustal W algorithm (InforMax, Bethesda, MD, http://www.informaxinc.com), with default settings for gap opening penalty of 10 and a gap extension of 0.05.

A person skilled in the art will also readily be able to identify sequences having motif I: R/H/V/N/Q E/G LK G/N and motif ll: K Q/N CXXX G/A/S, where X may be any amino acid. This may be achieved by making an alignment and searching for homologous regions.

Table 1 below shows motif l: R/H/V/N/Q E/G LK G/N and motif ll: K Q/N CXXX G/A/S (where X may be any amino acid) as found in the sequence of SEQ ID NO: 2 and the corresponding motifs in homologous sequences. The overall percentage identity shown in Table 1 is when comparing SEQ ID NO: 2 with the accession numbers shown in the Table (full-length sequence to full-length sequence).

**Table 1: Conserved motifs found in Group I shaggy-like kinases and in homologues thereof**

| Accession number: Group I shaggy-like kinase | Gene name | Motif 1 | Motif 2 | % overall identity with SEO ID NO: 2 (EMBOSS) |
|---|---|---|---|---|
| BAB40983.1 (SEQ ID NO: 147) | Rice OSK gamma | HELKG | KQCSYAG | - |
| Y103545 | Corn | HELKG | KQCSYVG | 94.4% |
| AY108486 | Corn | HELKN | KQCAFVG | 84.4% |
| AAM77397.1 | Triticum | HELKG | KNCAFVG | 80.9% |
| CAA48538.1, At5g26750 | Ara ASK Alpha | HELKG | KQCPWLG | 85.6% |
| CAA48474.1 | Medicago MSK-1 | HELKG | KQCPFLG | 83.5% |
| CAA04265 | Ara Ask alpha | HGLKG | KQCPWLG | 84.6% |
| CAA54803.1 | Tobacco NtK1 | HELKG | KQCPSLG | 85.3% |
| CAA48472.1 | Medicago MSK3 | NELKG | KQCALFG | 85.0% |
| CAA48473.1 | Medicago MSK2 | VELKG | KQCSLFA | 82.0% |
| CAA58594.1 | Petunia Shaggy 4 | QELKG | KQCTFLG | 81.5% |
| At5g14640 | Ara ASK epsilon | QELKG | KQCSFLA | 83.9% |
| At3g05840 | Ara ASK gamma | HELKG | KQCPWLS | 84.6% |
| CAA04265 | Rice shaggy alpha | HGLKG | KQCPWLG | 84.6% |
| AK058276 | Rice shaggy | HELKG | KQCAFVG | 83.2% |
| AK099599 | Rice Shaggy | RELKG | KQCAFLG | 78.5% |

| Other shaggy-like groups | | | | |
|---|---|---|---|---|
| At4g18710 | Ara Ask eta | No | No | 70.0% |
| At2g30980 | Ara Ask dzeta | No | No | 72.9% |
| At4g00720 | Ara Ask theta | No | No | 64.1% |
| At1 g57870 | Ara Ask delta | No | No | 73.4% |
| At1 g09840 | Ara Ask kappa | No | No | 72.4% |
| At1 g06390 | Ara Ask iota | No | No | 71.6% |
| At3g61160 | Ara Ask beta | No | No | 61.3% |

Examples of polypeptides falling under the definition of a "shaggy-like kinase or a homologue thereof" include the following sequences: SEQ ID NO: 2, a gamma shaggy-like kinase from rice (NCBI Accession number AB059621); SEQ ID NO: 4 (NCBI Accession number AK058276) a shaggy-like kinase from rice; SEQ ID NO: 6 (NCBI Accession number AK099599) a shaggy like kinase from rice; SEQ ID NO: 8, an alpha shaggy-like kinase from Arabidopsis thaliana (NCBI Accession number At5g26750); SEQ ID NO: 10, a gamma shaggy-like kinase from Arabidopsis thaliana (NCBI Accession number At3g05840); SEQ ID NO: 12, an alpha shaggy-like kinase from Arabidopsis thaliana (NCBI Accession number CAA48538.1); SEQ ID NO: 14, an epsilon shaggy-like kinase from Arabidopsis thaliana (NCBI Accession number At5g14640); SEQ ID NO: 16, a gamma shaggy-like kinase from Arabidopsis thaliana (NCBI Accession number CAA73247.1); SEQ ID NO: 18 from maize (NCBI Accession number AY103545); SEQ ID NO: 20 from maize (NCBI Accession number AY108486.1); SEQ ID NO: 22 from Medicago (NCBI Accession number CAA48472.1); SEQ ID NO: 24 from Medicago (NCBI Accession number CAA48474.1); SEQ ID NO: 26 from Medicago (NCBI Accession number CAA48473.1); SEQ ID NO: 28 from tobacco (NCBI Accession number CAA54803.1); SEQ ID NO: 30 a protein prediction of a shaggy-like kinase from Triticum aestivum (NCBI Accession number AAM77397.1); SEQ ID NO: 32 from Petunia hybrida (NCBI Accession number CAA58594.1).

The "term shaggy-like kinase or a homologue thereof" is not to be limited to the sequences represented by the SEQ ID NOs mentioned in the aforementioned paragraph, but that any polypeptide meeting the criteria of having: (i) at least 77% sequence identity to the amino acid sequence represented by SEQ ID NO: 2; and (ii) motif l: R/H/V/N/Q E/G LK G/N and motif II: K Q/N CXXX G/A/S, where X may be any amino acid, would be suitable for use in the methods of the invention.

Shaggy-like kinases, as the name suggests, have kinase activity. An assay for glycogen synthase kinase-3 (GSK-3), the animal homologue, is reported in The Biochemical Journal, Vol. 303 (Pt3), Nov. 1, 1994, pp701-704 (Stambolic and Woodgett).

Sequences useful in the methods of the invention are not limited to the aforementioned Group I shaggy-like kinases and their encoding polypeptides. The methods according to the present invention may also be performed using variants of nucleic acids encoding Group I shaggy-like kinases and homologues thereof.

Examples of such variants include portions, hybridizing sequences, allelic variants, splice variants and variants obtained by gene shuffling.

A portion may be prepared, for example, by making one or more deletions to a nucleic acid. The portions may be used in isolated form or they may be fused to other coding (or non coding) sequences in order to, for example, produce a protein that combines several activities. When fused to other coding sequences, the resulting polypeptide produced upon translation may be bigger than that predicted for the portion.

Where the sequence useful in the methods of the invention in a Group I shaggy-like kinase, the portion refers to a shaggy-like kinase-encoding piece of DNA of at least 1,200 nucleotides in length, and which portion encodes a polypeptide having: (i) at least 77% sequence identity to the amino acid sequence represented by SEQ ID NO: 2; and (ii) having motif I: R/H/V/N/Q E/G LK G/N and motif ll: K Q/N CXXX G/A/S, where X may be any amino acid. Preferably, the portion is a portion of a nucleic acid as represented by any one of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29 and SEQ ID NO: 31.

The invention therefore provides a method for improving plant growth characteristics comprising modulating expression in a plant of a portion of a nucleic acid encoding a Group I shaggy-like kinase or a homologue thereof.

Another nucleic acid variant is a nucleic acid capable of hybridising under reduced stringency conditions, preferably under stringent conditions, with a nucleic acid encoding a Group I shaggy-like kinase or homologue thereof.

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids. The stringency of hybridisation is influenced by conditions such as temperature, salt concentration, ionic strength and hybridisation buffer composition.

"Stringent hybridisation conditions" and "stringent hybridisation wash conditions" in the context of nucleic acid hybridisation experiments such as Southern and Northern hybridisations are sequence dependent and are different under different environmental parameters. The skilled artisan is aware of various parameters which may be altered during hybridisation and washing and which will either maintain or change the stringency conditions.

The Tₘ is the temperature under defined ionic strength and pH, at which 50% of the target sequence hybridises to a perfectly matched probe. The Tₘ is dependent upon the solution conditions and the base composition and length of the probe. For example, longer sequences hybridise specifically at higher temperatures. The maximum rate of hybridisation is obtained from about 16°C up to 32°C below Tₘ. The presence of monovalent cations in the hybridisation solution reduce the electrostatic repulsion between the two nucleic acid strands thereby promoting hybrid formation; this effect is visible for sodium concentrations of up to 0.4M. Formamide reduces the melting temperature of DNA-DNA and DNA-RNA duplexes with 0.6 to 0.7°C for each percent formamide, and addition of 50% formamide allows hybridisaton to be performed at 30 to 45°C, though the rate of hybridisation will be lowered. Base pair mismatches reduce the hybridisation rate and the thermal stability of the duplexes. On average and for large probes, the Tₘ decreases about 1°C per % base mismatch. The Tₘ may be calculated using the following equations, depending on the types of hybrids:
1. DNA-DNA hybrids (Meinkoth and Wahl, Anal. Biochem., 138: 267-284, 1984):
   Tₘ= 81.5°C + 16.6xlog[Na⁺]^{a} + 0.41x%[G/C^{b}] - 500x[L^{c}]-¹ - 0.61x% formamide
2. DNA-RNA or RNA-RNA hybrids:
   Tₘ= 79.8 + 18.5 (log₁₀[Na⁺]^{a}) + 0.58 (%G/C^{b}) + 11.8 (%G/C^{b})² - 820/L^{c}
3. oligo-DNA or oligo-RNA^{d} hybrids:
   For <20 nucleotides: Tₘ= 2 (lₙ)
   For 20-35 nucleotides: Tₘ= 22 + 1.46 (lₙ)
   a or for other monovalent cation, but only accurate in the 0.01-0.4 M range.
   b only accurate for %GC in the 30% to 75% range.
   c L = length of duplex in base pairs.
   d Oligo, oligonucleotide; Iₙ, effective length of primer = 2x(no.of G/C)+(no. of A/T).
   Note: for each 1% formamide, the Tₘ is reduced by about 0.6 to 0.7°C, while the presence of 6 M urea reduces the Tₘ by about 30°C

Specificity of hybridisation is typically the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. Generally, suitable stringent conditions for nucleic acid hybridisation assays or gene amplification detection procedures are as set forth above. Conditions of greater or less stringency may also be selected. Generally, low stringency conditions are selected to be about 50°C lower than the thermal melting point (Tₘ) for the specific sequence at a defined ionic strength and pH. Medium stringency conditions are when the temperature is 20°C below Tₘ, and high stringency conditions are when the temperature is 10°C below Tₘ. For example, stringent conditions are those that are at least as stringent as, for example, conditions A-L; and reduced stringency conditions are at least as stringent as, for example, conditions M-R. Non-specific binding may be controlled using any one of a number of known techniques such as, for example, blocking the membrane with protein containing solutions, additions of heterologous RNA, DNA, and SDS to the hybridisation buffer, and treatment with RNase. Examples of hybridisation and wash conditions are listed in Table 2 below.

**Table 2: Examples of hybridisation and wash conditions**

| Stringency Condition | Polynucleotide Hybrid ± | Hybrid Length (bp) | Hybridisation Temperature and Buffer t | Wash Temperature and Buffert |
|---|---|---|---|---|
| A | DNA:DNA | >or equal to 50 | 65°C 1 ×SSC; or 42°C, 1 ×SSC and 50% formamide | 65°C; 0.3xSSC |
| B | DNA:DNA | <50 | Tb*; 1×SSC | Tb*; 1×SSC |
| c | DNA:RNA | >or equal to 50 | 67°C 1×SSC; or 45°C, 1×SSC and 50% formamide | 67°C; 0.3xSSC |
| D | DNA:RNA | <50 | Td*; 1×SSC | Td*; 1×SSC |
| E | RNA:RNA | >or equal to 50 | 70°C 1 ×SSC; or 50°C, 1 ×SSC and 50% formamide | 70°C; 0.3xSSC |
| F | RNA:RNA | <50 | Tf*; 1×SSC | Tf*; 1×SSC |
| G | DNA:DNA | >or equal to 50 | 65°C 4xSSC; or 45°C, 4×SSC and 50% formamide | 65°C; 1×SSC |
| H | DNA:DNA | <50 | Th*; 4×SSC | Th*; 4×SSC |
| l | DNA:RNA | >or equal to 50 | 67°C 4xSSC; or 45°C, 4×SSC and 50% formamide | 67°C; 1×SSC |
| J | DNA:RNA | <50 | Tj*; 4×SSC | Tj*; 4×SSC |
| K | RNA:RNA | >or equal to 50 | 70°C 4xSSC; or 40°C, 6×SSC and 50% formamide | 67°C; 1×SSC |
| L | RNA:RNA | <50 | TI*; 2×SSC | TI*; 2×SSC |
| M | DNA:DNA | >or equal to 50 | 50°C 4xSSC; or 40°C, 6xSSC and 50% formamide | 50°C; 2xSSC |
| N | DNA:DNA | <50 | Tn*; 6×SSC | Tn*; 6×SSC |
| O | DNA:RNA | > or equal to 50 | 55°C 4xSSC; or 42°C, 6×SSC and 50% formamide | 55°C; 2xSSC |
| P | DNA:RNA | <50 | Tp*; 6×SSC | Tp*; 6×SSC |
| Q | RNA:RNA | >or equal to 50 | 60°C 4xSSC; or 45°C, 6xSSC and 50% formamide | 60°C.; 2xSSC |
| R | RNA:RNA | <50 | Tr*; 4×SSC | Tr*; 4xSSC |

| | | | | |
|---|---|---|---|---|
| t The "hybrid length" is the anticipated length for the hybridising nucleic acid. When nucleic acids of known sequence are hybridised, the hybrid length may be determined by aligning the sequences and identifying the conserved regions described herein. t SSPE (1×SSPE is 0.15M NaCl, 10mM NaH₂PO₄, and 1.25mM EDTA, pH7.4) may be substituted for SSC (1×SSC is 0.15M NaCI and 15mM sodium citrate) in the hybridisation and wash buffers; washes are performed for 15 minutes after hybridisation is complete. The hybridisations and washes may additionally include 5 × Denhardt's reagent, 0.5-1.0% SDS, 100 µg/ml denatured, fragmented salmon sperm DNA, 0.5% sodium pyrophosphate, and up to 50% formamide. * Tb-Tr: The hybridisation temperature for hybrids anticipated to be less than 50 base pairs in length should be 5-10°C less than the melting temperature Tₘ of the hybrids; the Tₘ is determined according to the above-mentioned equations. ± The present invention also encompasses the substitution of any one, or more DNA or RNA hybrid partners with either a PNA, or a modified nucleic acid. | | | | |

For the purposes of defining the level of stringency, reference can be made to Sambrook et al. (2001) Molecular Cloning: a laboratory manual, 3rd Edition Cold Spring Harbor Laboratory Press, CSH, New York or to Current Protocols in Molecular Biology, John Wiley & Sons, N.Y. (1989).

Where the nucleic acid useful in the methods of the invention is a nucleic acid encoding a Group I shaggy-like kinase, the hybridising sequence is a nucleic acid capable of hybridising under reduced stringency conditions, preferably under stringent conditions, with a Group I shaggy-like kinase-encoding nucleic acid/gene as hereinbefore defined, which hybridising sequence encodes a polypeptide having: (i) at least 77% sequence identity to the amino acid sequence represented by SEQ ID NO: 2; and (ii) motif I: R/H/V/N/Q E/G LK G/N and motif II: K Q/N CXXX G/A/S, where X may be any amino acid. The hybridising sequence is at least 1,200 nucleotides in length. Preferably, the hybridising sequence is capable of hybridising to a nucleic acid as represented by any one of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29 and SEQ ID NO: 31.

The invention therefore provides a method for improving plant growth characteristics comprising modulating expression in a plant of a nucleic acid capable of hybridising under reduced stringency, preferably under stringent conditions to a nucleic acid encoding a Group I shaggy-like kinase or a homologue thereof.

The nucleic acids or variant thereof may be derived from any natural or artificial source. The nucleic acid/gene or variant thereof may be isolated from a microbial source, such as yeast or fungi, or from a plant, algae or animal (including human) source. This nucleic acid may be modified from its native form in composition and/or genomic environment through deliberate human manipulation. The nucleic acid is preferably of plant origin, whether from the same plant species (for example to the one in which it is to be introduced) or whether from a different plant species. The nucleic acid may be isolated from a monocotyledonous species, preferably from the family Poaceae, further preferably from Oryza genus, most preferably from Oryza sativa. The nucleic acid may be isolated from a dicotyledonous species, preferably from the family Brassicaceae, further preferably from Arabidopsis thaliana.

The expression of a nucleic acid may be modulated by introducing a genetic modification (preferably in the locus of the gene in question). The locus of a gene as defined herein is taken to mean a genomic region, which includes the gene of interest and 10 kb up- or down stream of the coding region.

The genetic modification may be introduced, for example, by any one (or more) of the following methods: T-DNA activation, TILLING, site-directed mutagenesis, directed evolution and homologous recombination or by introducing and expressing in a plant a nucleic acid encoding a Group I shaggy-like kinase or a homologue thereof. Following introduction of the genetic modification, there follows a step of selecting for modulated expression of the nucleic acid, which modulation in expression gives plants having improved growth characteristics, particularly increased yield.

T-DNA activation tagging (Hayashi et al. Science (1992) 1350-1353) involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through Agrobacterium infection and leads to overexpression of genes near the inserted T-DNA. The resulting transgenic plants show dominant phenotypes due to overexpression of genes close to the introduced promoter. The promoter to be introduced may be any promoter capable of directing expression of a gene in the desired organism, in this case a plant. For example, constitutive, tissue-preferred, cell type-preferred and inducible promoters are all suitable for use in T-DNA activation.

A genetic modification may also be introduced in the locus of the gene in question using the technique of TILLING (Targeted Induced Local Lesions In Genomes). This is a mutagenesis technology useful to generate and/or identify and isolate mutagenised variants. TILLING also allows selection of plants carrying such mutant variants. These mutant variants may even exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Site-directed mutagenesis may be used to generate variant nucleic acids. Several methods are available to achieve site-directed mutagenesis, the most common being PCR based methods (Current Protocols in Molecular Biology, Wiley Eds http://www.4ulr.com/products/currentprotocols/index.html).

Directed evolution or gene shuffling may also be used to generate nucleic acid variants. This consists of iterations of DNA shuffling followed by appropriate screening and/or selection to generate variants having a modified biological activity (Castle et al., (2004) Science 304(5674): 1151-4; US patents 5,811,238 and 6,395,547).

T-DNA activation, TILLING, site-directed mutagenesis and directed evolution are examples of technologies that enable the generation of novel alleles and variants.

Homologous recombination allows introduction in a genome of a selected nucleic acid at a defined selected position. Homologous recombination is a standard technology used routinely in biological sciences for lower organisms such as yeast or the moss Physcomitrella. Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; lida and Terada (2004) Curr Opin Biotech 15(2): 132-8). The nucleic acid to be targeted need not be targeted to the locus of the gene in question, but may be introduced in, for example, regions of high expression. The nucleic acid to be targeted may be an improved allele used to replace the endogenous gene or may be introduced in addition to the endogenous gene.

A preferred method for introducing a genetic modification is to introduce and express in a plant a nucleic acid encoding a Group I shaggy-like kinase or a homologue thereof. The nucleic acid to be introduced into a plant may be a full-length nucleic acid or may be a portion or a hybridising sequence as hereinbefore defined.

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived. To produce such homologues, amino acids of the protein may be replaced by other amino acids having similar properties (such as similar hydrophobicity, hydrophilicity, antigenicity, propensity to form or break α-helical structures or β-sheet structures). Conservative substitution tables are well known in the art (see for example Creighton (1984) Proteins. W.H. Freeman and Company and Table 4 below). The homologues useful in the methods according to the invention are preferably polypeptides have in increasing order of preference at least 30%, 40%, 50%, 60%, 70%, 80%, 85%, 90%, 95%, 96%, 97%, 98% or 99% or more sequence identity or similarity (functional identity) to the sequences mentioned herein as being useful in the methods of the invention, for example the sequences given in Table A and I herein

Also encompassed by the term "homologues" are two special forms of homology, which include orthologous sequences and paralogous sequences, which encompass evolutionary concepts used to describe ancestral relationships of genes. The term "paralogous" relates to gene-duplications within the genome of a species leading to paralogous genes. The term "orthologous" relates to homologous genes in different organisms due to speciation.

Orthologues in, for example, monocot plant species may easily be found by performing a so-called reciprocal blast search. Taking the example of finding an orthologue or paralogue of a a class I HDZip ho×5 nucleic acid or class I HDZip ho×5 polypeptide, this may be done by a first blast involving blasting the sequence in question against any sequence database, such as the publicly available NCBI database which may be found at: http://www.ncbi.nlm.nih.gov. BLASTN or TBLASTX may be used when starting from nucleotide sequence, or BLASTP or TBLASTN when starting from the protein, with standard default values. The BLAST results may be filtered. The full-length sequences of either the filtered results or the non-filtered results are then BLASTed back (second BLAST) against the sequences of the organism from which the sequence in question is derived. The results of the first and second BLASTs are then compared. When the results of the second BLAST give as hits with the highest similarity a class I HDZip ho×5 nucleic acid or class I HDZip ho×5 polypeptide, then a paralogue has been found, if it originates from the same organism as for the sequence used in the first BLAST. In case it originates from an organism other than that of the sequence used in the first BLAST, then an orthologue has been found. In the case of large families, ClustalW may be used, followed by a neighbour joining tree, to help visualize the clustering.

The same procedure may also be used to find orthologues and paralogues of nucleic acids encoding Group I shaggy-like kinases and Group I shaggy-like kinase polypeptides.

A homologue may be in the form of a "substitutional variant" of a protein, i.e. where at least one residue in an amino acid sequence has been removed and a different residue inserted in its place. Amino acid substitutions are typically of single residues, but may be clustered depending upon functional constraints placed upon the polypeptide; insertions will usually be of the order of about 1 to 10 amino acid residues. Preferably, amino acid substitutions comprise conservative amino acid substitutions. Conservative substitution tables are readily available in the art. The table below gives examples of conserved amino acid substitutions.

**Table 4: Examples of conserved amino acid substitutions**

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

A homologue may also be in the form of an "insertional variant" of a protein, i.e. where one or more amino acid residues are introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tang-100 epitope, c-myc epitope, FLAG@-epitope, lacZ, CMP (calmodulin-binding peptide), HA epitope, protein C epitope and VSV epitope.

Homologues in the form of "deletion variants" of a protein are characterised by the removal of one or more amino acids from a protein.

Amino acid variants of a protein may readily be made using peptide synthetic techniques well known in the art, such as solid phase peptide synthesis and the like, or by recombinant DNA manipulations. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

The nucleic acid encoding a Group I shaggy-like kinase or a homologue thereof may be a derivative. "Derivatives" include peptides, oligopeptides, polypeptides, proteins and enzymes which may comprise substitutions, deletions or additions of naturally and non-naturally occurring amino acid residues compared to the amino acid sequence of a naturally-occurring form of the protein. The Derivatives may comprise naturally occurring altered, glycosylated, acylated, prenylated or non-naturally occurring amino acid residues compared to the amino acid sequence of a naturally-occurring form of the polypeptide. A derivative may also comprise one or more non-amino acid substituents compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein.

The Group I shaggy-like kinase or a homologue thereof may be encoded by an alternative splice variant.

Where the sequence useful in the methodsof the invention is a nucleic acid encoding a Group I shaggy-like kinase or homologue thereof, the alternative splice variants are splice variants of the nucleic acid represented by any one of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29 and SEQ ID NO: 31. Further preferred are splice variants encoding a polypeptide having: (i) at least 77% sequence identity to the amino acid sequence represented by SEQ ID NO: 2; and (ii) motif I: R/H/V/N/Q E/G LK G/N and motif II: K Q/N CXXX G/A/S, where X may be any amino acid.

The present invention therefore provides a method for improving plant growth characteristics comprising modulating expression in a plant of an alternative splice variant of a nucleic acid encoding an Group I glycogen synthase kinase (Group I shaggy-like kinase) or a homologue thereof.

The term "alternative splice variant" as used herein encompasses variants of a nucleic acid sequence in which selected introns and/or exons have been excised, replaced or added, or in which introns have been shortened or lengthened. Such variants will be ones in which the biological activity of the protein is retained, which may be achieved by selectively retaining functional segments of the protein. Such splice variants may be found in nature or may be manmade. Methods for making such splice variants are well known in the art.

The homologue may also be encoded by an allelic variant of a nucleic acid encoding a Group I shaggy-like kinase or a homologue thereof.

Where the sequence useful in the methodsof the invention is a nucleic acid encoding a Group I shaggy-like kinase or homologue thereof, the allelic variants are allelic variants of the nucleic acid represented by any one of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29 and SEQ ID NO: 31. Further preferred are allelic variants encoding a polypeptide having: (i) at least 77% sequence identity to the amino acid sequence represented by SEQ ID NO: 2; and (ii) motif I: R/H/V/N/Q E/G LK G/N and motif II: K Q/N CXXX G/A/S, where X may be any amino acid.

The present invention therefore provides a method for improving plant growth characteristics comprising modulating expression in a plant of an allelic variant of a nucleic acid encoding an Group I glycogen synthase kinase (Group I shaggy-like kinase) or a homologue thereof.

Allelic variants exist in nature, and encompassed within the methods of the present invention is the use of these natural alleles. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

According to the present invention, modulated expression of the nucleic acid or variant thereof is envisaged. Methods for modulating expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of the nucleic acid or variant thereof. For example, endogenous promoters may be altered in vivo by mutation, deletion, and/or substitution (see, Kmiec, U.S. Pat. No. 5,565,350; Zarling et al., PCT/US93/03868), or isolated promoters may be introduced into a plant cell in the proper orientation and distance from a gene of the present invention so as to control the expression of the gene.

Methods for reducing the expression of genes or gene products are well documented in the art.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or from any other eukaryotic gene.

An intron sequence may also be added to the 5' untranslated region or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. See generally, The Maize Handbook, Chapter 116, Freeling and Walbot, Eds., Springer, N.Y. (1994).

The invention also provides genetic constructs and vectors to facilitate introduction and/or expression of the nucleotide sequences useful in the methods according to the invention.

Therefore, there is provided a gene construct comprising:
(i) A Group I shaggy-like kinase-encoding nucleic acid or variant thereof;
(ii) One or more control sequences capable of driving expression of the nucleic acid sequence of (i); and optionally
(iii) A transcription termination sequence.

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells. The invention therefore provides use of a gene construct as defined hereinabove in the methods of the invention.

Plants are transformed with a vector comprising the sequence of interest. The sequence of interest is operably linked to one or more control sequences (at least to a promoter). The terms "regulatory element", "control sequence" and "promoter" are all used interchangeably herein and are to be taken in a broad context to refer to regulatory nucleic acid sequences capable of effecting expression of the sequences to which they are ligated. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ. The term "operably linked" as used herein refers to a functional linkage between the promoter sequence and the gene of interest, such that the promoter sequence is able to initiate transcription of the gene of interest.

Advantageously, any type of promoter may be used to drive expression of the nucleic acid sequence. The promoter may be an inducible promoter, i.e. having induced or increased transcription initiation in response to a developmental, chemical, environmental or physical stimulus. An example of an inducible promoter being a stress-inducible promoter, i.e. a promoter activated when a plant is exposed to various stress conditions. Additionally or alternatively, the promoter may be a tissue-preferred promoter, i.e. one that is capable of preferentially initiating transcription in certain tissues, such as the leaves, roots, seed tissue etc. Promoters able to initiate transcription in certain tissues only are referred to herein as "tissue-specific".

Preferably, the Group I shaggy-like kinase-encoding nucleic acid or variant thereof is operably linked to a constitutive promoter. A constitutive promoter is transcriptionally active during most but not necessarily all phases of growth and development and is substantially ubiquitously expressed. The constitutive promoter is preferably a GOS2 promoter, more preferably the constitutive promoter is a rice GOS2 promoter, further preferably the constitutive promoter is represented by a nucleic acid sequence substantially similar to SEQ ID NO: 33 most preferably the constitutive promoter is as represented by SEQ ID NO: 33. It should be clear that the applicability of the present invention is not restricted to the Group I shaggy-like kinase-encoding nucleic acid or variant thereof when driven by a GOS2 promoter. Examples of other constitutive promoters which may also be used to perform the methods of the invention are shown in Table 5 below.

**Table 5: Examples of constitutive promoters**

| Gene Source | Reference |
|---|---|
| Actin | McElroy et al, Plant Cell, 2: 163-171, 1990 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| Nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Examples of seed-specific promoters which may also be used to drive expression of a nucleic acid are shown in Table 6 below.

**Table 6: Examples of seed-specific promoters**

| Gene source | Expression pattern | Reference |
|---|---|---|
| seed-specific genes | seed | Simon, et al., Plant Mol. Biol. 5: 191, 1985; Scofield, et al., J. Biol. Chem. 262: 12202, 1987.; Baszczynski, et al., Plant Mol. Biol. 14: 633, 1990. |
| Brazil Nut albumin | seed | Pearson, et al., Plant Mol. Biol. 18: 235-245, 1992. |
| Legumin | seed | Ellis, et al., Plant Mol. Biol. 10: 203-214, 1988. |
| Glutelin (rice) | seed | Takaiwa, et al., Mol. Gen. Genet. 208: 15-22, 1986; Takaiwa, et al., FEBS Letts. 221: 43-47, 1987. |
| Zein | seed | Matzke et al Plant Mol Biol, 14(3):323-32 1990 |
| napA | seed | Stalberg, et al, Planta 199: 515-519, 1996. |
| wheat LMW and HMW glutenin-1 | endosperm | Mol Gen Genet 216:81-90, 1989; NAR 17:461-2, 1989 |
| wheat SPA | seed | Albani et al, Plant Cell, 9: 171-184, 1997 |
| wheat α, β, γ-gliadins | endosperm | EMBO 3:1409-15, 1984 |
| barley Itr1 promoter | endosperm | |
| barley B1, C, D, hordein | endosperm | Theor Appl Gen 98:1253-62, 1999; Plant J 4:343-55, 1993; Mol Gen Genet 250:750-60, 1996 |
| barley DOF | endosperm | Mena et al, The Plant Journal, 116(1): 53-62, 1998 |
| blz2 | endosperm | EP99106056.7 |
| synthetic promoter | endosperm | Vicente-Carbajosa et al., Plant J. 13: 629-640, 1998. |
| rice prolamin NRP33 | endosperm | Wu et al, Plant Cell Physiology 39(8) 885-889, 1998 |
| rice α-globulin Glb-1 | endosperm | Wu et al, Plant Cell Physiology 39(8) 885-889, 1998 |
| rice OSH1 | embryo | Sato et al, Proc. Natl. Acad. Sci. USA, 93: 8117-8122, 1996 |
| rice α-globulin REB/OHP-1 | endosperm | Nakase et al. Plant Mol. Biol. 33: 513-522, 1997 |
| rice ADP-glucose PP | endosperm | Trans Res 6:157-68, 1997 |
| maize ESR gene family | endosperm | Plant J 12:235-46, 1997 |
| Sorgum γ-kafirin | endosperm | PMB 32:1029-35, 1996 |
| KNOX | embryo | Postma-Haarsma et al, Plant Mol. Biol. 39:257-71, 1999 |
| rice oleosin | embryo and aleuron | Wu et at, J. Biochem., 123:386, 1998 |
| sunflower oleosin | seed (embryo and dry seed) | Cummins, et al., Plant Mol. Biol. 19: 873-876, 1992 |
| putative rice 40S ribosomal protein | weak in endosperm | |
| rice alpha-globulin | strong in endosperm | |
| rice alanine aminotransferase | weak in endosperm | |
| trypsin inhibitor ITR1 (barley) | weak in endosperm | |
| rice WSI18 | embryo + stress | |
| rice RAB21 | embryo + stress | |
| rice oleosin 18kd | aleurone + embryo | |

For the identification of functionally equivalent promoters, the promoter strength and/or expression pattern of a candidate promoter may be analysed for example by operably linking the promoter to a reporter gene and assay the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1,000 transcripts per cell.

Optionally, one or more terminator sequences may also be used in the construct introduced into a plant. The term "terminator" encompasses a control sequence which is a DNA sequence at the end of a transcriptional unit which signals 3' processing and polyadenylation of a primary transcript and termination of transcription. Additional regulatory elements may include transcriptional as well as translational enhancers. Those skilled in the art will be aware of terminator and enhancer sequences that may be suitable for use in performing the invention. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

The genetic construct may optionally comprise a selectable marker gene. As used herein, the term "selectable marker gene" includes any gene that confers a phenotype on a cell in which it is expressed to facilitate the identification and/or selection of cells that are transfected or transformed with a nucleic acid construct of the invention.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. As used herein, the term "selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptll that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xlose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

In a preferred embodiment, there is provided a gene construct comprising:
(i) A Group I shaggy-like kinase-encoding nucleic acid or variant thereof;
(ii) A constitutive promoter capable of driving expression of the nucleic acid sequence of (i); and optionally
(iii) A transcription termination sequence.

The constitutive promoter is preferably a GOS2 promoter, more preferably the constitutive promoter is the rice GOS2 promoter, further preferably the constitutive promoter is represented by a nucleic acid sequence substantially similar to SEQ ID NO: 33, most preferably the constitutive promoter is as represented by SEQ ID NO: 33. The invention further provides use of a construct as defined hereinabove in the methods of the invention.

The present invention also encompasses plants obtainable by the methods according to the present invention. The present invention therefore provides plants, plant parts (including plant cells) obtainable by the methods according to the present invention, which plants or parts (including cells) comprise a transgene Group I shaggy-like kinase-encoding nucleic acid or variant thereof.

The invention also provides a method for the production of transgenic plants having improved growth characteristics, particularly increased yield, relative to corresponding wild type or other control plants, comprising introduction and expression in a plant of any of the nucleic acids described herein as being useful in the methods of the invention.

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either
(a) the nucleic acid sequences encoding proteins useful in the methods of the invention, or
(b) genetic control sequence(s) which is operably linked with the nucleic acid sequence according to the invention, for example a promoter, or
(c) a) and b)
are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

More specifically, the present invention provides a method for the production of transgenic plants having improved growth characteristics (particularly increased yield) which method comprises:
(i) introducing and expressing in a plant, plant part or plant cell a Group I shaggy-like kinase-encoding nucleic acid or variant thereof; and
(ii) cultivating the plant cell under conditions promoting plant growth and development.

The nucleic acid may be introduced directly into a plant cell or into the plant itself (including introduction into a tissue, organ or any other part of a plant). According to a preferred feature of the present invention, the nucleic acid is preferably introduced into a plant by transformation.

The term "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

Transformation of plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al. (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al. (1986) Mol. Gen Genet 202: 179-185); DNA or RNA-coated particle bombardment (Klein TM et al. (1987) Nature 327: 70) infection with (non-integrative) viruses and the like. Transgenic rice plants are preferably produced via Agrobacterium-mediated transformation using any of the well known methods for rice transformation, such as described in any of the following: published European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either lshida et al. (Nat. Biotechnol 14(6): 745-50, 1996) or Frame et al. (Plant Physiol 129(1): 13-22, 2002), which disclosures are incorporated by reference herein as if fully set forth.

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, or quantitative PCR, all techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells comprising a Group I shaggy-like kinase-encoding nucleic acid or variant thereof. Preferred host cells are plant cells.

The invention also extends to harvestable parts of a plant such as, but not limited to seeds, leaves, fruits, flowers, stems, rhizomes, tubers and bulbs. The invention furthermore relates to products derived from a harvestable part of such a plant, such as dry pellets or powders, oil, fat and fatty acids, starch or proteins.

The present invention also use of Group I shaggy-like kinase-encoding nucleic acids and variants thereof and use of Group I shaggy-like kinase polypetides and homologues thereof. Such uses relate to improving any of the plant growth characteristics as defined hereinabove.

Group I shaggy-like kinase-encoding nucleic acids and variants thereof, or Group I shaggy-like kinase polypetides and homologues thereof may find use in breeding programmes in which a DNA marker is identified which may be genetically linked to one of the aforementioned nucleic acids or variants. The nucleic acids or variants, or polypeptides or homologues thereof may be used to define a molecular marker. This DNA or protein marker may then be used in breeding programmes to select plants having improved growth characteristics (such as increased yield). The nucleic acids or variants are as defined hereinabove.

Allelic variants of a Group I shaggy-like kinase-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give improved growth characteristics, such as increased yield. Selection is typically carried out by monitoring growth performance of plants containing different allelic variants of the sequence in question, for example, different allelic variants of any of the nucleic acids/genes described herein as being useful in the methods of the invention. Growth performance may be monitored in a greenhouse or in the field. Further optional steps include crossing plants in which the superior allelic variant was identified with another plant. This could be used, for example, to make a combination of interesting phenotypic features.

The aforementioned nucleic acid and variants may also be used as probes for genetically and physically mapping the genes that they are a part of, and as markers for traits linked to those genes. Such information may be useful in plant breeding in order to develop lines with desired phenotypes. Such use of the nucleic acids or variants requires only a nucleic acid sequence of at least 15 nucleotides in length. The nucleic acids or variants may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids or variants. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid or variant in the genetic map previously obtained using this population (Botstein et al. (1980) Am. J. Hum. Genet. 32:314-331).

The production and use of plant gene-derived probes for use in genetic mapping is described in Bematzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favor use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having improved growth characteristics, as described hereinbefore. These improved growth characteristics may also be combined with other economically advantageous traits, such as further yield-enhancing traits, tolerance to other abiotic and biotic stresses, traits modifying various architectural features and/or biochemical and/or physiological features.

### Description of figures

The present invention will now be described with reference to the following figures in which:

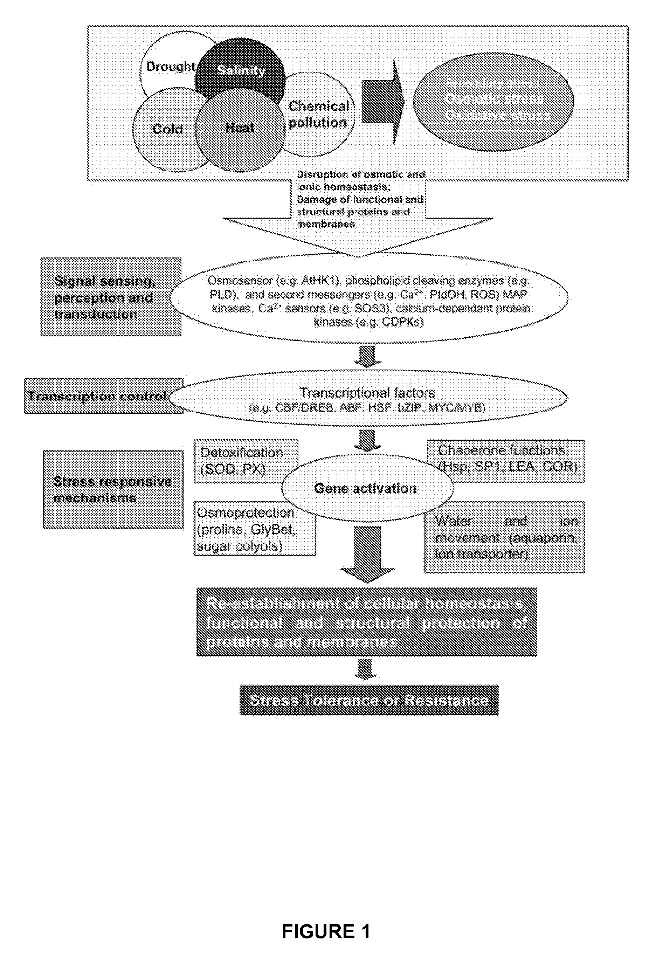

Fig. 1 taken from Planta (2003) 218: 1-14 (Wang et al.), shows plant response to abiotic stress. Primary stresses, such as drought, salinity, cold, heat and chemical pollution are often interconnected, and cause cellular damage and secondary stresses, such as osmotic and oxidative stress. The initial stress signals (e.g. osmotic and ionic effects, or temperature, membrane fluidity changes) trigger the downstream signalling process and transcription controls which activate stress-responsive mechanism to re-establish homeostasis and protect and repair damaged proteins and membranes. Inadequate response at one or several steps in the signalling and gene activation may ultimately result in irreversible changes of cellular homeostasis and in destruction of functional and structural proteins and membranes, leading to cell death.

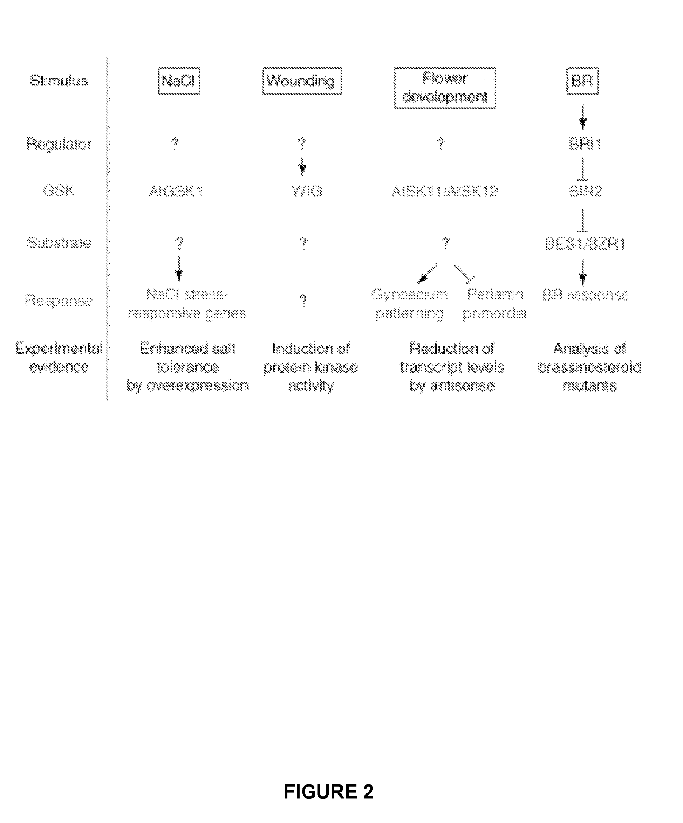

Fig. 2 taken from from TRENDS in plant Science Vol. 7, No. 10, Oct. 2002 (Claudia Jonak and Heribert Hirt) shows possible shaggy-like kinase pathways in plants. AtGSK1 is a positive regulator of the high-salt response. WIG (wound-induced GSK) is implicated in wound signalling. AtSK11 and AtSK12 are involved in correct flower patterning. Genetic and biochemical analyses indicate that BIN2 (brassinosteroid-insensitive 1)-mediated brassinosteroid (BR) signalling, which controls nuclear accumulation of BES1 (BR11-EMS-suppressor 1) and BRZ1 (brassinazole-resistant 1).
Fig. 3 taken from TRENDS in plant Science Vol. 7, No. 10, Oct. 2002 (Claudia Jonak and Heribert Hirt) shows: (a) Phylogenetic analysis of the ten Arabidopsis glycogen synthase kinase 3/SHAGGY-like protein kinases (GSKs). The Arabidopsis GSKs can be classified into four subgroups. (b) Phylogenetic tree of full-length GSK cDNAs of different plant species obtained from various BLAST searches. Brassica napus, BSKθ (Y12674); Medicago sativa, MSK1 (X68411), MSK2 (X68410), MSK3 (X68409), MSK4 (AF432225), WIG (AJ295939); Nicotiana tabacum, NSK6 (Y08607), NSK59 (AJ002315), NSK91 (AJ224163), NSK111 (AJ002314), NTK-1 (X77763); Oryza sativa, OSKy (AB59612), OSKη (Y13437); Petunia hybrida, PSK4 (X83619), PSK6 (AJ224164), PSK7 (AJ224165), SPK6 (X83620). Alignments were performed with Clustal X by the neighbor-joining method using Arabidopsis MPK1 (Q39021) as an out-group; trees were designed by the TreeView program.
Fig. 4 shows a CLUSTAL multiple alignment of plant Group I shaggy-like kinases. Motifs I and II are boxed.

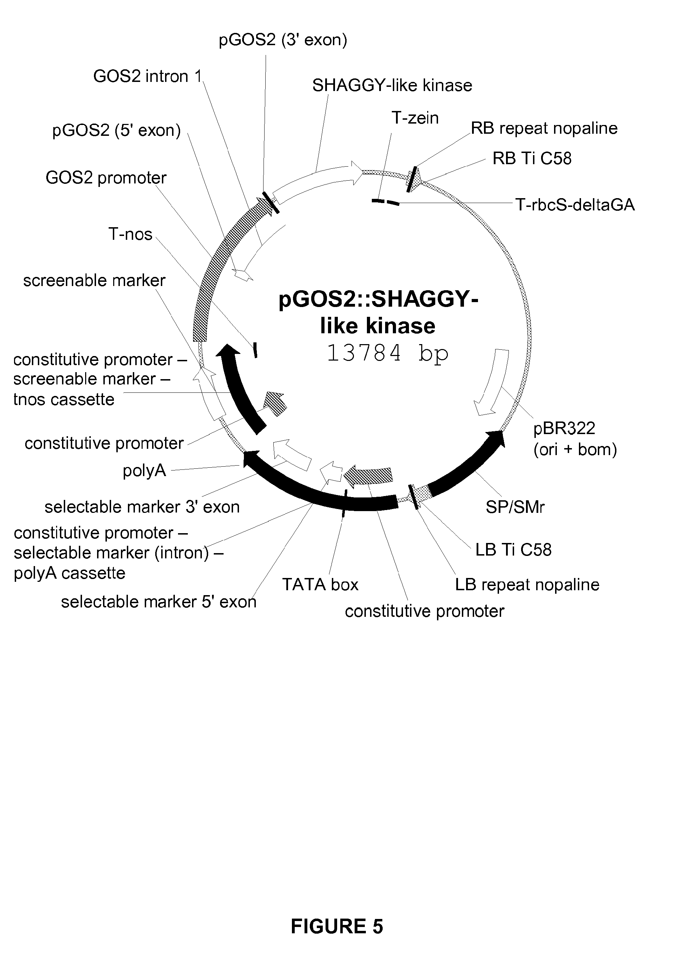

Fig. 5 shows a binary vector for expression in Oryza sativa of a Group I shaggy-like kinase from Oryza sativa under the control of a GOS2 promoter

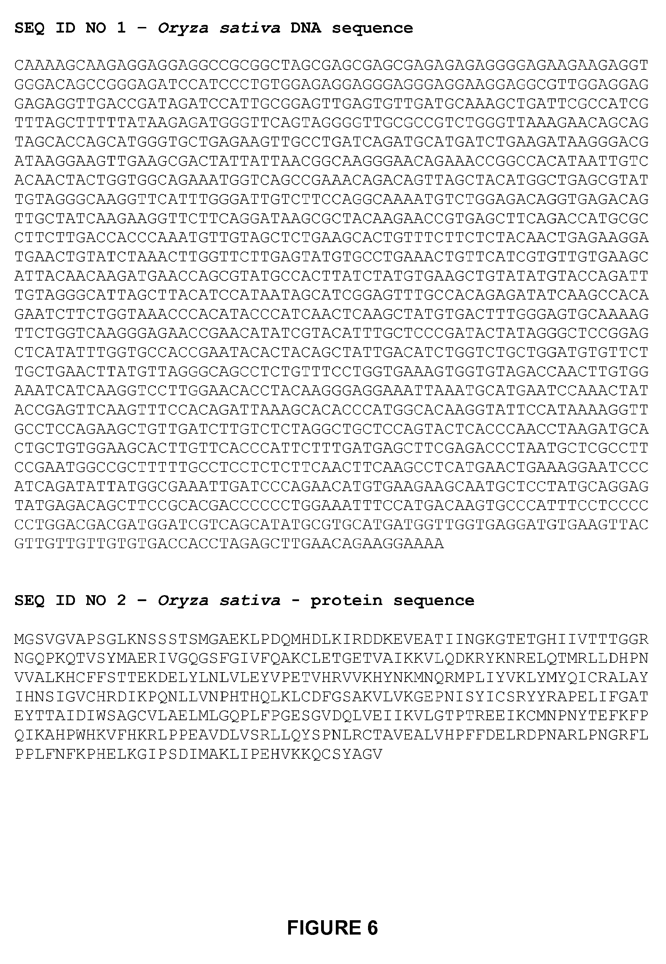

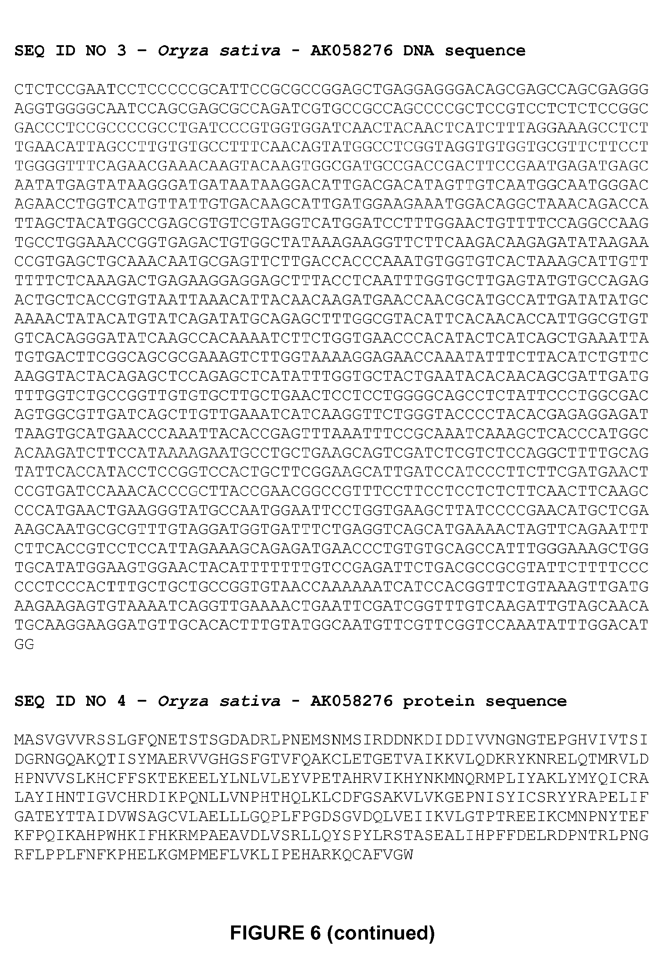

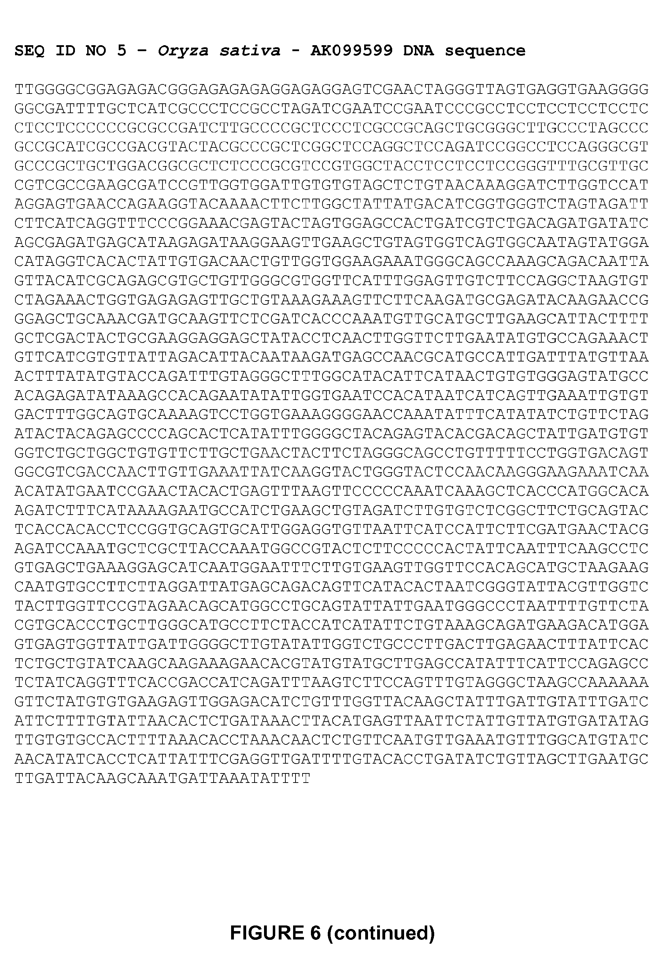

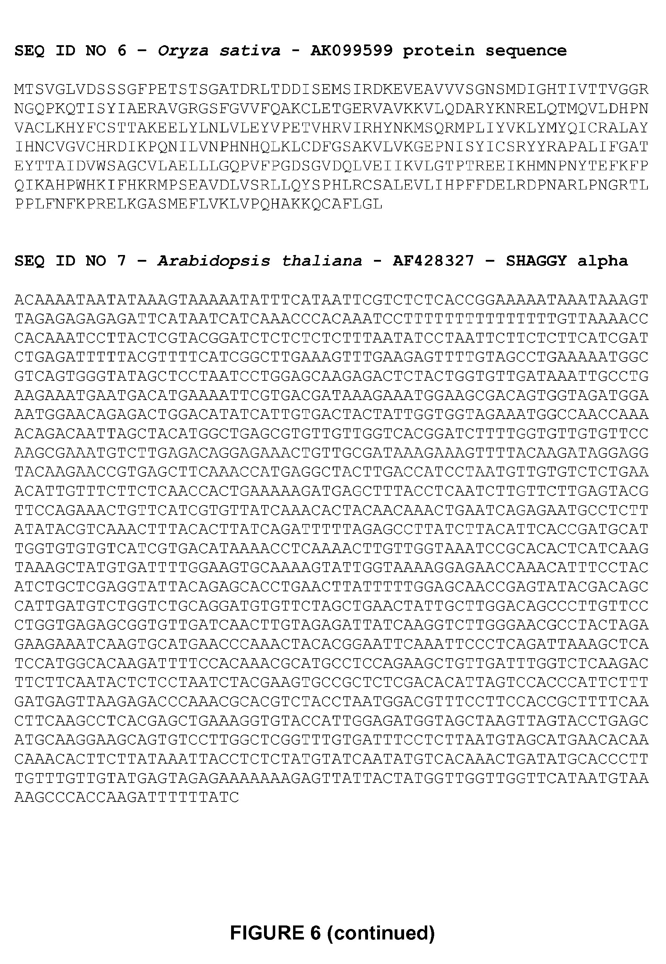

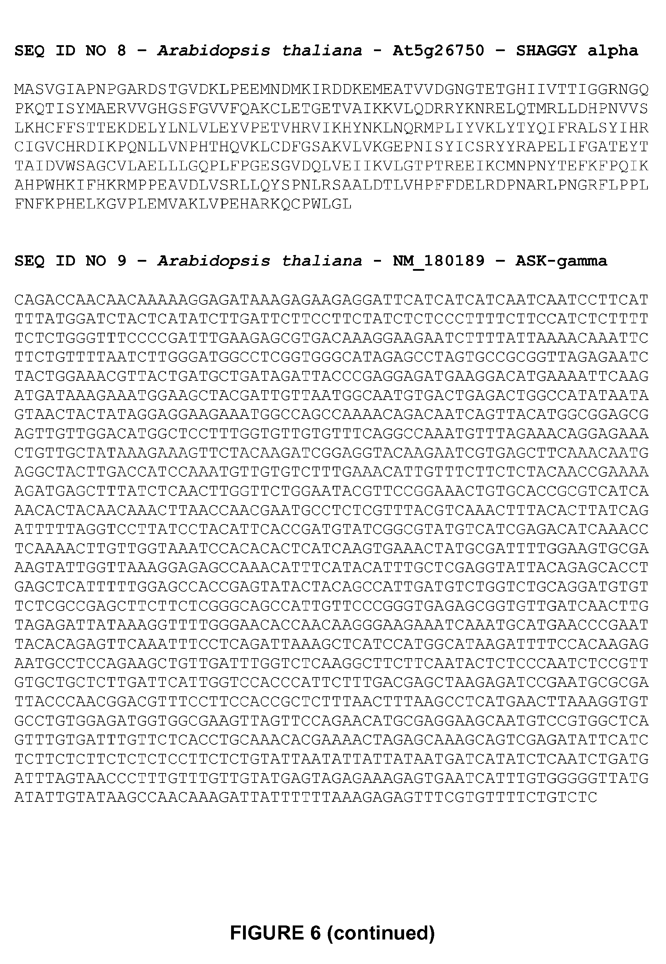

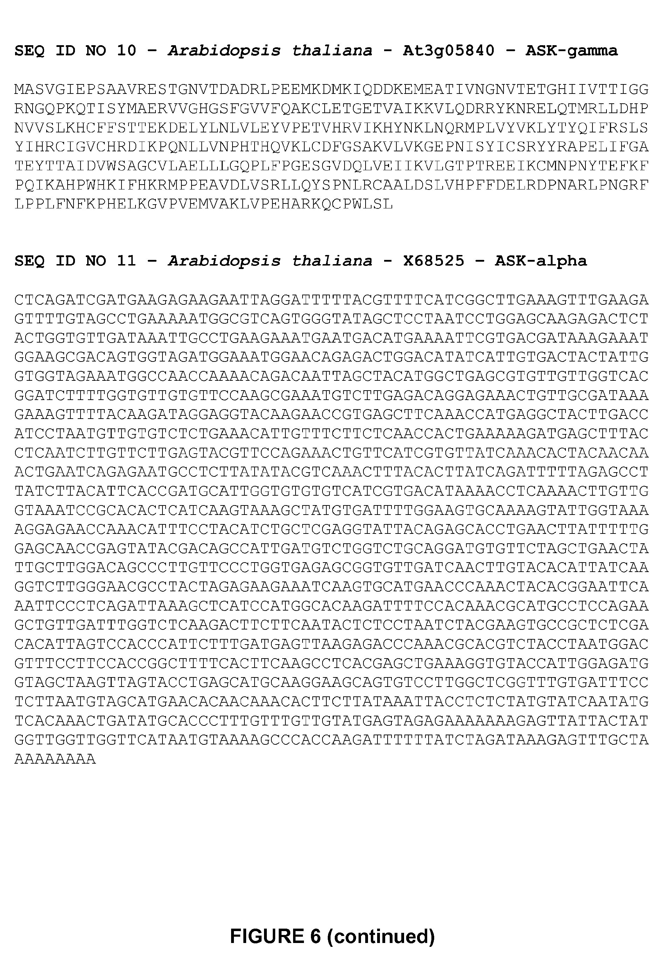

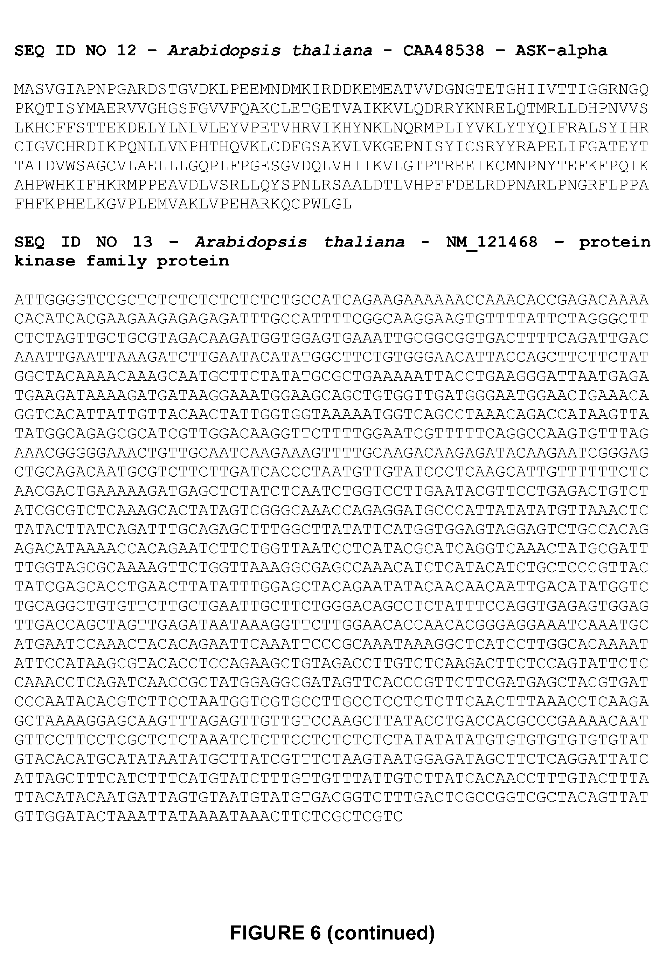

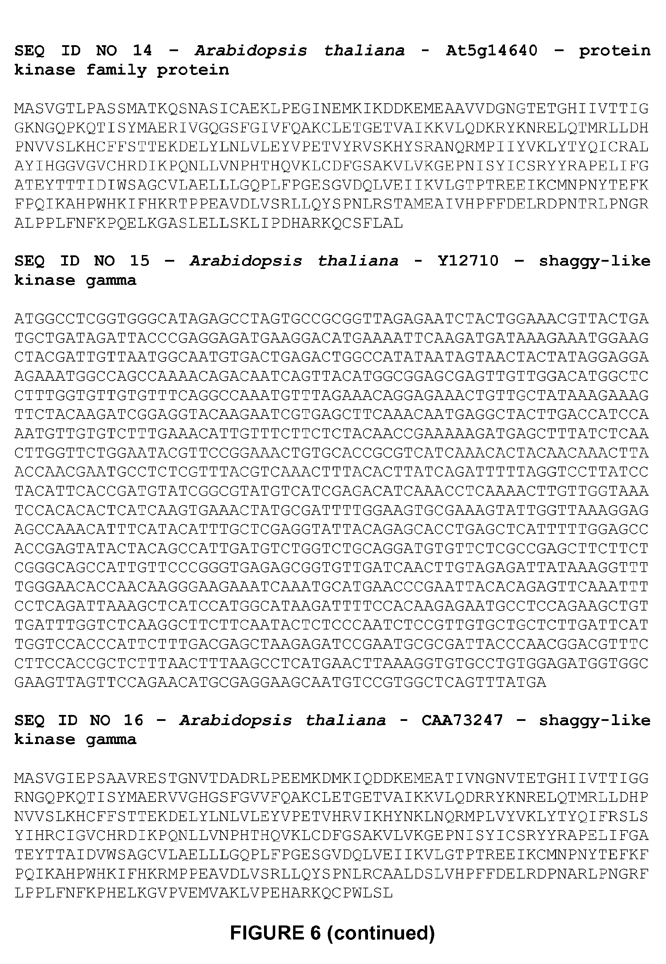

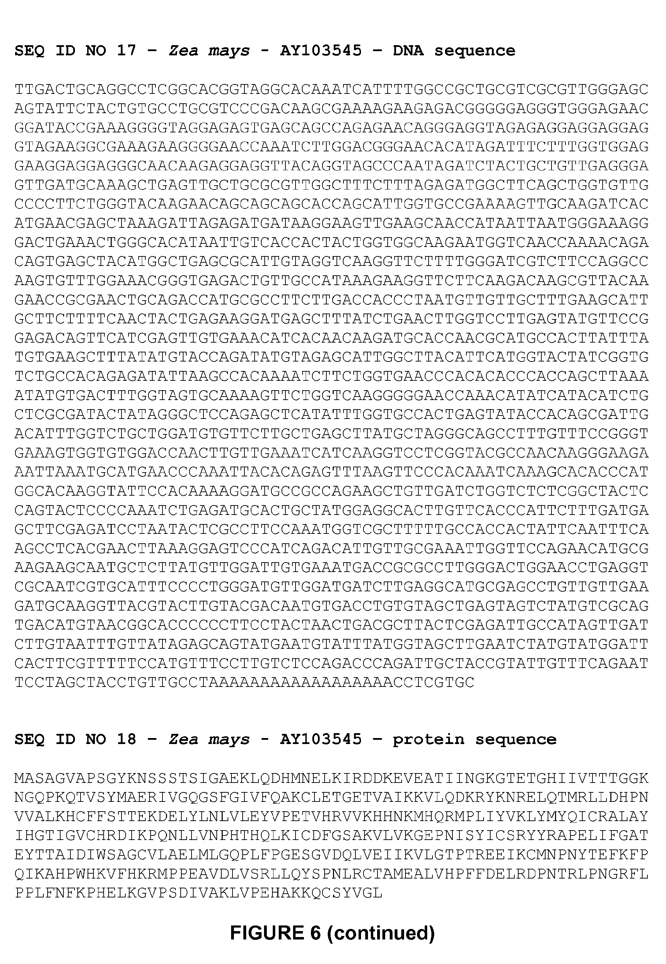

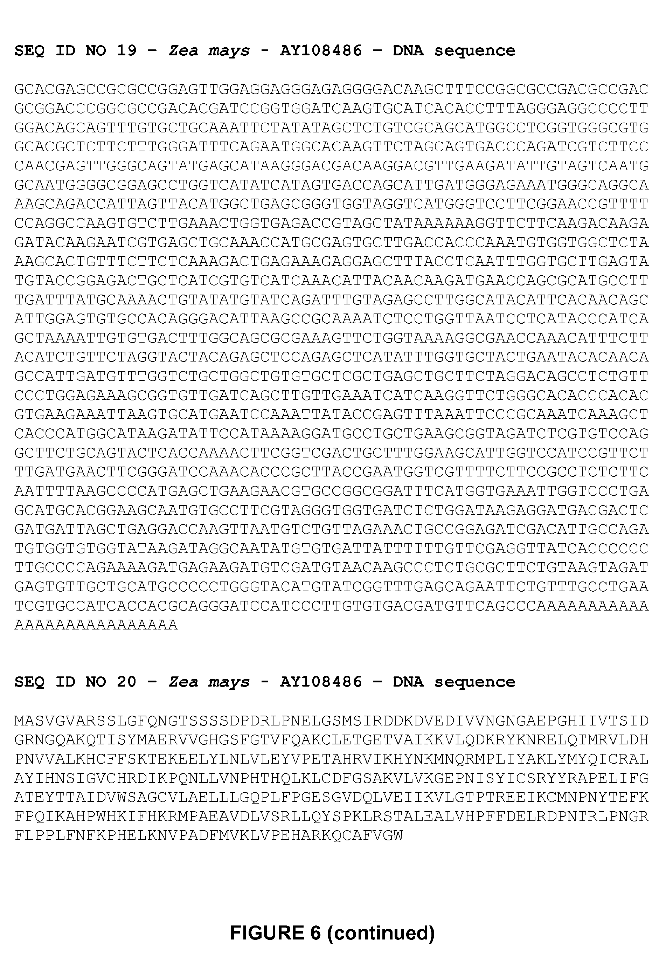

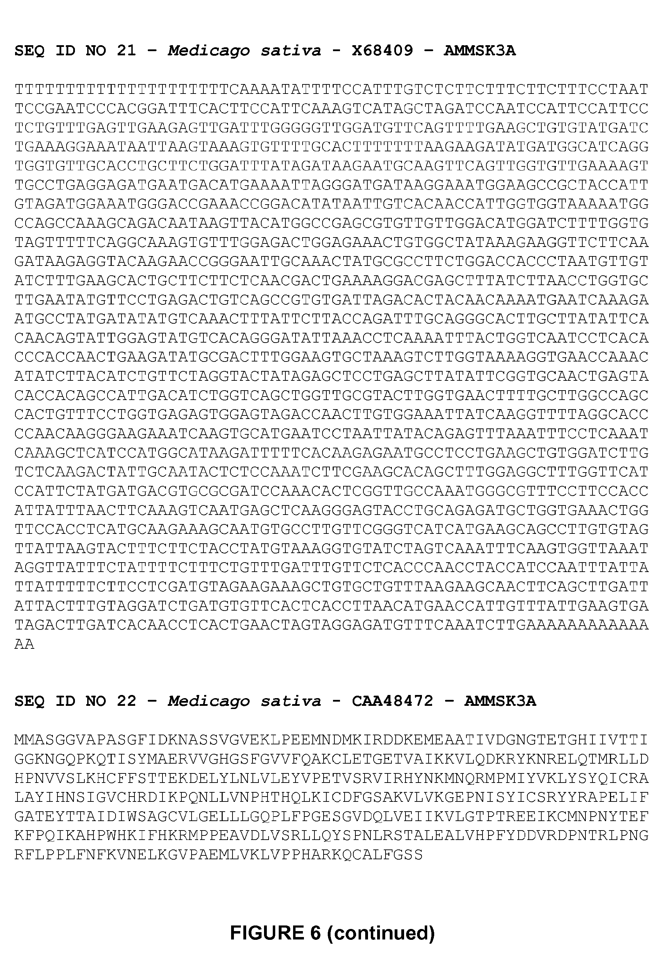

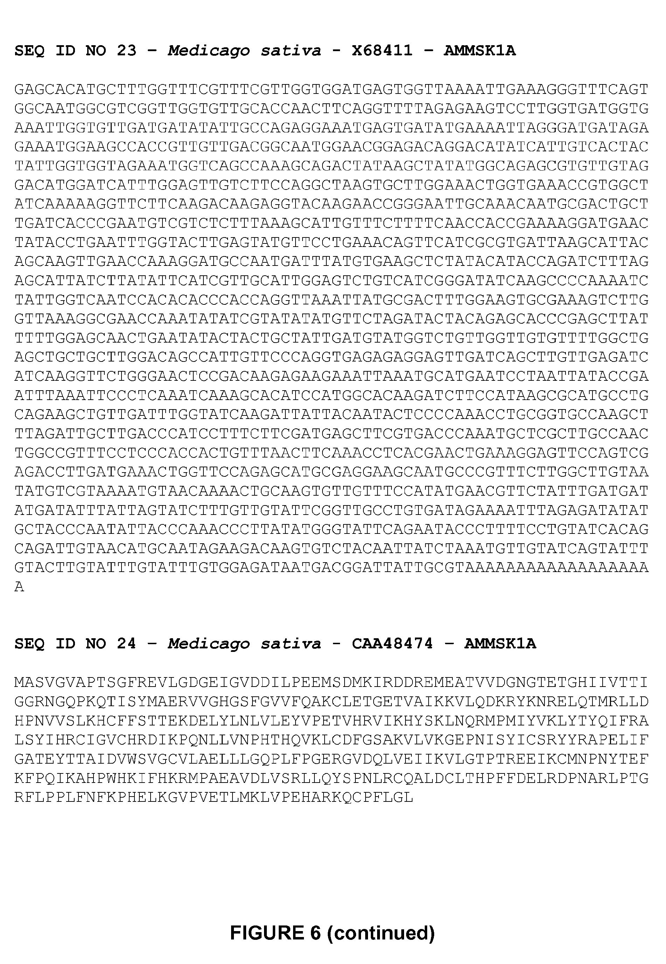

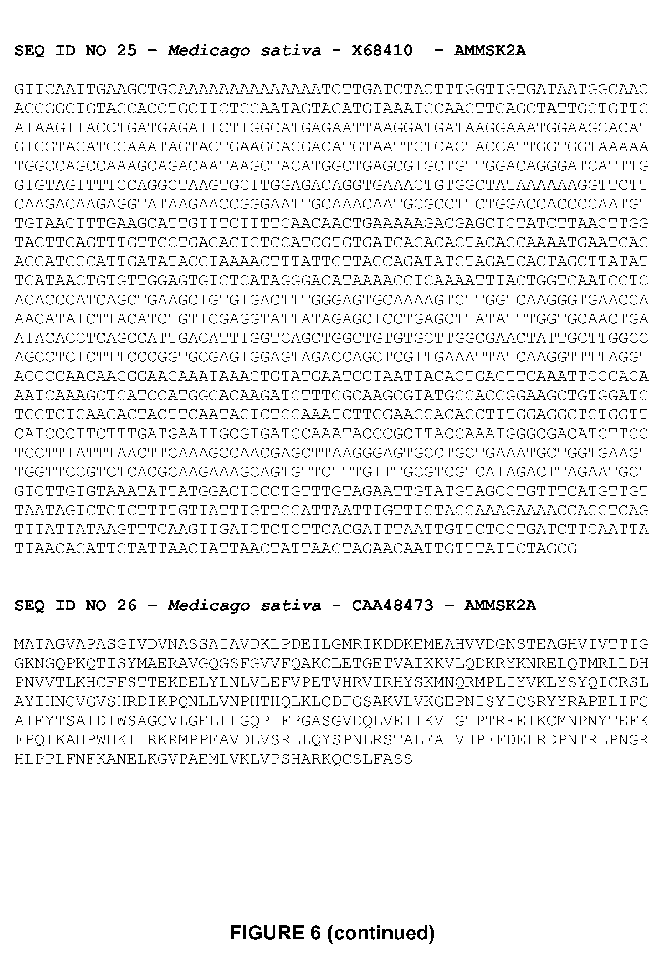

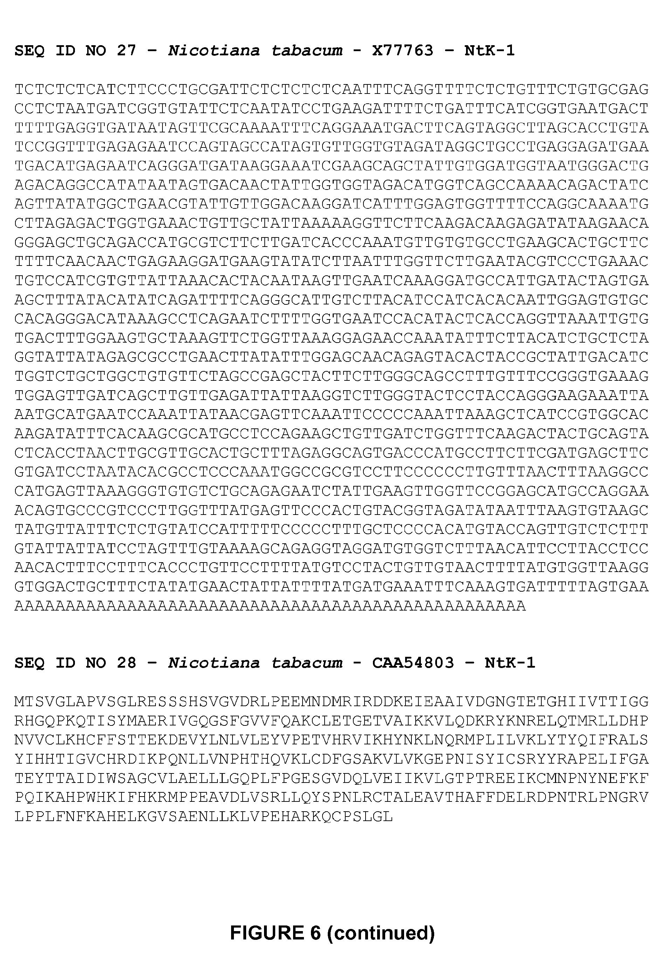

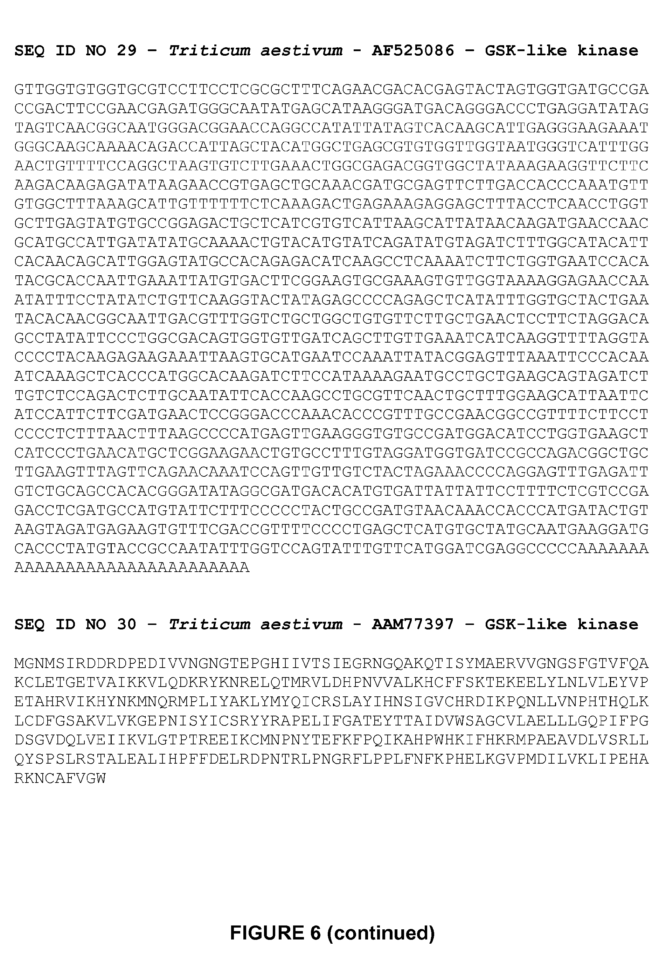

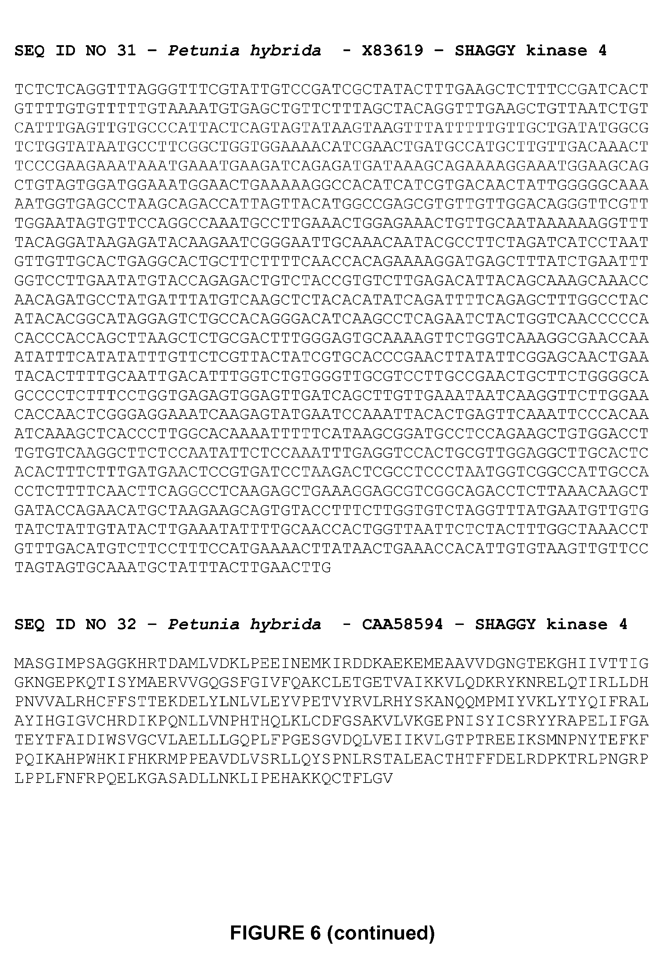

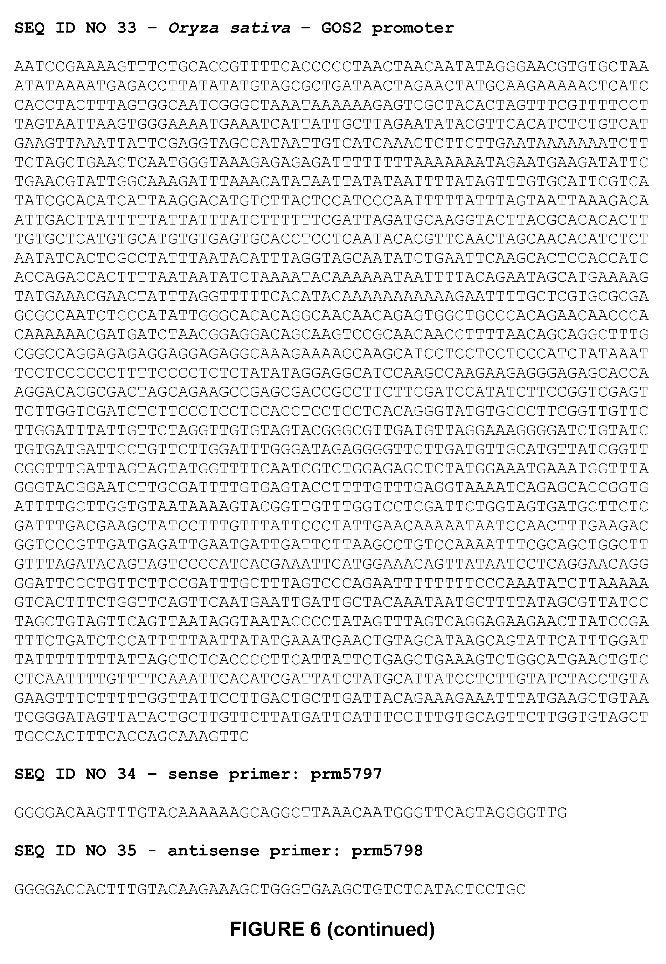

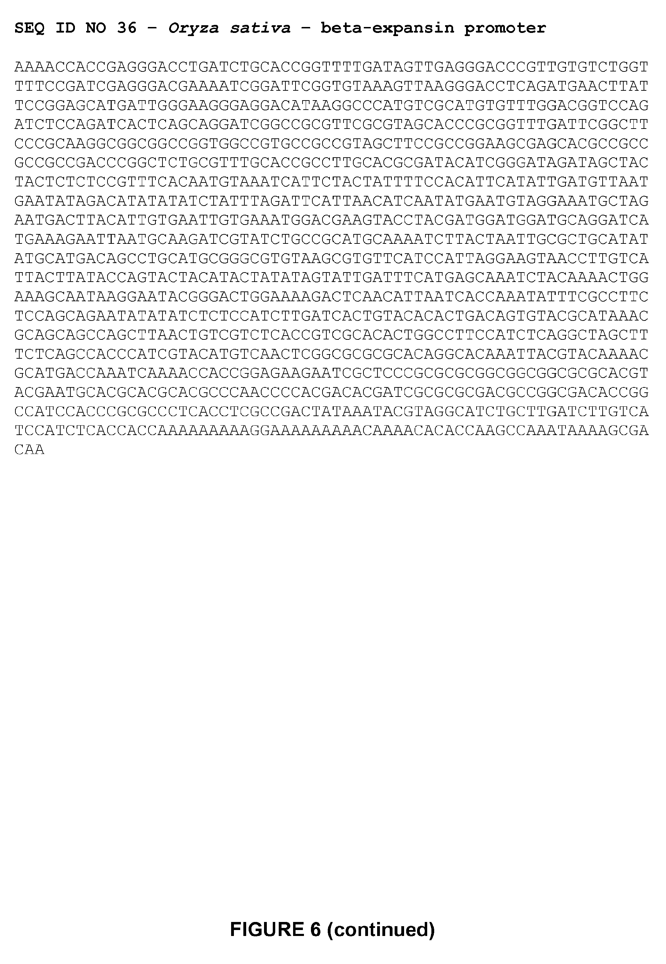

Fig. 6 details examples of Group I shaggy-like kinase sequences useful in performing the methods according to the present invention.

### Examples

The present invention will now be described with reference to the following examples, which are by way of illustration alone and are not intended to completely define or to otherwise limit the scope of the invention.

Unless otherwise stated, recombinant DNA techniques were performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfase (1993) by R.D.D. Croy, published by BIOS Scientific Publications Ltd (UK) and Blackwell Scientific Publications (UK).

### Example 1: Gene Cloning

The Oryza sativa Group I shaggy-like kinase-encoding gene was amplified by PCR using as a template an Arabidopsis thaliana seedling cDNA library (Invitrogen, Paisley, UK). After reverse transcription of RNA extracted from seedlings, the cDNAs were cloned into pCMV Sport 6.0. Average insert size of the bank was 1.5 kb and the original number of clones was of the order of 1.59x10⁷ cfu. Original titer was determined to be 9.6x10⁵ cfu/ml after first amplification of 6×10¹¹ cfu/ml. After plasmid extraction, 200 ng of template was used in a 50 µl PCR mix. Primers prm5797 (SEQ ID NO: 34; sense, start codon in bold, AttB1 site in italic: 5'-ggggacaagtttgtacaaaaaagcaggcttaaacaatgggttcagtaggggttg-3') and prm5798 (SEQ ID NO: 35; reverse, complementary, stop codon in bold, AttB2 site in italic: 5'-ggggaccactttgtacaaga aagctgggtgaagctgtctcatactcctgc-3'), which include the AttB sites for Gateway recombination, were used for PCR amplification. PCR was performed using Hifi Taq DNA polymerase in standard conditions. A PCR fragment of 1328 bp was amplified and purified also using standard methods. The first step of the Gateway procedure, the BP reaction, was then performed, during which the PCR fragment recombines in vivo with the pDONR201 plasmid to produce, according to the Gateway terminology, an "entry clone". Plasmid pDONR201 was purchased from Invitrogen, as part of the Gateway@ technology.

### Example 2: Vector Construction

The entry clone was subsequently used in an LR reaction with a destination vector used for Oryza sativa transformation. This vector contains as functional elements within the T-DNA borders: a plant selectable marker; a screenable marker expression cassette; and a Gateway cassette intended for LR in vivo recombination with the sequence of interest already cloned in the entry clone. A GOS2 promoter from rice, for constitutive expression, was upstream of this Gateway cassette (De Pater et al., Plant J. 1992 Nov; 2(6):837-44). After the LR recombination step, the resulting expression vector (Figure 5) was transformed into Agrobacterium strain LBA4044 and subsequently to Oryza sativa plants as described in Example 3.

### Example 3: Rice Transformation

Mature dry seeds of a rice japonica cultivar were dehusked. Sterilization was carried out by incubating for one minute in 70% ethanol, followed by 30 minutes in 0.2%HgCI2, followed by a 6X15 minute wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity). Agrobacterium strain LBA4404 harbouring binary T-DNA vectors were used for co-cultivation. Agrobacterium was inoculated on AB medium with the appropriate antibiotics and cultured for 3 days at 28°C. The bacteria were then collected and suspended in liquid co-cultivation medium to a density (OD600) of about 1. The suspension was then transferred to a petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a suitable concentration of the selective agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse. Seeds were then harvested three to five months after transplanting. The method yielded single locus transformants at a rate of over 50 (Aldemita and Hodges, Planta, 199 612-617, 1996; Chan et al., Plant Mol. Biol. 22 (3) 491-506, 1993, Hiei et al., Plant J., 6 (2) 271-282, 1994).

### Example 4: Salt Stress Screen

Seeds were sown and seedlings selected by monitoring visual marker expression. Ten days after sowing, the seedlings were transplanted to plastic pots, 12cm in diameter, filled with a 1:1 mixture of moist sand and vermiculite. The pots were soaked in fresh water before transplantation. Seedlings were then transplanted from tissue culture chambers to a greenhouse for growing and harvest of T1 seed. The pots were submitted to salt conditions one day after transplantation. Pots were watered 4 times a day at 8am, 12pm, 4pm, and 9pm with a salt-stress nutrient solution containing 25mM NaCI and the components listed below.
● NPK Nutrient mix, 20-20-20 Peters professional (Scotts, Marysville, OH, USA) at a concentration of 1 kg/m³.
● Magnesium chelate, Chelal Mg (BMS, Bornem, Belgium) at 333.33 ml / m³
● Iron chelate, Libfer (CIBA, Bradford, UK) at 21.67 g / m³
● NaCI 1.425 kg / m3

Salt concentration was monitored on a weekly basis and additions were made where necessary. Plants were grown under these conditions until the start of grain filling. They were then transferred to a different compartment of the greenhouse where they were irrigated daily with fresh water until seed harvest. Growth and yield parameters were recorded as detailed in Example 5.

### Example 5: Evaluation and Results

Approximately 15 to 20 independent T0 rice transformants were generated. The primary transformants were transferred from a tissue culture chamber to a greenhouse for growing and harvest of T1 seed. At least 5 events for which the T1 progeny segregated 3:1 for presence/absence of the transgene were retained. For each of these events, approximately 10 T1 seedlings containing the transgene (hetero- and homo-zygotes) and approximately 10 T1 seedlings lacking the transgene (nullizygotes) were selected by monitoring visual marker expression. 4 of the best performing T1 events were further evaluated in the T2 generation following the same evaluation procedure as for the T1 generation but with more individuals per event.

### Statistical analysis: F-test

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and for an overall effect of the gene, also known as a global gene effect. The threshold for significance for a true global gene effect was set at a 5% probability level. A significant F-test value points to a gene effect, meaning that it is not only the presence or position of the gene that is causing the differences in phenotype.

### 5.1 Seed-related parameter measurements

Mature primary panicles were harvested, bagged, barcode-labelled and then dried for three days in the oven at 37°C. The panicles were then threshed and all the seeds were collected and counted. Filled husks were separated from the empty ones using an airblowing device. The empty husks were discarded and the remaining fraction was counted again. Filled husks were weighed on an analytical balance. The total seed yield was measured by weighing all filled husks harvested from a plant. The harvest index in the present invention is defined as a ratio of total seed yield and the aboveground area (mm²) multiplied by a factor 106.

The Table of results below show the p values from the F test for T1 and T2 evaluations. The percentage difference between the transgenics and the corresponding nullizygotes (or plants without the transgene) is also shown. For example, for total seed weight in the T1 generation, 2 events were positive for total seed weight (i.e., showed an increase in total seed weight (of 54%, with a p-value from the F-test of <0.1938) compared to the seed weight of corresponding nullizygote plants in conditions of salt stress). In the T2 generation, 1 event was positive for total seed weight (i.e., showed an increase in total seed weight (of 65%, with a p-value from the F-test of 0.0252) compared to the seed weight of corresponding nullizygote plants in conditions of salt stress).

**Table Q: T1 and T2 generation shaggy-like kinase transgenic plants and corresponding non-transgenic plants under salt-stress**

| Phenotype | T1 | Diff | p-value | T2 | Diff | p-value |
|---|---|---|---|---|---|---|
| Area max | 1 event | 47% | 0.0254 | 1 event | 19% | 0.0951 |
| Number filled seeds | 1 event | 58% | 0.0376 | 1 event | 61% | 0.0313 |
| Total number seeds | 2 events | 41% | <0.191 | 1 event | 28% | 0.0999 |
| Flower per panicle | 1 event | 55% | 0.0177 | 1 event | 26% | 0.0072 |
| Total seed weight | 2 events | 54% | <0.1938 | 1 event | 65% | 0.0252 |
| TKW | | | | 1 event | 6% | 0.1008 |
| Harvest Index | 2 events | 57% | <0.0627 | 1 event | 54% | 0.0086 |

### Transformation of corn, wheat, soybean, canola, alfalfa with sequences useful in the methods of the invention Corn transformation

Transformation of maize (Zea mays) is performed with a modification of the method described by lshida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with Agrobacterium tumefaciens containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Wheat transformation

Transformation of wheat is performed with the method described by lshida et al. (1996) Nature Biotech 14(6): 745-50. The cultivar Bobwhite (available from CIMMYT, Mexico) is commonly used in transformation. Immature embryos are co-cultivated with Agrobacterium tumefaciens containing the expression vector, and transgenic plants are recovered through organogenesis. After incubation with Agrobacterium, the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for in vitro sowing. The hypocotyl, the radicle and one cotyledon are excised from seven-day old young seedlings. The epicotyl and the remaining cotyledon are further grown to develop axillary nodes. These axillary nodes are excised and incubated with Agrobacterium tumefaciens containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

### Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with Agrobacterium (containing the expression vector) by dipping the cut end of the petiole explant into the bacterial suspension. The explants are then cultured for 2 days on MSBAP-3 medium containing 3 mg/I BAP, 3 % sucrose, 0.7 % Phytagar at 23 °C, 16 hr light. After two days of co-cultivation with Agrobacterium, the petiole explants are transferred to MSBAP-3 medium containing 3 mg/l BAP, cefotaxime, carbenicillin, or timentin (300 mg/I) for 7 days, and then cultured on MSBAP-3 medium with cefotaxime, carbenicillin, or timentin and selection agent until shoot regeneration. When the shoots are 5-10 mm in length, they are cut and transferred to shoot elongation medium (MSBAP-0.5, containing 0.5 mg/I BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

### Alfalfa transformation

A regenerating clone of alfalfa (Medicago sativa) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of Agrobacterium tumefaciens C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

## Claims

1. Method for improving abiotic stress tolerance in plants, comprising increasing activity in a plant of a Group I shaggy-like kinase or a homologue thereof, which Group I shaggy-like kinase is one having: (i) at least 77% sequence identity to the amino acid sequence represented by SEQ ID NO: 2; and (ii) motif I: R/H/V/N/Q E/G LK G/N and motif II: K Q/N CXXX G/A/S, where X may be any amino acid.

2. Method according to claim 1, wherein said increased activity is effected by introducing a genetic modification, preferably in the locus of a Group I shaggy-like kinase-encoding gene, said locus being a genomic region which includes the gene of interest and 10KB up- or down stream of the coding region.

3. Method according to claim 2, wherein said genetic modification is effected by any one of: site-directed mutagenesis, homologous recombination, tilling, T-DNA activation and by introducing and expressing in a plant a nucleic acid encoding a Group I shaggy-like kinase or homologue thereof.

4. Method for improving abiotic stress tolerance in plants, comprising introducing and expressing in a plant a Group I shaggy-like kinase-encoding nucleic acid/gene or variant thereof.

5. Method according to claim 4, wherein said variant is a portion of a Group I shaggy-like kinase-encoding nucleic acid/gene and/or is a nucleic acid capable of hybridising with a Group I shaggy-like kinase-encoding nucleic acid/gene, which portion or hybridising sequence is at least 1,200 nucleotides in length, and which portion or hybridising sequence encodes a polypeptide having: (i) at least 77% sequence identity to the amino acid sequence represented by SEQ ID NO: 2; and (ii) having motif l: R/H/V/N/Q E/G LK G/N and motif ll: K Q/N CXXX G/A/S, where X may be any amino acid.

6. Method according to claim 4 or 5, wherein said Group I shaggy-like kinase-encoding nucleic acid/gene or variant thereof is overexpressed in a plant.

7. Method according to any one of claims 4 to 6, wherein said Group I shaggy-like kinase-encoding nucleic acid/gene or variant thereof is of plant origin, preferably from a monocotyledonous plant, further preferably from the family Poaceae, more preferably from the genus Oryza, most preferably from Oryza sativa.

8. Method according to any one of claims 4 to 7, wherein said Group I shaggy-like kinase-encoding nucleic acid/gene or variant thereof is operably linked to a constitutive promoter, preferably to a GOS 2 promoter, such as the GOS2 promoter from rice.

9. Method according to any one of claims 1 to 8, wherein said abiotic stress is any one or more of: water stress, anaerobic stress, salt stress, temperature stress, chemical toxicity stress and oxidative stress.

10. Method according to any one of claims 1 to 9, wherein said abiotic stress is an osmotic stress, selected from salt stress, water stress, oxidative stress, ionic stress.

11. Plant or plant cell obtainable by a method according to any one of claims 1 to 10, which plant or plant cell has increased activity of a Group I shaggy-like kinase and/or increased expression of a Group I shaggy-like kinase-encoding nucleic acid.

12. Construct comprising:
(i) A Group I shaggy-like kinase-encoding nucleic acid or variant thereof;
(ii) one or more control sequences capable of driving expression of the nucleic acid sequence of (i); and optionally
(iii) a transcription termination sequence.

13. Construct according to claim 12, wherein said control sequence comprises a constitutive promoter, preferably the GOS2 promoter, such as the GOS2 promoter from rice.

14. Plant or plant cell transformed with a construct according to claim 12 or 13.

15. Method for the production of transgenic plants having improved tolerance to abiotic stress, which method comprises:
(i) introducing into a plant or plant cell a Group I shaggy-like kinase-encoding nucleic acid or variant thereof; and
(ii) cultivating the plant cell under conditions promoting plant growth and development.

16. Method according to claim 15, wherein said abiotic stress is an osmotic stress, selected from salt stress, water stress, oxidative stress, ionic stress.

17. Transgenic plant having improved tolerance to abiotic stress relative to corresponding wild-type plants, which transgenic plant has increased activity of a Group I shaggy-like kinase and/or increased expression of a Group I shaggy-like kinase-encoding nucleic acid.

18. Transgenic plant according to any one of claims 11, 14 and 17, which transgenic plant is a crop plant such as soybean, sunflower, canola, alfalfa, rapeseed, cotton, tomato, potato or tobacco, further preferably a monocotyledonous plant, such as sugar cane, more preferably a cereal, such as rice, maize, wheat, barley, millet, rye, sorghum or oats.

19. Harvestable parts, including seeds, of transgenic plant according to any one of claims 11, 14, 17 to 18.

20. Use of a Group I shaggy-like kinase-encoding nucleic acid or variant thereof or use of a Group I shaggy-like kinase or homologue thereof in increasing plant tolerance to abiotic stress.

21. Use according to claim 20, wherein said abiotic stress is an osmotic stress, selected from salt stress, water stress, oxidative stress, ionic stress.

22. Use of a Group I shaggy-like kinase-encoding nucleic acid or variant thereof or use of a Group I shaggy-like kinase or homologue thereof as a molecular marker.


SEQUENCE LISTING
<110>  CropDesign N.V.

<120>  Plants having improved growth characteristics and a method for
       making the same

<130>  PF57967

<150>  EP 05110413.1
<151>  2005-11-07

<150>  EP 05110429.7
<151>  2005-11-07

<150>  US 60/736,194
<151>  2005-11-14

<150>  EP 05110900.7
<151>  2005-11-17

<150>  US 60/739,686
<151>  2005-11-23

<150>  EP 05111260.5
<151>  2005-11-24

<150>  US 60/742,287
<151>  2005-12-05

<160>  38

<170>  PatentIn version 3.3

<210>  1
<211>  1592
<212>  DNA
<213>  Oryza sativa

<400>  1
caaaagcaag aggaggaggc cgcggctagc gagcgagcga gagagagggg agaagaagag      60
gtgggacagc cgggagatcc atccctgtgg agaggaggga gggaggaagg aggcgttgga     120
ggaggagagg ttgaccgata gatccattgc ggagttgagt gttgatgcaa agctgattcg     180
ccatcgttta gctttttata agagatgggt tcagtagggg ttgcgccgtc tgggttaaag     240
aacagcagta gcaccagcat gggtgctgag aagttgcctg atcagatgca tgatctgaag     300
ataagggacg ataaggaagt tgaagcgact attattaacg gcaaggaaac agaaaccggc     360
cacataattg tcacaactac tggtggcaga aatggtcagc cgaaacagac agttagctac     420
atggctgagc gtattgtagg gcaaggttca tttgggattg tcttccaggc aaaatgtctg     480
gagacaggtg agacagttgc tatcaagaag gttcttcagg ataagcgcta caagaaccgt     540
gagcttcaga ccatgcgcct tcttgaccac ccaaatgttg tagctctgaa gcactgtttc     600
ttctctacaa ctgagaagga tgaactgtat ctaaacttgg ttcttgagta tgtgcctgaa     660
actgttcatc gtgttgtgaa gcattacaac aagatgaacc agcgtatgcc acttatctat     720
gtgaagctgt atatgtacca gatttgtagg gcattagctt acatccataa tagcatcgga     780
gtttgccaca gagatatcaa gccacagaat cttctggtaa acccacatac ccatcaactc     840
aagctatgtg actttgggag tgcaaaagtt ctggtcaagg gagaaccgaa catatcgtac     900
atttgctccc gatactatag ggctccggag ctcatatttg gtgccaccga atacactaca     960
gctattgaca tctggtctgc tggatgtgtt cttgctgaac ttatgttagg gcagcctctg    1020
tttcctggtg aaagtggtgt agaccaactt gtggaaatca tcaaggtcct tggaacacct    1080
acaagggagg aaattaaatg catgaatcca aactataccg agttcaagtt tccacagatt    1140
aaagcacacc catggcacaa ggtattccat aaaaggttgc ctccagaagc tgttgatctt    1200

gtctctaggc tgctccagta ctcacccaac ctaagatgca ctgctgtgga agcacttgtt 1260
cacccattct ttgatgagct tcgagaccct aatgctcgcc ttccgaatgg ccgctttttg 1320
cctcctctct tcaacttcaa gcctcatgaa ctgaaaggaa tcccatcaga tattatggcg 1380
aaattgatcc cagaacatgt gaagaagcaa tgctcctatg caggagtatg agacagcttc 1440
cgcacgaccc ccctggaaat ttccatgaca agtgcccatt tcctcccccc tggacgacga 1500
tggatcgtca gcatatgcgt gcatgatggt tggtgaggat gtgaagttac gttgttgttg 1560
tgtgaccacc tagagcttga acagaaggaa aa 1592

<210> 2
<211> 408
<212> PRT
<213> Oryza sativa

<400> 2
Met Gly Ser Val Gly Val Ala Pro Ser Gly Leu Lys Asn Ser Ser Ser
1               5                   10                  15
Thr Ser Met Gly Ala Glu Lys Leu Pro Asp Gln Met His Asp Leu Lys
            20                  25                  30
Ile Arg Asp Asp Lys Glu Val Glu Ala Thr Ile Ile Asn Gly Lys Gly
        35                  40                  45
Thr Glu Thr Gly His Ile Ile Val Thr Thr Thr Gly Gly Arg Asn Gly
    50                  55                  60
Gln Pro Lys Gln Thr Val Ser Tyr Met Ala Glu Arg Ile Val Gly Gln
65                  70                  75                  80
Gly Ser Phe Gly Ile Val Phe Gln Ala Lys Cys Leu Glu Thr Gly Glu
                85                  90                  95
Thr Val Ala Ile Lys Lys Val Leu Gln Asp Lys Arg Tyr Lys Asn Arg
            100                 105                 110
Glu Leu Gln Thr Met Arg Leu Leu Asp His Pro Asn Val Val Ala Leu
        115                 120                 125
Lys His Cys Phe Phe Ser Thr Thr Glu Lys Asp Glu Leu Tyr Leu Asn
    130                 135                 140
Leu Val Leu Glu Tyr Val Pro Glu Thr Val His Arg Val Val Lys His
145                 150                 155                 160
Tyr Asn Lys Met Asn Gln Arg Met Pro Leu Ile Tyr Val Lys Leu Tyr
                165                 170                 175
Met Tyr Gln Ile Cys Arg Ala Leu Ala Tyr Ile His Asn Ser Ile Gly
            180                 185                 190
Val Cys His Arg Asp Ile Lys Pro Gln Asn Leu Leu Val Asn Pro His
        195                 200                 205
Thr His Gln Leu Lys Leu Cys Asp Phe Gly Ser Ala Lys Val Leu Val
    210                 215                 220
Lys Gly Glu Pro Asn Ile Ser Tyr Ile Cys Ser Arg Tyr Tyr Arg Ala
225                 230                 235                 240
Pro Glu Leu Ile Phe Gly Ala Thr Glu Tyr Thr Thr Ala Ile Asp Ile
                245                 250                 255
Trp Ser Ala Gly Cys Val Leu Ala Glu Leu Met Leu Gly Gln Pro Leu
            260                 265                 270
Phe Pro Gly Glu Ser Gly Val Asp Gln Leu Val Glu Ile Ile Lys Val
        275                 280                 285
Leu Gly Thr Pro Thr Arg Glu Glu Ile Lys Cys Met Asn Pro Asn Tyr
    290                 295                 300
Thr Glu Phe Lys Phe Pro Gln Ile Lys Ala His Pro Trp His Lys Val
305                 310                 315                 320
Phe His Lys Arg Leu Pro Pro Glu Ala Val Asp Leu Val Ser Arg Leu
                325                 330                 335
Leu Gln Tyr Ser Pro Asn Leu Arg Cys Thr Ala Val Glu Ala Leu Val
            340                 345                 350
His Pro Phe Phe Asp Glu Leu Arg Asp Pro Asn Ala Arg Leu Pro Asn

        355                 360                 365
Gly Arg Phe Leu Pro Pro Leu Phe Asn Phe Lys Pro His Glu Leu Lys
    370                 375                 380
Gly Ile Pro Ser Asp Ile Met Ala Lys Leu Ile Pro Glu His Val Lys
385                 390                 395                 400
Lys Gln Cys Ser Tyr Ala Gly Val
                405

<210>  3
<211>  1800
<212>  DNA
<213>  Oryza sativa

<400>  3
ctctccgaat cctcccccgc attccgcgcc ggagctgagg agggacagcg agccagcgag      60
ggaggtgggg caatccagcg agcgccagat cgtgccgcca gccccgctcc gtcctctctc    120
cggcgaccct ccgccccgcc tgatcccgtg gtggatcaac tacaactcat ctttaggaaa    180
gcctcttgaa cattagcctt gtgtgccttt caacagtatg gcctcggtag gtgtggtgcg    240
ttcttccttg gggtttcaga acgaaacaag tacaagtggc gatgccgacc gacttccgaa    300
tgagatgagc aatatgagta taaggatgat taataaggac attgacgaca tagttgtcaa    360
tggcaatggg acagaacctg gtcatgttat tgtgacaagc attgatggaa gaaatggaca    420
ggctaaacag accattagct acatggccga gcgtgtcgta ggtcatggat cctttggaac    480
tgttttccag gccaagtgcc tggaaaccgg tgagactgtg gctataaaga aggttcttca    540
agacaagaga tataagaacc gtgagctgca aacaatgcga gttcttgacc acccaaatgt    600
ggtgtcacta aagcattgtt ttttctcaaa gactgagaag gaggagcttt acctcaattt    660
ggtgcttgag tatgtgccag agactgctca ccgtgtaatt aaacattaca acaagatgaa    720
ccaacgcatg ccattgatat atgcaaaact atacatgtat cagatatgca gagctttggc    780
gtacattcac aacaccattg gcgtgtgtca cagggatatc aagccacaaa atcttctggt    840
gaacccacat actcatcagc tgaaattatg tgacttcggc agcgcgaaag tcttggtaaa    900
aggagaacca aatatttctt acatctgttc aaggtactac agagctccag agctcatatt    960
tggtgctact gaatacacaa cagcgattga tgtttggtct gccggttgtg tgcttgctga   1020
actcctcctg gggcagcctc tattccctgg cgacagtggc gttgatcagc ttgttgaaat   1080
catcaaggtt ctgggtaccc ctacacgaga ggagattaag tgcatgaacc caaattacac   1140
cgagtttaaa tttccgcaaa tcaaagctca cccatggcac aagatcttcc ataaaagaat   1200
gcctgctgaa gcagtcgatc tcgtctccag gcttttgcag tattcaccat acctccggtc   1260
cactgcttcg gaagcattga tccatccctt cttcgatgaa ctccgtgatc caaacacccg   1320
cttaccgaac ggccgtttcc ttcctcctct cttcaacttc aagccccatg aactgaaggg   1380
tatgccaatg gaattcctgg tgaagcttat ccccgaacat gctcgaaagc aatgcgcgtt   1440
tgtaggatgg tgatttctga ggtcagcatg aaaactagtt cagaatttct tcaccgtcct   1500
ccattagaaa gcagagatga accctgtgtg cagccatttg ggaaagctgg tgcatatgga   1560
agtggaacta catttttttg tccgagattc tgacgccgcg tattcttttc ccccctccca   1620
ctttgctgct gccggtgtaa ccaaaaaatc atccacggtt ctgtaaagtt gatgaagaag   1680
agtgtaaaat caggttgaaa actgaattcg atcggtttgt caagattgta gcaacatgca   1740
aggaaggatg ttgcacactt tgtatggcaa tgttcgttcg gtccaaatat ttggacatgg   1800

<210>  4
<211>  411
<212>  PRT
<213>  Oryza sativa

<400>  4
Met Ala Ser Val Gly Val Val Arg Ser Ser Leu Gly Phe Gln Asn Glu
1               5                   10                  15
Thr Ser Thr Ser Gly Asp Ala Asp Arg Leu Pro Asn Glu Met Ser Asn
            20                  25                  30
Met Ser Ile Arg Asp Asp Asn Lys Asp Ile Asp Asp Ile Val Val Asn
        35                  40                  45
Gly Asn Gly Thr Glu Pro Gly His Val Ile Val Thr Ser Ile Asp Gly
    50                  55                  60

Arg Asn Gly Gln Ala Lys Gln Thr Ile Ser Tyr Met Ala Glu Arg Val
65                  70                  75                  80
Val Gly His Gly Ser Phe Gly Thr Val Phe Gln Ala Lys Cys Leu Glu
                85                  90                  95
Thr Gly Glu Thr Val Ala Ile Lys Lys Val Leu Gln Asp Lys Arg Tyr
            100                 105                 110
Lys Asn Arg Glu Leu Gln Thr Met Arg Val Leu Asp His Pro Asn Val
        115                 120                 125
Val Ser Leu Lys His Cys Phe Phe Ser Lys Thr Glu Lys Glu Glu Leu
    130                 135                 140
Tyr Leu Asn Leu Val Leu Glu Tyr Val Pro Glu Thr Ala His Arg Val
145                 150                 155                 160
Ile Lys His Tyr Asn Lys Met Asn Gln Arg Met Pro Leu Ile Tyr Ala
                165                 170                 175
Lys Leu Tyr Met Tyr Gln Ile Cys Arg Ala Leu Ala Tyr Ile His Asn
            180                 185                 190
Thr Ile Gly Val Cys His Arg Asp Ile Lys Pro Gln Asn Leu Leu Val
        195                 200                 205
Asn Pro His Thr His Gln Leu Lys Leu Cys Asp Phe Gly Ser Ala Lys
    210                 215                 220
Val Leu Val Lys Gly Glu Pro Asn Ile Ser Tyr Ile Cys Ser Arg Tyr
225                 230                 235                 240
Tyr Arg Ala Pro Glu Leu Ile Phe Gly Ala Thr Glu Tyr Thr Thr Ala
                245                 250                 255
Ile Asp Val Trp Ser Ala Gly Cys Val Leu Ala Glu Leu Leu Leu Gly
            260                 265                 270
Gln Pro Leu Phe Pro Gly Asp Ser Gly Val Asp Gln Leu Val Glu Ile
        275                 280                 285
Ile Lys Val Leu Gly Thr Pro Thr Arg Glu Glu Ile Lys Cys Met Asn
    290                 295                 300
Pro Asn Tyr Thr Glu Phe Lys Phe Pro Gln Ile Lys Ala His Pro Trp
305                 310                 315                 320
His Lys Ile Phe His Lys Arg Met Pro Ala Glu Ala Val Asp Leu Val
                325                 330                 335
Ser Arg Leu Leu Gln Tyr Ser Pro Tyr Leu Arg Ser Thr Ala Ser Glu
            340                 345                 350
Ala Leu Ile His Pro Phe Phe Asp Glu Leu Arg Asp Pro Asn Thr Arg
        355                 360                 365
Leu Pro Asn Gly Arg Phe Leu Pro Pro Leu Phe Asn Phe Lys Pro His
    370                 375                 380
Glu Leu Lys Gly Met Pro Met Glu Phe Leu Val Lys Leu Ile Pro Glu
385                 390                 395                 400
His Ala Arg Lys Gln Cys Ala Phe Val Gly Trp
                405                 410

<210>  5
<211>  2261
<212>  DNA
<213>  Oryza sativa

<400>  5
ttggggcgga gagacgggag agagaggaga ggagtcgaac tagggttagt gaggtgaagg      60
ggggcgattt tgctcatcgc cctccgccta gatcgaatcc gaatcccgcc tcctcctcct     120
cctcctcctc cccccgcgcc gatcttgccc cgctccctcg ccgcagctgc gggcttgccc     180
tagcccgccg catcgccgac gtactacgcc cgctcggctc caggctccag atccggcctc     240
cagggcgtgc ccgctgctgg acggcgctct cccgcgtccg tggctacctc ctcctccggg     300
tttgcgttgc cgtcgccgaa gcgatccgtt ggtggattgt gtgtagctct gtaacaaagg     360
atcttggtcc ataggagtga accagaaggt acaaaacttc ttggctatta tgacatcggt     420
gggtctagta gattcttcat caggtttccc ggaaacgagt actagtggag ccactgatcg     480

tctgacagat gatatcagcg agatgagcat aagagataag gaagttgaag ctgtagtggt 540
cagtggcaat agtatggaca taggtcacac tattgtgaca actgttggtg gaagaaatgg 600
gcagccaaag cagacaatta gttacatcgc agagcgtgct gttgggcgtg gttcatttgg 660
agttgtcttc caggctaagt gtctagaaac tggtgagaga gttgctgtaa agaaagttct 720
tcaagatgcg agatacaaga accgggagct gcaaacgatg caagttctcg atcacccaaa 780
tgttgcatgc ttgaagcatt acttttgctc gactactgcg aaggaggagc tatacctcaa 840
cttggttctt gaatatgtgc cagaaactgt tcatcgtgtt attagacatt acaataagat 900
gagccaacgc atgccattga tttatgttaa actttatatg taccagattt gtagggcttt 960
ggcatacatt cataactgtg tgggagtatg ccacagagat ataaagccac agaatatatt 1020
ggtgaatcca cataatcatc agttgaaatt gtgtgacttt ggcagtgcaa aagtcctggt 1080
gaaaggggaa ccaaatattt catatatctg ttctagatac tacagagccc cagcactcat 1140
atttggggct acagagtaca cgacagctat tgatgtgtgg tctgctggct gtgttcttgc 1200
tgaactactt ctagggcagc ctgtttttcc tggtgacagt ggcgtcgacc aacttgttga 1260
aattatcaag gtactgggta ctccaacaag ggaagaaatc aaacatatga atccgaacta 1320
cactgagttt aagttccccc aaatcaaagc tcacccatgg cacaagatct ttcataaaag 1380
aatgccatct gaagctgtag atcttgtgtc tcggcttctg cagtactcac cacacctccg 1440
gtgcagtgca ttggaggtgt taattcatcc attcttcgat gaactacgag atccaaatgc 1500
tcgcttacca aatggccgta ctcttccccc actattcaat ttcaagcctc gtgagctgaa 1560
aggagcatca atggaatttc ttgtgaagtt ggttccacag catgctaaga agcaatgtgc 1620
cttcttagga ttatgagcag acagttcata cactaatcgg gtattacgtt ggtctacttg 1680
gttccgtaga acagcatggc ctgcagtatt attgaatggg ccctaatttt gttctacgtg 1740
caccctgctt gggcatgcct tctaccatca tattctgtaa agcagatgaa gacatggagt 1800
gagtggttat tgattggggc ttgtatattg gtctgccctt gacttgagaa ctttattcac 1860
tctgctgtat caagcaagaa agaacacgta tgtatgcttg agccatattt cattccagag 1920
cctctatcag gtttcaccga ccatcagatt taagtcttcc agtttgtagg gctaagccaa 1980
aaaagttcta tgtgtgaaga gttggagaca tctgtttggt tacaagctat ttgattgtat 2040
ttgatcattc ttttgtatta acactctgat aaacttacat gagttaattc tattgttatg 2100
tgatatagtt gtgtgccact tttaaacacc taaacaactc tgttcaatgt tgaaatgttt 2160
ggcatgtatc aacatatcac ctcattattt cgaggttgat tttgtacacc tgatatctgt 2220
tagcttgaat gcttgattac aagcaaatga ttaaatattt t 2261

<210> 6
<211> 408
<212> PRT
<213> Oryza sativa

<400> 6
Met Thr Ser Val Gly Leu Val Asp Ser Ser Ser Gly Phe Pro Glu Thr
1               5                   10                  15
Ser Thr Ser Gly Ala Thr Asp Arg Leu Thr Asp Asp Ile Ser Glu Met
            20                  25                  30
Ser Ile Arg Asp Lys Glu Val Glu Ala Val Val Val Ser Gly Asn Ser
        35                  40                  45
Met Asp Ile Gly His Thr Ile Val Thr Thr Val Gly Gly Arg Asn Gly
    50                  55                  60
Gln Pro Lys Gln Thr Ile Ser Tyr Ile Ala Glu Arg Ala Val Gly Arg
65                  70                  75                  80
Gly Ser Phe Gly Val Val Phe Gln Ala Lys Cys Leu Glu Thr Gly Glu
                85                  90                  95
Arg Val Ala Val Lys Lys Val Leu Gln Asp Ala Arg Tyr Lys Asn Arg
            100                 105                 110
Glu Leu Gln Thr Met Gln Val Leu Asp His Pro Asn Val Ala Cys Leu
        115                 120                 125
Lys His Tyr Phe Cys Ser Thr Thr Ala Lys Glu Glu Leu Tyr Leu Asn
    130                 135                 140
Leu Val Leu Glu Tyr Val Pro Glu Thr Val His Arg Val Ile Arg His
145                 150                 155                 160
Tyr Asn Lys Met Ser Gln Arg Met Pro Leu Ile Tyr Val Lys Leu Tyr
                165                 170                 175

Met Tyr Gln Ile Cys Arg Ala Leu Ala Tyr Ile His Asn Cys Val Gly
            180                 185                 190
Val Cys His Arg Asp Ile Lys Pro Gln Asn Ile Leu Val Asn Pro His
        195                 200                 205
Asn His Gln Leu Lys Leu Cys Asp Phe Gly Ser Ala Lys Val Leu Val
    210                 215                 220
Lys Gly Glu Pro Asn Ile Ser Tyr Ile Cys Ser Arg Tyr Tyr Arg Ala
225                 230                 235                 240
Pro Ala Leu Ile Phe Gly Ala Thr Glu Tyr Thr Thr Ala Ile Asp Val
                245                 250                 255
Trp Ser Ala Gly Cys Val Leu Ala Glu Leu Leu Leu Gly Gln Pro Val
            260                 265                 270
Phe Pro Gly Asp Ser Gly Val Asp Gln Leu Val Glu Ile Ile Lys Val
        275                 280                 285
Leu Gly Thr Pro Thr Arg Glu Glu Ile Lys His Met Asn Pro Asn Tyr
    290                 295                 300
Thr Glu Phe Lys Phe Pro Gln Ile Lys Ala His Pro Trp His Lys Ile
305                 310                 315                 320
Phe His Lys Arg Met Pro Ser Glu Ala Val Asp Leu Val Ser Arg Leu
                325                 330                 335
Leu Gln Tyr Ser Pro His Leu Arg Cys Ser Ala Leu Glu Val Leu Ile
            340                 345                 350
His Pro Phe Phe Asp Glu Leu Arg Asp Pro Asn Ala Arg Leu Pro Asn
        355                 360                 365
Gly Arg Thr Leu Pro Pro Leu Phe Asn Phe Lys Pro Arg Glu Leu Lys
    370                 375                 380
Gly Ala Ser Met Glu Phe Leu Val Lys Leu Val Pro Gln His Ala Lys
385                 390                 395                 400
Lys Gln Cys Ala Phe Leu Gly Leu
                405

<210>  7
<211>  1634
<212>  DNA
<213>  Arabidopsis thaliana

<400>  7
acaaaataat ataaagtaaa aatatttcat aattcgtctc tcaccggaaa aataaataaa      60
gttagagaga gagattcata atcatcaaac ccacaaatcc tttttttttt tttttgttaa     120
aacccacaaa tccttactcg tacggatctc tctctcttta atatcctaat tcttctcttc     180
atcgatctga gatttttacg ttttcatcgg cttgaaagtt tgaagagttt tgtagcctga     240
aaaatggcgt cagtgggtat agctcctaat cctggagcaa gagactctac tggtgttgat     300
aaattgcctg aagaaatgaa tgacatgaaa attcgtgacg ataaagaaat ggaagcgaca     360
gtggtagatg gaaatggaac agagactgga catatcattg tgactactat tggtggtaga     420
aatggccaac caaaacagac aattagctac atggctgagc gtgttgttgg tcacggatct     480
tttggtgttg tgttccaagc gaaatgtctt gagacaggag aaactgttgc gataaagaaa     540
gttttacaag ataggaggta caagaaccgt gagcttcaaa ccatgaggct acttgaccat     600
cctaatgttg tgtctctgaa acattgtttc ttctcaacca ctgaaaaaga tgagctttac     660
ctcaatcttg ttcttgagta cgttccagaa actgttcatc gtgttatcaa acactacaac     720
aaactgaatc agagaatgcc tcttatatac gtcaaacttt acacttatca gatttttaga     780
gccttatctt acattcaccg atgcattggt gtgtgtcatc gtgacataaa acctcaaaac     840
ttgttggtaa atccgcacac tcatcaagta aagctatgtg attttggaag tgcaaaagta     900
ttggtaaaag gagaaccaaa catttcctac atctgctcga ggtattacag agcacctgaa     960
cttatttttg gagcaaccga gtatacgaca gccattgatg tctggtctgc aggatgtgtt    1020
ctagctgaac tattgcttgg acagcccttg ttccctggtg agagcggtgt tgatcaactt    1080
gtagagatta tcaaggtctt gggaacgcct actagagaag aaatcaagtg catgaaccca    1140
aactacacgg aattcaaatt ccctcagatt aaagctcatc catggcacaa gattttccac    1200
aaacgcatgc ctccagaagc tgttgatttg gtctcaagac ttcttcaata ctctcctaat    1260
ctacgaagtg ccgctctcga cacattagtc cacccattct ttgatgagtt aagagaccca    1320

aacgcacgtc tacctaatgg acgtttcctt ccaccgcttt tcaacttcaa gcctcacgag 1380
ctgaaaggtg taccattgga gatggtagct aagttagtac ctgagcatgc aaggaagcag 1440
tgtccttggc tcggtttgtg atttcctctt aatgtagcat gaacacaaca aacacttctt 1500
ataaattacc tctctatgta tcaatatgtc acaaactgat atgcaccctt tgtttgttgt 1560
atgagtagag aaaaaaagag ttattactat ggttggttgg ttcataatgt aaaagcccac 1620
caagattttt tatc 1634

<210> 8
<211> 405
<212> PRT
<213> Arabidopsis thaliana

<400> 8
Met Ala Ser Val Gly Ile Ala Pro Asn Pro Gly Ala Arg Asp Ser Thr
1               5                   10                  15
Gly Val Asp Lys Leu Pro Glu Glu Met Asn Asp Met Lys Ile Arg Asp
            20                  25                  30
Asp Lys Glu Met Glu Ala Thr Val Val Asp Gly Asn Gly Thr Glu Thr
        35                  40                  45
Gly His Ile Ile Val Thr Thr Ile Gly Gly Arg Asn Gly Gln Pro Lys
    50                  55                  60
Gln Thr Ile Ser Tyr Met Ala Glu Arg Val Val Gly His Gly Ser Phe
65                  70                  75                  80
Gly Val Val Phe Gln Ala Lys Cys Leu Glu Thr Gly Glu Thr Val Ala
                85                  90                  95
Ile Lys Lys Val Leu Gln Asp Arg Arg Tyr Lys Asn Arg Glu Leu Gln
            100                 105                 110
Thr Met Arg Leu Leu Asp His Pro Asn Val Val Ser Leu Lys His Cys
        115                 120                 125
Phe Phe Ser Thr Thr Glu Lys Asp Glu Leu Tyr Leu Asn Leu Val Leu
    130                 135                 140
Glu Tyr Val Pro Glu Thr Val His Arg Val Ile Lys His Tyr Asn Lys
145                 150                 155                 160
Leu Asn Gln Arg Met Pro Leu Ile Tyr Val Lys Leu Tyr Thr Tyr Gln
                165                 170                 175
Ile Phe Arg Ala Leu Ser Tyr Ile His Arg Cys Ile Gly Val Cys His
            180                 185                 190
Arg Asp Ile Lys Pro Gln Asn Leu Leu Val Asn Pro His Thr His Gln
        195                 200                 205
Val Lys Leu Cys Asp Phe Gly Ser Ala Lys Val Leu Val Lys Gly Glu
    210                 215                 220
Pro Asn Ile Ser Tyr Ile Cys Ser Arg Tyr Tyr Arg Ala Pro Glu Leu
225                 230                 235                 240
Ile Phe Gly Ala Thr Glu Tyr Thr Thr Ala Ile Asp Val Trp Ser Ala
                245                 250                 255
Gly Cys Val Leu Ala Glu Leu Leu Leu Gly Gln Pro Leu Phe Pro Gly
            260                 265                 270
Glu Ser Gly Val Asp Gln Leu Val Glu Ile Ile Lys Val Leu Gly Thr
        275                 280                 285
Pro Thr Arg Glu Glu Ile Lys Cys Met Asn Pro Asn Tyr Thr Glu Phe
    290                 295                 300
Lys Phe Pro Gln Ile Lys Ala His Pro Trp His Lys Ile Phe His Lys
305                 310                 315                 320
Arg Met Pro Pro Glu Ala Val Asp Leu Val Ser Arg Leu Leu Gln Tyr
                325                 330                 335
Ser Pro Asn Leu Arg Ser Ala Ala Leu Asp Thr Leu Val His Pro Phe
            340                 345                 350
Phe Asp Glu Leu Arg Asp Pro Asn Ala Arg Leu Pro Asn Gly Arg Phe
        355                 360                 365

Leu Pro Pro Leu Phe Asn Phe Lys Pro His Glu Leu Lys Gly Val Pro
    370                 375                 380
Leu Glu Met Val Ala Lys Leu Val Pro Glu His Ala Arg Lys Gln Cys
385                 390                 395                 400
Pro Trp Leu Gly Leu
                405

<210>  9
<211>  1669
<212>  DNA
<213>  Arabidopsis thaliana

<400>  9
cagaccaaca acaaaaagga gataaagaga agaggattca tcatcatcaa tcaatccttc      60
attttatgga tctactcata tcttgattct tccttctatc tctccctttt cttccatctc     120
tttttctctg ggtttccccg atttgaagag cgtgacaaag gaagaatctt ttattaaaac     180
aaattcttct gttttaatct tgggatggcc tcggtgggca tagagcctag tgccgcggtt     240
agagaatcta ctggaaacgt tactgatgct gatagattac ccgaggagat gaaggacatg     300
aaaattcaag atgataaaga aatggaagct acgattgtta atggcaatgt gactgagact     360
ggccatataa tagtaactac tataggagga agaaatggcc agccaaaaca gacaatcagt     420
tacatggcgg agcgagttgt tggacatggc tcctttggtg ttgtgtttca ggccaaatgt     480
ttagaaacag gagaaactgt tgctataaag aaagttctac aagatcggag gtacaagaat     540
cgtgagcttc aaacaatgag gctacttgac catccaaatg ttgtgtcttt gaaacattgt     600
ttcttctcta caaccgaaaa agatgagctt tatctcaact tggttctgga atacgttccg     660
gaaactgtgc accgcgtcat caaacactac aacaaactta accaacgaat gcctctcgtt     720
tacgtcaaac tttacactta tcagattttt aggtccttat cctacattca ccgatgtatc     780
ggcgtatgtc atcgagacat caaacctcaa aacttgttgg taaatccaca cactcatcaa     840
gtgaaactat gcgattttgg aagtgcgaaa gtattggtta aaggagagcc aaacatttca     900
tacatttgct cgaggtatta cagagcacct gagctcattt ttggagccac cgagtatact     960
acagccattg atgtctggtc tgcaggatgt gttctcgccg agcttcttct cgggcagcca    1020
ttgttcccgg gtgagagcgg tgttgatcaa cttgtagaga ttataaaggt tttgggaaca    1080
ccaacaaggg aagaaatcaa atgcatgaac ccgaattaca cagagttcaa atttcctcag    1140
attaaagctc atccatggca taagattttc cacaagagaa tgcctccaga agctgttgat    1200
ttggtctcaa ggcttcttca atactctccc aatctccgtt gtgctgctct tgattcattg    1260
gtccacccat tctttgacga gctaagagat ccgaatgcgc gattacccaa cggacgtttc    1320
cttccaccgc tctttaactt taagcctcat gaacttaaag gtgtgcctgt ggagatggtg    1380
gcgaagttag ttccagaaca tgcgaggaag caatgtccgt ggctcagttt gtgatttgtt    1440
ctcacctgca aacacgaaaa ctagagcaaa gcagtcgaga tattcatctc ttctcttctc    1500
tctccttctc tgtattaata ttattataat gatcatatct caatctgatg atttagtaac    1560
cctttgtttg ttgtatgagt agagaaagag tgaatcattt gtgggggtta tgatattgta    1620
taagccaaca aagattattt tttaaagaga gtttcgtgtt ttctgtctc                1669

<210>  10
<211>  409
<212>  PRT
<213>  Arabidopsis thaliana

<400>  10
Met Ala Ser Val Gly Ile Glu Pro Ser Ala Ala Val Arg Glu Ser Thr
1               5                   10                  15
Gly Asn Val Thr Asp Ala Asp Arg Leu Pro Glu Glu Met Lys Asp Met
            20                  25                  30
Lys Ile Gln Asp Asp Lys Glu Met Glu Ala Thr Ile Val Asn Gly Asn
        35                  40                  45
Val Thr Glu Thr Gly His Ile Ile Val Thr Thr Ile Gly Gly Arg Asn
    50                  55                  60
Gly Gln Pro Lys Gln Thr Ile Ser Tyr Met Ala Glu Arg Val Val Gly
65                  70                  75                  80
His Gly Ser Phe Gly Val Val Phe Gln Ala Lys Cys Leu Glu Thr Gly

                85                  90                  95
Glu Thr Val Ala Ile Lys Lys Val Leu Gln Asp Arg Arg Tyr Lys Asn
                100                 105                 110
Arg Glu Leu Gln Thr Met Arg Leu Leu Asp His Pro Asn Val Val Ser
        115                 120                 125
Leu Lys His Cys Phe Phe Ser Thr Thr Glu Lys Asp Glu Leu Tyr Leu
    130                 135                 140
Asn Leu Val Leu Glu Tyr Val Pro Glu Thr Val His Arg Val Ile Lys
145                 150                 155                 160
His Tyr Asn Lys Leu Asn Gln Arg Met Pro Leu Val Tyr Val Lys Leu
                165                 170                 175
Tyr Thr Tyr Gln Ile Phe Arg Ser Leu Ser Tyr Ile His Arg Cys Ile
            180                 185                 190
Gly Val Cys His Arg Asp Ile Lys Pro Gln Asn Leu Leu Val Asn Pro
        195                 200                 205
His Thr His Gln Val Lys Leu Cys Asp Phe Gly Ser Ala Lys Val Leu
    210                 215                 220
Val Lys Gly Glu Pro Asn Ile Ser Tyr Ile Cys Ser Arg Tyr Tyr Arg
225                 230                 235                 240
Ala Pro Glu Leu Ile Phe Gly Ala Thr Glu Tyr Thr Thr Ala Ile Asp
                245                 250                 255
Val Trp Ser Ala Gly Cys Val Leu Ala Glu Leu Leu Leu Gly Gln Pro
            260                 265                 270
Leu Phe Pro Gly Glu Ser Gly Val Asp Gln Leu Val Glu Ile Ile Lys
        275                 280                 285
Val Leu Gly Thr Pro Thr Arg Glu Glu Ile Lys Cys Met Asn Pro Asn
    290                 295                 300
Tyr Thr Glu Phe Lys Phe Pro Gln Ile Lys Ala His Pro Trp His Lys
305                 310                 315                 320
Ile Phe His Lys Arg Met Pro Pro Glu Ala Val Asp Leu Val Ser Arg
                325                 330                 335
Leu Leu Gln Tyr Ser Pro Asn Leu Arg Cys Ala Ala Leu Asp Ser Leu
            340                 345                 350
Val His Pro Phe Phe Asp Glu Leu Arg Asp Pro Asn Ala Arg Leu Pro
        355                 360                 365
Asn Gly Arg Phe Leu Pro Pro Leu Phe Asn Phe Lys Pro His Glu Leu
    370                 375                 380
Lys Gly Val Pro Val Glu Met Val Ala Lys Leu Val Pro Glu His Ala
385                 390                 395                 400
Arg Lys Gln Cys Pro Trp Leu Ser Leu
                405

<210>  11
<211>  1496
<212>  DNA
<213>  Arabidopsis thaliana

<400>  11
ctcagatcga tgaagagaag aattaggatt tttacgtttt catcggcttg aaagtttgaa      60
gagttttgta gcctgaaaaa tggcgtcagt gggtatagct cctaatcctg gagcaagaga     120
ctctactggt gttgataaat tgcctgaaga aatgaatgac atgaaaattc gtgacgataa     180
agaaatggaa gcgacagtgg tagatggaaa tggaacagag actggacata tcattgtgac     240
tactattggt ggtagaaatg gccaaccaaa acagacaatt agctacatgg ctgagcgtgt     300
tgttggtcac ggatcttttg gtgttgtgtt ccaagcgaaa tgtcttgaga caggagaaac     360
tgttgcgata aagaaagttt tacaagatag gaggtacaag aaccgtgagc ttcaaaccat     420
gaggctactt gaccatccta atgttgtgtc tctgaaacat tgtttcttct caaccactga     480
aaaagatgag ctttacctca atcttgttct tgagtacgtt ccagaaactg ttcatcgtgt     540
tatcaaacac tacaacaaac tgaatcagag aatgcctctt atatacgtca aactttacac     600
ttatcagatt tttagagcct tatcttacat tcaccgatgc attggtgtgt gtcatcgtga     660

cataaaacct caaaacttgt tggtaaatcc gcacactcat caagtaaagc tatgtgattt    720
tggaagtgca aaagtattgg taaaaggaga accaaacatt tcctacatct gctcgaggta    780
ttacagagca cctgaactta tttttggagc aaccgagtat acgacagcca ttgatgtctg    840
gtctgcagga tgtgttctag ctgaactatt gcttggacag cccttgttcc ctggtgagag    900
cggtgttgat caacttgtac acattatcaa ggtcttggga acgcctacta gagaagaaat    960
caagtgcatg aacccaaact acacggaatt caaattccct cagattaaag ctcatccatg   1020
gcacaagatt ttccacaaac gcatgcctcc agaagctgtt gatttggtct caagacttct   1080
tcaatactct cctaatctac gaagtgccgc tctcgacaca ttagtccacc cattctttga   1140
tgagttaaga gacccaaacg cacgtctacc taatggacgt ttccttccac cggcttttca   1200
cttcaagcct cacgagctga aaggtgtacc attggagatg gtagctaagt tagtacctga   1260
gcatgcaagg aagcagtgtc cttggctcgg tttgtgattt cctcttaatg tagcatgaac   1320
acaacaaaca cttcttataa attacctctc tatgtatcaa tatgtcacaa actgatatgc   1380
accctttgtt tgttgtatga gtagagaaaa aaagagttat tactatggtt ggttggttca   1440
taatgtaaaa gcccaccaag attttttatc tagataaaga gtttgctaaa aaaaaa        1496

<210>  12
<211>  405
<212>  PRT
<213>  Arabidopsis thaliana

<400>  12
Met Ala Ser Val Gly Ile Ala Pro Asn Pro Gly Ala Arg Asp Ser Thr
1               5                   10                  15
Gly Val Asp Lys Leu Pro Glu Glu Met Asn Asp Met Lys Ile Arg Asp
            20                  25                  30
Asp Lys Glu Met Glu Ala Thr Val Val Asp Gly Asn Gly Thr Glu Thr
        35                  40                  45
Gly His Ile Ile Val Thr Thr Ile Gly Gly Arg Asn Gly Gln Pro Lys
    50                  55                  60
Gln Thr Ile Ser Tyr Met Ala Glu Arg Val Val Gly His Gly Ser Phe
65                  70                  75                  80
Gly Val Val Phe Gln Ala Lys Cys Leu Glu Thr Gly Glu Thr Val Ala
                85                  90                  95
Ile Lys Lys Val Leu Gln Asp Arg Arg Tyr Lys Asn Arg Glu Leu Gln
            100                 105                 110
Thr Met Arg Leu Leu Asp His Pro Asn Val Val Ser Leu Lys His Cys
        115                 120                 125
Phe Phe Ser Thr Thr Glu Lys Asp Glu Leu Tyr Leu Asn Leu Val Leu
    130                 135                 140
Glu Tyr Val Pro Glu Thr Val His Arg Val Ile Lys His Tyr Asn Lys
145                 150                 155                 160
Leu Asn Gln Arg Met Pro Leu Ile Tyr Val Lys Leu Tyr Thr Tyr Gln
                165                 170                 175
Ile Phe Arg Ala Leu Ser Tyr Ile His Arg Cys Ile Gly Val Cys His
            180                 185                 190
Arg Asp Ile Lys Pro Gln Asn Leu Leu Val Asn Pro His Thr His Gln
        195                 200                 205
Val Lys Leu Cys Asp Phe Gly Ser Ala Lys Val Leu Val Lys Gly Glu
    210                 215                 220
Pro Asn Ile Ser Tyr Ile Cys Ser Arg Tyr Tyr Arg Ala Pro Glu Leu
225                 230                 235                 240
Ile Phe Gly Ala Thr Glu Tyr Thr Thr Ala Ile Asp Val Trp Ser Ala
                245                 250                 255
Gly Cys Val Leu Ala Glu Leu Leu Leu Gly Gln Pro Leu Phe Pro Gly
            260                 265                 270
Glu Ser Gly Val Asp Gln Leu Val His Ile Ile Lys Val Leu Gly Thr
        275                 280                 285
Pro Thr Arg Glu Glu Ile Lys Cys Met Asn Pro Asn Tyr Thr Glu Phe
    290                 295                 300

Lys Phe Pro Gln Ile Lys Ala His Pro Trp His Lys Ile Phe His Lys
305                 310                 315                 320
Arg Met Pro Pro Glu Ala Val Asp Leu Val Ser Arg Leu Leu Gln Tyr
                325                 330                 335
Ser Pro Asn Leu Arg Ser Ala Ala Leu Asp Thr Leu Val His Pro Phe
            340                 345                 350
Phe Asp Glu Leu Arg Asp Pro Asn Ala Arg Leu Pro Asn Gly Arg Phe
        355                 360                 365
Leu Pro Pro Ala Phe His Phe Lys Pro His Glu Leu Lys Gly Val Pro
    370                 375                 380
Leu Glu Met Val Ala Lys Leu Val Pro Glu His Ala Arg Lys Gln Cys
385                 390                 395                 400
Pro Trp Leu Gly Leu
                405

<210>  13
<211>  1712
<212>  DNA
<213>  Arabidopsis thaliana

<400>  13
attggggtcc gctctctctc tctctctgcc atcagaagaa aaaaccaaac accgagacaa      60
aacacatcac gaagaagaga gagatttgcc attttcggca aggaagtgtt ttattctagg     120
gcttctctag ttgctgcgta gacaagatgg tggagtgaaa ttgcggcggt gacttttcag     180
attgacaaat tgaattaaag atcttgaata catatggctt ctgtgggaac attaccagct     240
tcttctatgg ctacaaaaca aagcaatgct tctatatgcg ctgaaaaatt acctgaaggg     300
attaatgaga tgaagataaa agatgataag gaaatggaag cagctgtggt tgatgggaat     360
ggaactgaaa caggtcacat tattgttaca actattggtg gtaaaaatgg tcagcctaaa     420
cagaccataa gttatatggc agagcgcatc gttggacaag gttcttttgg aatcgttttt     480
caggccaagt gtttagaaac gggggaaact gttgcaatca agaaagtttt gcaagacaag     540
agatacaaga atcgggagct gcagacaatg cgtcttcttg atcaccctaa tgttgtatcc     600
ctcaagcatt gttttttctc aacgactgaa aaagatgagc tctatctcaa tctggtcctt     660
gaatacgttc ctgagactgt ctatcgcgtc tcaaagcact atagtcgggc aaaccagagg     720
atgcccatta tatatgttaa actctatact tatcagattt gcagagcttt ggcttatatt     780
catggtggag taggagtctg ccacagagac ataaaaccac agaatcttct ggttaatcct     840
catacgcatc aggtcaaact atgcgatttt ggtagcgcaa aagttctggt taaaggcgag     900
ccaaacatct catacatctg ctcccgttac tatcgagcac ctgaacttat atttggagct     960
acagaatata caacaacaat tgacatatgg tctgcaggct gtgttcttgc tgaattgctt    1020
ctgggacagc ctctatttcc aggtgagagt ggagttgacc agctagttga gataataaag    1080
gttcttggaa caccaacacg ggaggaaatc aaatgcatga atccaaacta cacagaattc    1140
aaattcccgc aaataaaggc tcatccttgg cacaaaatat tccataagcg tacacctcca    1200
gaagctgtag accttgtctc aagacttctc cagtattctc caaacctcag atcaaccgct    1260
atggaggcga tagttcaccc gttcttcgat gagctacgtg atcccaatac acgtcttcct    1320
aatggtcgtg ccttgcctcc tctcttcaac tttaaacctc aagagctaaa aggagcaagt    1380
ttagagttgt tgtccaagct tatacctgac cacgcccgaa aacaatgttc cttcctcgct    1440
ctctaaatct cttcctctct ctctatatat atgtgtgtgt gtgtgtatgt acacatgcat    1500
ataatatgct tatcgtttct aagtaatgga gatagcttct caggattatc attagctttc    1560
atctttcatg tatctttgtt gtttattgtc ttatcacaac ctttgtactt tattacatac    1620
aatgattagt gtaatgtatg tgacggtctt tgactcgccg gtcgctacag ttatgttgga    1680
tactaaatta taaaataaac ttctcgctcg tc                                  1712

<210>  14
<211>  410
<212>  PRT
<213>  Arabidopsis thaliana

<400>  14
Met Ala Ser Val Gly Thr Leu Pro Ala Ser Ser Met Ala Thr Lys Gln
1               5                   10                  15

Ser Asn Ala Ser Ile Cys Ala Glu Lys Leu Pro Glu Gly Ile Asn Glu
            20                  25                  30
Met Lys Ile Lys Asp Asp Lys Glu Met Glu Ala Ala Val Val Asp Gly
        35                  40                  45
Asn Gly Thr Glu Thr Gly His Ile Ile Val Thr Thr Ile Gly Gly Lys
    50                  55                  60
Asn Gly Gln Pro Lys Gln Thr Ile Ser Tyr Met Ala Glu Arg Ile Val
65                  70                  75                  80
Gly Gln Gly Ser Phe Gly Ile Val Phe Gln Ala Lys Cys Leu Glu Thr
                85                  90                  95
Gly Glu Thr Val Ala Ile Lys Lys Val Leu Gln Asp Lys Arg Tyr Lys
            100                 105                 110
Asn Arg Glu Leu Gln Thr Met Arg Leu Leu Asp His Pro Asn Val Val
        115                 120                 125
Ser Leu Lys His Cys Phe Phe Ser Thr Thr Glu Lys Asp Glu Leu Tyr
    130                 135                 140
Leu Asn Leu Val Leu Glu Tyr Val Pro Glu Thr Val Tyr Arg Val Ser
145                 150                 155                 160
Lys His Tyr Ser Arg Ala Asn Gln Arg Met Pro Ile Ile Tyr Val Lys
                165                 170                 175
Leu Tyr Thr Tyr Gln Ile Cys Arg Ala Leu Ala Tyr Ile His Gly Gly
            180                 185                 190
Val Gly Val Cys His Arg Asp Ile Lys Pro Gln Asn Leu Leu Val Asn
        195                 200                 205
Pro His Thr His Gln Val Lys Leu Cys Asp Phe Gly Ser Ala Lys Val
    210                 215                 220
Leu Val Lys Gly Glu Pro Asn Ile Ser Tyr Ile Cys Ser Arg Tyr Tyr
225                 230                 235                 240
Arg Ala Pro Glu Leu Ile Phe Gly Ala Thr Glu Tyr Thr Thr Thr Ile
                245                 250                 255
Asp Ile Trp Ser Ala Gly Cys Val Leu Ala Glu Leu Leu Leu Gly Gln
            260                 265                 270
Pro Leu Phe Pro Gly Glu Ser Gly Val Asp Gln Leu Val Glu Ile Ile
        275                 280                 285
Lys Val Leu Gly Thr Pro Thr Arg Glu Glu Ile Lys Cys Met Asn Pro
    290                 295                 300
Asn Tyr Thr Glu Phe Lys Phe Pro Gln Ile Lys Ala His Pro Trp His
305                 310                 315                 320
Lys Ile Phe His Lys Arg Thr Pro Pro Glu Ala Val Asp Leu Val Ser
                325                 330                 335
Arg Leu Leu Gln Tyr Ser Pro Asn Leu Arg Ser Thr Ala Met Glu Ala
            340                 345                 350
Ile Val His Pro Phe Phe Asp Glu Leu Arg Asp Pro Asn Thr Arg Leu
        355                 360                 365
Pro Asn Gly Arg Ala Leu Pro Pro Leu Phe Asn Phe Lys Pro Gln Glu
    370                 375                 380
Leu Lys Gly Ala Ser Leu Glu Leu Leu Ser Lys Leu Ile Pro Asp His
385                 390                 395                 400
Ala Arg Lys Gln Cys Ser Phe Leu Ala Leu
                405                 410

<210>  15
<211>  1230
<212>  DNA
<213>  Arabidopsis thaliana

<400>  15
atggcctcgg tgggcataga gcctagtgcc gcggttagag aatctactgg aaacgttact      60
gatgctgata gattacccga ggagatgaag gacatgaaaa ttcaagatga taaagaaatg     120

gaagctacga ttgttaatgg caatgtgact gagactggcc atataatagt aactactata    180
ggaggaagaa atggccagcc aaaacagaca atcagttaca tggcggagcg agttgttgga    240
catggctcct ttggtgttgt gtttcaggcc aaatgtttag aaacaggaga aactgttgct    300
ataaagaaag ttctacaaga tcggaggtac aagaatcgtg agcttcaaac aatgaggcta    360
cttgaccatc caaatgttgt gtctttgaaa cattgtttct tctctacaac cgaaaaagat    420
gagctttatc tcaacttggt tctggaatac gttccggaaa ctgtgcaccg cgtcatcaaa    480
cactacaaca aacttaacca acgaatgcct ctcgtttacg tcaaacttta cacttatcag    540
atttttaggt ccttatccta cattcaccga tgtatcggcg tatgtcatcg agacatcaaa    600
cctcaaaact tgttggtaaa tccacacact catcaagtga aactatgcga ttttggaagt    660
gcgaaagtat tggttaaagg agagccaaac atttcataca tttgctcgag gtattacaga    720
gcacctgagc tcatttttgg agccaccgag tatactacag ccattgatgt ctggtctgca    780
ggatgtgttc tcgccgagct tcttctcggg cagccattgt tcccgggtga gagcggtgtt    840
gatcaacttg tagagattat aaaggttttg ggaacaccaa caagggaaga aatcaaatgc    900
atgaacccga attacacaga gttcaaattt cctcagatta aagctcatcc atggcataag    960
attttccaca agagaatgcc tccagaagct gttgatttgg tctcaaggct tcttcaatac   1020
tctcccaatc tccgttgtgc tgctcttgat tcattggtcc acccattctt tgacgagcta   1080
agagatccga atgcgcgatt acccaacgga cgtttccttc caccgctctt taactttaag   1140
cctcatgaac ttaaaggtgt gcctgtggag atggtggcga agttagttcc agaacatgcg   1200
aggaagcaat gtccgtggct cagtttatga                                    1230

<210>  16
<211>  409
<212>  PRT
<213>  Arabidopsis thaliana

<400>  16
Met Ala Ser Val Gly Ile Glu Pro Ser Ala Ala Val Arg Glu Ser Thr
1               5                   10                  15
Gly Asn Val Thr Asp Ala Asp Arg Leu Pro Glu Glu Met Lys Asp Met
            20                  25                  30
Lys Ile Gln Asp Asp Lys Glu Met Glu Ala Thr Ile Val Asn Gly Asn
        35                  40                  45
Val Thr Glu Thr Gly His Ile Ile Val Thr Thr Ile Gly Gly Arg Asn
    50                  55                  60
Gly Gln Pro Lys Gln Thr Ile Ser Tyr Met Ala Glu Arg Val Val Gly
65                  70                  75                  80
His Gly Ser Phe Gly Val Val Phe Gln Ala Lys Cys Leu Glu Thr Gly
                85                  90                  95
Glu Thr Val Ala Ile Lys Lys Val Leu Gln Asp Arg Arg Tyr Lys Asn
            100                 105                 110
Arg Glu Leu Gln Thr Met Arg Leu Leu Asp His Pro Asn Val Val Ser
        115                 120                 125
Leu Lys His Cys Phe Phe Ser Thr Thr Glu Lys Asp Glu Leu Tyr Leu
    130                 135                 140
Asn Leu Val Leu Glu Tyr Val Pro Glu Thr Val His Arg Val Ile Lys
145                 150                 155                 160
His Tyr Asn Lys Leu Asn Gln Arg Met Pro Leu Val Tyr Val Lys Leu
                165                 170                 175
Tyr Thr Tyr Gln Ile Phe Arg Ser Leu Ser Tyr Ile His Arg Cys Ile
            180                 185                 190
Gly Val Cys His Arg Asp Ile Lys Pro Gln Asn Leu Leu Val Asn Pro
        195                 200                 205
His Thr His Gln Val Lys Leu Cys Asp Phe Gly Ser Ala Lys Val Leu
    210                 215                 220
Val Lys Gly Glu Pro Asn Ile Ser Tyr Ile Cys Ser Arg Tyr Tyr Arg
225                 230                 235                 240
Ala Pro Glu Leu Ile Phe Gly Ala Thr Glu Tyr Thr Thr Ala Ile Asp
                245                 250                 255
Val Trp Ser Ala Gly Cys Val Leu Ala Glu Leu Leu Leu Gly Gln Pro

                260                 265                 270
Leu Phe Pro Gly Glu Ser Gly Val Asp Gln Leu Val Glu Ile Ile Lys
            275                 280                 285
Val Leu Gly Thr Pro Thr Arg Glu Glu Ile Lys Cys Met Asn Pro Asn
    290                 295                 300
Tyr Thr Glu Phe Lys Phe Pro Gln Ile Lys Ala His Pro Trp His Lys
305                 310                 315                 320
Ile Phe His Lys Arg Met Pro Pro Glu Ala Val Asp Leu Val Ser Arg
                325                 330                 335
Leu Leu Gln Tyr Ser Pro Asn Leu Arg Cys Ala Ala Leu Asp Ser Leu
                340                 345                 350
Val His Pro Phe Phe Asp Glu Leu Arg Asp Pro Asn Ala Arg Leu Pro
            355                 360                 365
Asn Gly Arg Phe Leu Pro Pro Leu Phe Asn Phe Lys Pro His Glu Leu
    370                 375                 380
Lys Gly Val Pro Val Glu Met Val Ala Lys Leu Val Pro Glu His Ala
385                 390                 395                 400
Arg Lys Gln Cys Pro Trp Leu Ser Leu
                405

<210>  17
<211>  1967
<212>  DNA
<213>  Zea mays

<400>  17
ttgactgcag gcctcggcac ggtaggcaca aatcattttg gccgctgcgt cgcgttggga      60
gcagtattct actgtgcctg cgtcccgaca agcgaaaaga agagacgggg gagggtggga     120
gaacggatac cgaaaggggt aggagagtga gcagccagag aacaggaggg tagagaggag     180
gaggaggtag aaggcgaaag aaggggaacc aaatcttgga cgggaacaca tagatttctt     240
tggtggagga aggaggaggg caacaagagg aggttacagg tagcccaata gatctactgc     300
tgttgaggga gttgatgcaa agctgagttg ctgcgcgttg gctttcttta gagatggctt     360
cagctggtgt tgccccttct gggtacaaga acagcagcag caccagcatt ggtgccgaaa     420
agttgcaaga tcacatgaac gagctaaaga ttagagatga taaggaagtt gaagcaacca     480
taattaatgg gaaagggact gaaactgggc acataattgt caccactact ggtggcaaga     540
atggtcaacc aaaacagaca gtgagctaca tggctgagcg cattgtaggt caaggttctt     600
ttgggatcgt cttccaggcc aagtgtttgg aaacgggtga gactgttgcc ataaagaagg     660
ttcttcaaga caagcgttac aagaaccgcg aactgcagac catgcgcctt cttgaccacc     720
ctaatgttgt tgctttgaag cattgcttct tttcaactac tgagaaggat gagctttatc     780
tgaacttggt ccttgagtat gttccggaga cagttcatcg agttgtgaaa catcacaaca     840
agatgcacca acgcatgcca cttatttatg tgaagcttta tatgtaccag atatgtagag     900
cattggctta cattcatggt actatcggtg tctgccacag agatattaag ccacaaaatc     960
ttctggtgaa cccacacacc caccagctta aaatatgtga ctttggtagt gcaaaagttc    1020
tggtcaaggg ggaaccaaac atatcataca tctgctcgcg atactatagg gctccagagc    1080
tcatatttgg tgccactgag tataccacag cgattgacat ttggtctgct ggatgtgttc    1140
ttgctgagct tatgctaggg cagcctttgt ttccgggtga aagtggtgtg gaccaacttg    1200
ttgaaatcat caaggtcctc ggtacgccaa caagggaaga aattaaatgc atgaacccaa    1260
attacacaga gtttaagttc ccacaaatca aagcacaccc atggcacaag gtattccaca    1320
aaaggatgcc gccagaagct gttgatctgg tctctcggct actccagtac tccccaaatc    1380
tgagatgcac tgctatggag gcacttgttc acccattctt tgatgagctt cgagatccta    1440
atactcgcct tccaaatggt cgctttttgc caccactatt caatttcaag cctcacgaac    1500
ttaaaggagt cccatcagac attgttgcga aattggttcc agaacatgcg aagaagcaat    1560
gctcttatgt tggattgtga aatgaccgcg ccttgggact ggaacctgag gtcgcaatcg    1620
tgcatttccc ctgggatgtt ggatgatctt gaggcatgcg agcctgttgt tgaagatgca    1680
aggttacgta cttgtacgac aatgtgacct gtgtagctga gtagtctatg tcgcagtgac    1740
atgtaacggc accccccttc ctactaactg acgcttactc gagattgcca tagttgatct    1800
tgtaatttgt tatagagcag tatgaatgta tttatggtag cttgaatcta tgtatggatt    1860
cacttcgttt ttccatgttt ccttgtctcc agacccagat tgctaccgta ttgtttcaga    1920
attcctagct acctgttgcc taaaaaaaaa aaaaaaaaac ctcgtgc                  1967

<210>  18
<211>  408
<212>  PRT
<213>  Zea mays

<400>  18
Met Ala Ser Ala Gly Val Ala Pro Ser Gly Tyr Lys Asn Ser Ser Ser
1               5                   10                  15
Thr Ser Ile Gly Ala Glu Lys Leu Gln Asp His Met Asn Glu Leu Lys
            20                  25                  30
Ile Arg Asp Asp Lys Glu Val Glu Ala Thr Ile Ile Asn Gly Lys Gly
        35                  40                  45
Thr Glu Thr Gly His Ile Ile Val Thr Thr Thr Gly Gly Lys Asn Gly
    50                  55                  60
Gln Pro Lys Gln Thr Val Ser Tyr Met Ala Glu Arg Ile Val Gly Gln
65                  70                  75                  80
Gly Ser Phe Gly Ile Val Phe Gln Ala Lys Cys Leu Glu Thr Gly Glu
                85                  90                  95
Thr Val Ala Ile Lys Lys Val Leu Gln Asp Lys Arg Tyr Lys Asn Arg
            100                 105                 110
Glu Leu Gln Thr Met Arg Leu Leu Asp His Pro Asn Val Val Ala Leu
        115                 120                 125
Lys His Cys Phe Phe Ser Thr Thr Glu Lys Asp Glu Leu Tyr Leu Asn
    130                 135                 140
Leu Val Leu Glu Tyr Val Pro Glu Thr Val His Arg Val Val Lys His
145                 150                 155                 160
His Asn Lys Met His Gln Arg Met Pro Leu Ile Tyr Val Lys Leu Tyr
                165                 170                 175
Met Tyr Gln Ile Cys Arg Ala Leu Ala Tyr Ile His Gly Thr Ile Gly
            180                 185                 190
Val Cys His Arg Asp Ile Lys Pro Gln Asn Leu Leu Val Asn Pro His
        195                 200                 205
Thr His Gln Leu Lys Ile Cys Asp Phe Gly Ser Ala Lys Val Leu Val
    210                 215                 220
Lys Gly Glu Pro Asn Ile Ser Tyr Ile Cys Ser Arg Tyr Tyr Arg Ala
225                 230                 235                 240
Pro Glu Leu Ile Phe Gly Ala Thr Glu Tyr Thr Thr Ala Ile Asp Ile
                245                 250                 255
Trp Ser Ala Gly Cys Val Leu Ala Glu Leu Met Leu Gly Gln Pro Leu
            260                 265                 270
Phe Pro Gly Glu Ser Gly Val Asp Gln Leu Val Glu Ile Ile Lys Val
        275                 280                 285
Leu Gly Thr Pro Thr Arg Glu Glu Ile Lys Cys Met Asn Pro Asn Tyr
    290                 295                 300
Thr Glu Phe Lys Phe Pro Gln Ile Lys Ala His Pro Trp His Lys Val
305                 310                 315                 320
Phe His Lys Arg Met Pro Pro Glu Ala Val Asp Leu Val Ser Arg Leu
                325                 330                 335
Leu Gln Tyr Ser Pro Asn Leu Arg Cys Thr Ala Met Glu Ala Leu Val
            340                 345                 350
His Pro Phe Phe Asp Glu Leu Arg Asp Pro Asn Thr Arg Leu Pro Asn
        355                 360                 365
Gly Arg Phe Leu Pro Pro Leu Phe Asn Phe Lys Pro His Glu Leu Lys
    370                 375                 380
Gly Val Pro Ser Asp Ile Val Ala Lys Leu Val Pro Glu His Ala Lys
385                 390                 395                 400
Lys Gln Cys Ser Tyr Val Gly Leu
                405

<210>  19
<211>  1752
<212>  DNA
<213>  Zea mays

<400>  19
gcacgagccg cgccggagtt ggaggaggga gaggggacaa gctttccggc gccgacgccg      60
acgcggaccc ggcgccgaca cgatccggtg gatcaagtgc atcacacctt tagggaggcc     120
ccttggacag cagtttgtgc tgcaaattct atatagctct gtcgcagcat ggcctcggtg     180
ggcgtggcac gctcttcttt gggatttcag aatggcacaa gttctagcag tgacccagat     240
cgtcttccca acgagttggg cagtatgagc ataagggacg acaaggacgt tgaagatatt     300
gtagtcaatg gcaatggggc ggagcctggt catatcatag tgaccagcat tgatgggaga     360
aatgggcagg caaagcagac cattagttac atggctgagc gggtggtagg tcatgggtcc     420
ttcggaaccg ttttccaggc caagtgtctt gaaactggtg agaccgtagc tataaaaaag     480
gttcttcaag acaagagata caagaatcgt gagctgcaaa ccatgcgagt gcttgaccac     540
ccaaatgtgg tggctctaaa gcactgtttc ttctcaaaga ctgagaaaga ggagctttac     600
ctcaatttgg tgcttgagta tgtaccggag actgctcatc gtgtcatcaa acattacaac     660
aagatgaacc agcgcatgcc tttgatttat gcaaaactgt atatgtatca gatttgtaga     720
gccttggcat acattcacaa cagcattgga gtgtgccaca gggacattaa gccgcaaaat     780
ctcctggtta atcctcatac ccatcagcta aaattgtgtg actttggcag cgcgaaagtt     840
ctggtaaaag gcgaaccaaa catttcttac atctgttcta ggtactacag agctccagag     900
ctcatatttg gtgctactga atacacaaca gccattgatg tttggtctgc tggctgtgtg     960
ctcgctgagc tgcttctagg acagcctctg ttccctggag aaagcggtgt tgatcagctt    1020
gttgaaatca tcaaggttct gggcacaccc acacgtgaag aaattaagtg catgaatcca    1080
aattataccg agtttaaatt cccgcaaatc aaagctcacc catggcataa gatattccat    1140
aaaaggatgc ctgctgaagc ggtagatctc gtgtccaggc ttctgcagta ctcaccaaaa    1200
cttcggtcga ctgctttgga agcattggtc catccgttct ttgatgaact tcgggatcca    1260
aacacccgct taccgaatgg tcgttttctt ccgcctctct tcaattttaa gccccatgag    1320
ctgaagaacg tgccggcgga tttcatggtg aaattggtcc ctgagcatgc acggaagcaa    1380
tgtgccttcg tagggtggtg atctctggat aagaggatga cgactcgatg attagctgag    1440
gaccaagtta atgtctgtta gaaactgccg gagatcgaca ttgccagatg tggtgtggta    1500
taagataggc aatatgtgtg attatttttt gttcgaggtt atcacccccc ttgccccaga    1560
aaagatgaga agatgtcgat gtaacaagcc ctctgcgctt ctgtaagtag atgagtgttg    1620
ctgcatgccc cctgggtaca tgtatcggtt tgagcagaat tctgtttgcc tgaatcgtgc    1680
catcaccacg cagggatcca tcccttgtgt gacgatgttc agcccaaaaa aaaaaaaaaa    1740
aaaaaaaaaa aa                                                        1752

<210>  20
<211>  410
<212>  PRT
<213>  Zea mays

<400>  20
Met Ala Ser Val Gly Val Ala Arg Ser Ser Leu Gly Phe Gln Asn Gly
1               5                   10                  15
Thr Ser Ser Ser Ser Asp Pro Asp Arg Leu Pro Asn Glu Leu Gly Ser
            20                  25                  30
Met Ser Ile Arg Asp Asp Lys Asp Val Glu Asp Ile Val Val Asn Gly
        35                  40                  45
Asn Gly Ala Glu Pro Gly His Ile Ile Val Thr Ser Ile Asp Gly Arg
    50                  55                  60
Asn Gly Gln Ala Lys Gln Thr Ile Ser Tyr Met Ala Glu Arg Val Val
65                  70                  75                  80
Gly His Gly Ser Phe Gly Thr Val Phe Gln Ala Lys Cys Leu Glu Thr
                85                  90                  95
Gly Glu Thr Val Ala Ile Lys Lys Val Leu Gln Asp Lys Arg Tyr Lys
            100                 105                 110
Asn Arg Glu Leu Gln Thr Met Arg Val Leu Asp His Pro Asn Val Val

115                 120                 125
Ala Leu Lys His Cys Phe Phe Ser Lys Thr Glu Lys Glu Glu Leu Tyr
    130                 135                 140
Leu Asn Leu Val Leu Glu Tyr Val Pro Glu Thr Ala His Arg Val Ile
145                 150                 155                 160
Lys His Tyr Asn Lys Met Asn Gln Arg Met Pro Leu Ile Tyr Ala Lys
                165                 170                 175
Leu Tyr Met Tyr Gln Ile Cys Arg Ala Leu Ala Tyr Ile His Asn Ser
            180                 185                 190
Ile Gly Val Cys His Arg Asp Ile Lys Pro Gln Asn Leu Leu Val Asn
        195                 200                 205
Pro His Thr His Gln Leu Lys Leu Cys Asp Phe Gly Ser Ala Lys Val
    210                 215                 220
Leu Val Lys Gly Glu Pro Asn Ile Ser Tyr Ile Cys Ser Arg Tyr Tyr
225                 230                 235                 240
Arg Ala Pro Glu Leu Ile Phe Gly Ala Thr Glu Tyr Thr Thr Ala Ile
                245                 250                 255
Asp Val Trp Ser Ala Gly Cys Val Leu Ala Glu Leu Leu Leu Gly Gln
            260                 265                 270
Pro Leu Phe Pro Gly Glu Ser Gly Val Asp Gln Leu Val Glu Ile Ile
        275                 280                 285
Lys Val Leu Gly Thr Pro Thr Arg Glu Glu Ile Lys Cys Met Asn Pro
    290                 295                 300
Asn Tyr Thr Glu Phe Lys Phe Pro Gln Ile Lys Ala His Pro Trp His
305                 310                 315                 320
Lys Ile Phe His Lys Arg Met Pro Ala Glu Ala Val Asp Leu Val Ser
                325                 330                 335
Arg Leu Leu Gln Tyr Ser Pro Lys Leu Arg Ser Thr Ala Leu Glu Ala
            340                 345                 350
Leu Val His Pro Phe Phe Asp Glu Leu Arg Asp Pro Asn Thr Arg Leu
        355                 360                 365
Pro Asn Gly Arg Phe Leu Pro Pro Leu Phe Asn Phe Lys Pro His Glu
    370                 375                 380
Leu Lys Asn Val Pro Ala Asp Phe Met Val Lys Leu Val Pro Glu His
385                 390                 395                 400
Ala Arg Lys Gln Cys Ala Phe Val Gly Trp
                405                 410

<210>  21
<211>  1800
<212>  DNA
<213>  Medicago sativa

<400>  21
tttttttttt tttttttttt tcaaaatatt ttccatttgt ctcttctttc ttctttccta      60
attccgaatc ccacggattt cacttccatt caaagtcata gctagatcca atccattcca     120
ttcctctgtt tgagttgaag agttgatttg ggggttggat gttcagtttt gaagctgtgt     180
atgatctgaa aggaaataat taagtaaagt gttttgcact ttttttaaga agatatgatg     240
gcatcaggtg gtgttgcacc tgcttctgga tttatagata agaatgcaag ttcagttggt     300
gttgaaaagt tgcctgagga gatgaatgac atgaaaatta gggatgataa ggaaatggaa     360
gccgctacca ttgtagatgg aaatgggacc gaaaccggac atataattgt cacaaccatt     420
ggtggtaaaa atggccagcc aaagcagaca ataagttaca tggccgagcg tgttgttgga     480
catggatctt ttggtgtagt ttttcaggca aagtgtttgg agactggaga aactgtggct     540
ataaagaagg ttcttcaaga taagaggtac aagaaccggg aattgcaaac tatgcgcctt     600
ctggaccacc ctaatgttgt atctttgaag cactgcttct tctcaacgac tgaaaaggac     660
gagctttatc ttaacctggt gcttgaatat gttcctgaga ctgtcagccg tgtgattaga     720
cactacaaca aaatgaatca aagaatgcct atgatatatg tcaaacttta ttcttaccag     780
atttgcaggg cacttgctta tattcacaac agtattggag tatgtcacag ggatattaaa     840
cctcaaaatt tactggtcaa tcctcacacc caccaactga agatatgcga ctttggaagt     900

gctaaagtct tggtaaaagg tgaaccaaac atatcttaca tctgttctag gtactataga    960
gctcctgagc ttatattcgg tgcaactgag tacaccacag ccattgacat ctggtcagct   1020
ggttgcgtac ttggtgaact tttgcttggc cagccactgt ttcctggtga gagtggagta   1080
gaccaacttg tggaaattat caaggtttta ggcaccccaa caagggaaga aatcaagtgc   1140
atgaatccta attatacaga gtttaaattt cctcaaatca aagctcatcc atggcataag   1200
atttttcaca agagaatgcc tcctgaagct gtggatcttg tctcaagact attgcaatac   1260
tctccaaatc ttcgaagcac agctttggag gctttggttc atccattcta tgatgacgtg   1320
cgcgatccaa acactcggtt gccaaatggg cgtttccttc caccattatt taacttcaaa   1380
gtcaatgagc tcaagggagt acctgcagag atgctggtga aactggttcc acctcatgca   1440
agaaagcaat gtgccttgtt cgggtcatca tgaagcagcc ttgtgtagtt attaagtact   1500
ttcttctacc tatgtaaagg tgtatctagt caaatttcaa gtggttaaat aggttatttc   1560
tattttcttt ctgtttgatt tgttctcacc caacctacca tccaatttat tattattttt   1620
cttcctcgat gtagaagaaa gctgtgctgt ttaagaagca acttcagctt gattattact   1680
ttgtaggatc tgatgtgttc actcacctta acatgaacca ttgtttattg aagtgataga   1740
cttgatcaca acctcactga actagtagga gatgtttcaa atcttgaaaa aaaaaaaaaa   1800

<210>  22
<211>  412
<212>  PRT
<213>  Medicago sativa

<400>  22
Met Met Ala Ser Gly Gly Val Ala Pro Ala Ser Gly Phe Ile Asp Lys
1               5                   10                  15
Asn Ala Ser Ser Val Gly Val Glu Lys Leu Pro Glu Glu Met Asn Asp
            20                  25                  30
Met Lys Ile Arg Asp Asp Lys Glu Met Glu Ala Ala Thr Ile Val Asp
        35                  40                  45
Gly Asn Gly Thr Glu Thr Gly His Ile Ile Val Thr Thr Ile Gly Gly
    50                  55                  60
Lys Asn Gly Gln Pro Lys Gln Thr Ile Ser Tyr Met Ala Glu Arg Val
65                  70                  75                  80
Val Gly His Gly Ser Phe Gly Val Val Phe Gln Ala Lys Cys Leu Glu
                85                  90                  95
Thr Gly Glu Thr Val Ala Ile Lys Lys Val Leu Gln Asp Lys Arg Tyr
            100                 105                 110
Lys Asn Arg Glu Leu Gln Thr Met Arg Leu Leu Asp His Pro Asn Val
        115                 120                 125
Val Ser Leu Lys His Cys Phe Phe Ser Thr Thr Glu Lys Asp Glu Leu
    130                 135                 140
Tyr Leu Asn Leu Val Leu Glu Tyr Val Pro Glu Thr Val Ser Arg Val
145                 150                 155                 160
Ile Arg His Tyr Asn Lys Met Asn Gln Arg Met Pro Met Ile Tyr Val
                165                 170                 175
Lys Leu Tyr Ser Tyr Gln Ile Cys Arg Ala Leu Ala Tyr Ile His Asn
            180                 185                 190
Ser Ile Gly Val Cys His Arg Asp Ile Lys Pro Gln Asn Leu Leu Val
        195                 200                 205
Asn Pro His Thr His Gln Leu Lys Ile Cys Asp Phe Gly Ser Ala Lys
    210                 215                 220
Val Leu Val Lys Gly Glu Pro Asn Ile Ser Tyr Ile Cys Ser Arg Tyr
225                 230                 235                 240
Tyr Arg Ala Pro Glu Leu Ile Phe Gly Ala Thr Glu Tyr Thr Thr Ala
                245                 250                 255
Ile Asp Ile Trp Ser Ala Gly Cys Val Leu Gly Glu Leu Leu Leu Gly
            260                 265                 270
Gln Pro Leu Phe Pro Gly Glu Ser Gly Val Asp Gln Leu Val Glu Ile
        275                 280                 285
Ile Lys Val Leu Gly Thr Pro Thr Arg Glu Glu Ile Lys Cys Met Asn

290                 295                 300
Pro Asn Tyr Thr Glu Phe Lys Phe Pro Gln Ile Lys Ala His Pro Trp
305                 310                 315                 320
His Lys Ile Phe His Lys Arg Met Pro Pro Glu Ala Val Asp Leu Val
                325                 330                 335
Ser Arg Leu Leu Gln Tyr Ser Pro Asn Leu Arg Ser Thr Ala Leu Glu
            340                 345                 350
Ala Leu Val His Pro Phe Tyr Asp Asp Val Arg Asp Pro Asn Thr Arg
        355                 360                 365
Leu Pro Asn Gly Arg Phe Leu Pro Pro Leu Phe Asn Phe Lys Val Asn
    370                 375                 380
Glu Leu Lys Gly Val Pro Ala Glu Met Leu Val Lys Leu Val Pro Pro
385                 390                 395                 400
His Ala Arg Lys Gln Cys Ala Leu Phe Gly Ser Ser
                405                 410

<210>  23
<211>  1613
<212>  DNA
<213>  Medicago sativa

<400>  23
gagcacatgc tttggtttcg tttcgttggt ggatgagtgg ttaaaattga aagggtttca      60
gtggcaatgg cgtcggttgg tgttgcacca acttcaggtt ttagagaagt ccttggtgat     120
ggtgaaattg gtgttgatga tatattgcca gaggaaatga gtgatatgaa aattagggat     180
gatagagaaa tggaagccac cgttgttgac ggcaatggaa cggagacagg acatatcatt     240
gtcactacta ttggtggtag aaatggtcag ccaaagcaga ctataagcta tatggcagag     300
cgtgttgtag gacatggatc atttggagtt gtcttccagg ctaagtgctt ggaaactggt     360
gaaaccgtgg ctatcaaaaa ggttcttcaa gacaagaggt acaagaaccg ggaattgcaa     420
acaatgcgac tgcttgatca cccgaatgtc gtctctttaa agcattgttt cttttcaacc     480
accgaaaagg atgaactata cctgaatttg gtacttgagt atgttcctga aacagttcat     540
cgcgtgatta agcattacag caagttgaac caaaggatgc caatgattta tgtgaagctc     600
tatacatacc agatctttag agcattatct tatattcatc gttgcattgg agtctgtcat     660
cgggatatca agccccaaaa tctattggtc aatccacaca cccaccaggt taaattatgc     720
gactttggaa gtgcgaaagt cttggttaaa ggcgaaccaa atatatcgta tatatgttct     780
agatactaca gagcacccga gcttattttt ggagcaactg aatatactac tgctattgat     840
gtatggtctg ttggttgtgt tttggctgag ctgctgcttg gacagccatt gttcccaggt     900
gagagaggag ttgatcagct tgttgagatc atcaaggttc tgggaactcc gacaagagaa     960
gaaattaaat gcatgaatcc taattatacc gaatttaaat tccctcaaat caaagcacat    1020
ccatggcaca agatcttcca taagcgcatg cctgcagaag ctgttgattt ggtatcaaga    1080
ttattacaat actccccaaa cctgcggtgc caagctttag attgcttgac ccatcctttc    1140
ttcgatgagc ttcgtgaccc aaatgctcgc ttgccaactg gccgtttcct cccaccactg    1200
tttaacttca aacctcacga actgaaagga gttccagtcg agaccttgat gaaactggtt    1260
ccagagcatg cgaggaagca atgcccgttt cttggcttgt aatatgtcgt aaaatgtaac    1320
aaaactgcaa gtgttgtttc catatgaacg ttctatttga tgatatgata tttattagta    1380
tctttgttgt attcggttgc ctgtgataga aaatttagag atatatgcta cccaatatta    1440
cccaaaccct tatatgggta ttcagaatac ccttttcctg tatcacagca gattgtaaca    1500
tgcaatagaa gacaagtgtc tacaattatc taaatgttgt atcagtattt gtacttgtat    1560
ttgtatttgt ggagataatg acggattatt gcgtaaaaaa aaaaaaaaaa aaa           1613

<210>  24
<211>  411
<212>  PRT
<213>  Medicago sativa

<400>  24
Met Ala Ser Val Gly Val Ala Pro Thr Ser Gly Phe Arg Glu Val Leu
1               5                   10                  15
Gly Asp Gly Glu Ile Gly Val Asp Asp Ile Leu Pro Glu Glu Met Ser

            20                  25                  30
Asp Met Lys Ile Arg Asp Asp Arg Glu Met Glu Ala Thr Val Val Asp
        35                  40                  45
Gly Asn Gly Thr Glu Thr Gly His Ile Ile Val Thr Thr Ile Gly Gly
    50                  55                  60
Arg Asn Gly Gln Pro Lys Gln Thr Ile Ser Tyr Met Ala Glu Arg Val
65                  70                  75                  80
Val Gly His Gly Ser Phe Gly Val Val Phe Gln Ala Lys Cys Leu Glu
                85                  90                  95
Thr Gly Glu Thr Val Ala Ile Lys Lys Val Leu Gln Asp Lys Arg Tyr
            100                 105                 110
Lys Asn Arg Glu Leu Gln Thr Met Arg Leu Leu Asp His Pro Asn Val
        115                 120                 125
Val Ser Leu Lys His Cys Phe Phe Ser Thr Thr Glu Lys Asp Glu Leu
    130                 135                 140
Tyr Leu Asn Leu Val Leu Glu Tyr Val Pro Glu Thr Val His Arg Val
145                 150                 155                 160
Ile Lys His Tyr Ser Lys Leu Asn Gln Arg Met Pro Met Ile Tyr Val
                165                 170                 175
Lys Leu Tyr Thr Tyr Gln Ile Phe Arg Ala Leu Ser Tyr Ile His Arg
            180                 185                 190
Cys Ile Gly Val Cys His Arg Asp Ile Lys Pro Gln Asn Leu Leu Val
        195                 200                 205
Asn Pro His Thr His Gln Val Lys Leu Cys Asp Phe Gly Ser Ala Lys
    210                 215                 220
Val Leu Val Lys Gly Glu Pro Asn Ile Ser Tyr Ile Cys Ser Arg Tyr
225                 230                 235                 240
Tyr Arg Ala Pro Glu Leu Ile Phe Gly Ala Thr Glu Tyr Thr Thr Ala
                245                 250                 255
Ile Asp Val Trp Ser Val Gly Cys Val Leu Ala Glu Leu Leu Leu Gly
            260                 265                 270
Gln Pro Leu Phe Pro Gly Glu Arg Gly Val Asp Gln Leu Val Glu Ile
        275                 280                 285
Ile Lys Val Leu Gly Thr Pro Thr Arg Glu Glu Ile Lys Cys Met Asn
    290                 295                 300
Pro Asn Tyr Thr Glu Phe Lys Phe Pro Gln Ile Lys Ala His Pro Trp
305                 310                 315                 320
His Lys Ile Phe His Lys Arg Met Pro Ala Glu Ala Val Asp Leu Val
                325                 330                 335
Ser Arg Leu Leu Gln Tyr Ser Pro Asn Leu Arg Cys Gln Ala Leu Asp
            340                 345                 350
Cys Leu Thr His Pro Phe Phe Asp Glu Leu Arg Asp Pro Asn Ala Arg
        355                 360                 365
Leu Pro Thr Gly Arg Phe Leu Pro Pro Leu Phe Asn Phe Lys Pro His
    370                 375                 380
Glu Leu Lys Gly Val Pro Val Glu Thr Leu Met Lys Leu Val Pro Glu
385                 390                 395                 400
His Ala Arg Lys Gln Cys Pro Phe Leu Gly Leu
                405                 410

<210> 25
<211> 1543
<212> DNA
<213> Medicago sativa

<400> 25
gttcaattga agctgcaaaa aaaaaaaaaa tcttgatcta ctttggttgt gataatggca 60
acagcgggtg tagcacctgc ttctggaata gtagatgtaa atgcaagttc agctattgct 120
gttgataagt tacctgatga gattcttggc atgagaatta aggatgataa ggaaatggaa 180

gcacatgtgg tagatggaaa tagtactgaa gcaggacatg taattgtcac taccattggt    240
ggtaaaaatg gccagccaaa gcagacaata agctacatgg ctgagcgtgc tgttggacag    300
ggatcatttg gtgtagtttt ccaggctaag tgcttggaga caggtgaaac tgtggctata    360
aaaaaggttc ttcaagacaa gaggtataag aaccgggaat tgcaaacaat gcgccttctg    420
gaccacccca atgttgtaac tttgaagcat tgtttctttt caacaactga aaaagacgag    480
ctctatctta acttggtact tgagtttgtt cctgagactg tccatcgtgt gatcagacac    540
tacagcaaaa tgaatcagag gatgccattg atatacgtaa aactttattc ttaccagata    600
tgtagatcac tagcttatat tcataactgt gttggagtgt ctcataggga cataaaacct    660
caaaatttac tggtcaatcc tcacacccat cagctgaagc tgtgtgactt tgggagtgca    720
aaagtcttgg tcaagggtga accaaacata tcttacatct gttcgaggta ttatagagct    780
cctgagctta tatttggtgc aactgaatac acctcagcca ttgacatttg gtcagctggc    840
tgtgtgcttg gcgaactatt gcttggccag cctctctttc ccggtgcgag tggagtagac    900
cagctcgttg aaattatcaa ggttttaggt accccaacaa gggaagaaat aaagtgtatg    960
aatcctaatt acactgagtt caaattccca caaatcaaag ctcatccatg gcacaagatc   1020
tttcgcaagc gtatgccacc ggaagctgtg gatctcgtct caagactact tcaatactct   1080
ccaaatcttc gaagcacagc tttggaggct ctggttcatc ccttctttga tgaattgcgt   1140
gatccaaata cccgcttacc aaatggcgca catcttcctc ctttatttaa cttcaaagcc   1200
aacgagctta agggagtgcc tgctgaaatg ctggtgaagt tggttccgtc tcacgcaaga   1260
aagcagtgtt ctttgtttgc gtcgtcatag acttagaatg ctgtcttgtg taaatattat   1320
ggactccctg tttgtagaat tgtatgtagc ctgtttcatg ttgttaatag tctctctttt   1380
gttatttgtt ccattaattt gtttctacca aagaaaacca cctcagttta ttataagttt   1440
caagttgatc tctcttcacg atttaattgt tctcctgatc ttcaattatt aacagattgt   1500
attaactatt aactattaac tagaacaatt gtttattcta gcg                     1543

<210>  26
<211>  411
<212>  PRT
<213>  Medicago sativa

<400>  26
Met Ala Thr Ala Gly Val Ala Pro Ala Ser Gly Ile Val Asp Val Asn
1               5                   10                  15
Ala Ser Ser Ala Ile Ala Val Asp Lys Leu Pro Asp Glu Ile Leu Gly
            20                  25                  30
Met Arg Ile Lys Asp Asp Lys Glu Met Glu Ala His Val Val Asp Gly
        35                  40                  45
Asn Ser Thr Glu Ala Gly His Val Ile Val Thr Thr Ile Gly Gly Lys
    50                  55                  60
Asn Gly Gln Pro Lys Gln Thr Ile Ser Tyr Met Ala Glu Arg Ala Val
65                  70                  75                  80
Gly Gln Gly Ser Phe Gly Val Val Phe Gln Ala Lys Cys Leu Glu Thr
                85                  90                  95
Gly Glu Thr Val Ala Ile Lys Lys Val Leu Gln Asp Lys Arg Tyr Lys
            100                 105                 110
Asn Arg Glu Leu Gln Thr Met Arg Leu Leu Asp His Pro Asn Val Val
        115                 120                 125
Thr Leu Lys His Cys Phe Phe Ser Thr Thr Glu Lys Asp Glu Leu Tyr
    130                 135                 140
Leu Asn Leu Val Leu Glu Phe Val Pro Glu Thr Val His Arg Val Ile
145                 150                 155                 160
Arg His Tyr Ser Lys Met Asn Gln Arg Met Pro Leu Ile Tyr Val Lys
                165                 170                 175
Leu Tyr Ser Tyr Gln Ile Cys Arg Ser Leu Ala Tyr Ile His Asn Cys
            180                 185                 190
Val Gly Val Ser His Arg Asp Ile Lys Pro Gln Asn Leu Leu Val Asn
        195                 200                 205
Pro His Thr His Gln Leu Lys Leu Cys Asp Phe Gly Ser Ala Lys Val
    210                 215                 220
Leu Val Lys Gly Glu Pro Asn Ile Ser Tyr Ile Cys Ser Arg Tyr Tyr

225                 230                 235                 240
Arg Ala Pro Glu Leu Ile Phe Gly Ala Thr Glu Tyr Thr Ser Ala Ile
                245                 250                 255
Asp Ile Trp Ser Ala Gly Cys Val Leu Gly Glu Leu Leu Leu Gly Gln
            260                 265                 270
Pro Leu Phe Pro Gly Ala Ser Gly Val Asp Gln Leu Val Glu Ile Ile
        275                 280                 285
Lys Val Leu Gly Thr Pro Thr Arg Glu Glu Ile Lys Cys Met Asn Pro
    290                 295                 300
Asn Tyr Thr Glu Phe Lys Phe Pro Gln Ile Lys Ala His Pro Trp His
305                 310                 315                 320
Lys Ile Phe Arg Lys Arg Met Pro Pro Glu Ala Val Asp Leu Val Ser
                325                 330                 335
Arg Leu Leu Gln Tyr Ser Pro Asn Leu Arg Ser Thr Ala Leu Glu Ala
            340                 345                 350
Leu Val His Pro Phe Phe Asp Glu Leu Arg Asp Pro Asn Thr Arg Leu
        355                 360                 365
Pro Asn Gly Arg His Leu Pro Pro Leu Phe Asn Phe Lys Ala Asn Glu
    370                 375                 380
Leu Lys Gly Val Pro Ala Glu Met Leu Val Lys Leu Val Pro Ser His
385                 390                 395                 400
Ala Arg Lys Gln Cys Ser Leu Phe Ala Ser Ser
                405                 410

<210>  27
<211>  1724
<212>  DNA
<213>  Nicotiana tabacum

<400>  27
tctctctcat cttccctgcg attctctctc tcaatttcag gttttctctg tttctgtgcg      60
agcctctaat gatcggtgta ttctcaatat cctgaagatt ttctgatttc atcggtgaat     120
gactttttga ggtgataata gttcgcaaaa tttcaggaaa tgacttcagt aggcttagca     180
cctgtatccg gtttgagaga atccagtagc catagtgttg gtgtagatag gctgcctgag     240
gagatgaatg acatgagaat cagggatgat aaggaaatcg aagcagctat tgtggatggt     300
aatgggactg agacaggcca tataatagtg acaactattg gtggtagaca tggtcagcca     360
aaacagacta tcagttatat ggctgaacgt attgttggac aaggatcatt tggagtggtt     420
ttccaggcaa aatgcttaga gactggtgaa actgttgcta ttaaaaaggt tcttcaagac     480
aagagatata agaacaggga gctgcagacc atgcgtcttc ttgatcaccc aaatgttgtg     540
tgcctgaagc actgcttctt ttcaacaact gagaaggatg aagtatatct taatttggtt     600
cttgaatacg tccctgaaac tgtccatcgt gttattaaac actacaataa gttgaatcaa     660
aggatgccat tgatactagt gaagctttat acatatcaga ttttcagggc attgtcttac     720
atccatcaca caattggagt gtgccacagg gacataaagc ctcagaatct tttggtgaat     780
ccacatactc accaggttaa attgtgtgac tttggaagtg ctaaagttct ggttaaagga     840
gaaccaaata tttcttacat ctgctctagg tattatagag cgcctgaact tatatttgga     900
gcaacagagt acactaccgc tattgacatc tggtctgctg gctgtgttct agccgagcta     960
cttcttgggc agcctttgtt tccgggtgaa agtggagttg atcagcttgt tgagattatt    1020
aaggtcttgg gtactcctac cagggaagaa attaaatgca tgaatccaaa ttataacgag    1080
ttcaaattcc cccaaattaa agctcatccg tggcacaaga tatttcacaa gcgcatgcct    1140
ccagaagctg ttgatctggt ttcaagacta ctgcagtact cacctaactt gcgttgcact    1200
gctttagagg cagtgaccca tgccttcttc gatgagcttc gtgatcctaa tacacgcctc    1260
ccaaatggcc gcgtccttcc ccccttgttt aactttaagg cccatgagtt aaagggtgtg    1320
tctgcagaga atctattgaa gttggttccg gagcatgcca ggaaacagtg cccgtccctt    1380
ggtttatgag ttcccactgt acggtagata taatttaagt gtaagctatg ttatttctct    1440
gtatccattt ttcccccttt gctccccaca tgtaccagtt gtctctttgt attattatcc    1500
tagtttgtaa aagcagaggt aggatgtggt ctttaacatt ccttacctcc aacactttcc    1560
tttcaccctg ttccttttat gtcctactgt tgtaactttt atgtggttaa gggtggactg    1620
ctttctatat gaactattat tttatgatga aatttcaaag tgatttttag tgaaaaaaaa    1680
aaaaaaaaaa aaaaaaaaaa aaaaaaaaaa aaaaaaaaaa aaaa                     1724

<210>  28
<211>  409
<212>  PRT
<213>  Nicotiana tabacum

<400>  28
Met Thr Ser Val Gly Leu Ala Pro Val Ser Gly Leu Arg Glu Ser Ser
1               5                   10                  15
Ser His Ser Val Gly Val Asp Arg Leu Pro Glu Glu Met Asn Asp Met
            20                  25                  30
Arg Ile Arg Asp Asp Lys Glu Ile Glu Ala Ala Ile Val Asp Gly Asn
        35                  40                  45
Gly Thr Glu Thr Gly His Ile Ile Val Thr Thr Ile Gly Gly Arg His
    50                  55                  60
Gly Gln Pro Lys Gln Thr Ile Ser Tyr Met Ala Glu Arg Ile Val Gly
65                  70                  75                  80
Gln Gly Ser Phe Gly Val Val Phe Gln Ala Lys Cys Leu Glu Thr Gly
                85                  90                  95
Glu Thr Val Ala Ile Lys Lys Val Leu Gln Asp Lys Arg Tyr Lys Asn
            100                 105                 110
Arg Glu Leu Gln Thr Met Arg Leu Leu Asp His Pro Asn Val Val Cys
        115                 120                 125
Leu Lys His Cys Phe Phe Ser Thr Thr Glu Lys Asp Glu Val Tyr Leu
    130                 135                 140
Asn Leu Val Leu Glu Tyr Val Pro Glu Thr Val His Arg Val Ile Lys
145                 150                 155                 160
His Tyr Asn Lys Leu Asn Gln Arg Met Pro Leu Ile Leu Val Lys Leu
                165                 170                 175
Tyr Thr Tyr Gln Ile Phe Arg Ala Leu Ser Tyr Ile His His Thr Ile
            180                 185                 190
Gly Val Cys His Arg Asp Ile Lys Pro Gln Asn Leu Leu Val Asn Pro
        195                 200                 205
His Thr His Gln Val Lys Leu Cys Asp Phe Gly Ser Ala Lys Val Leu
    210                 215                 220
Val Lys Gly Glu Pro Asn Ile Ser Tyr Ile Cys Ser Arg Tyr Tyr Arg
225                 230                 235                 240
Ala Pro Glu Leu Ile Phe Gly Ala Thr Glu Tyr Thr Thr Ala Ile Asp
                245                 250                 255
Ile Trp Ser Ala Gly Cys Val Leu Ala Glu Leu Leu Leu Gly Gln Pro
            260                 265                 270
Leu Phe Pro Gly Glu Ser Gly Val Asp Gln Leu Val Glu Ile Ile Lys
        275                 280                 285
Val Leu Gly Thr Pro Thr Arg Glu Glu Ile Lys Cys Met Asn Pro Asn
    290                 295                 300
Tyr Asn Glu Phe Lys Phe Pro Gln Ile Lys Ala His Pro Trp His Lys
305                 310                 315                 320
Ile Phe His Lys Arg Met Pro Pro Glu Ala Val Asp Leu Val Ser Arg
                325                 330                 335
Leu Leu Gln Tyr Ser Pro Asn Leu Arg Cys Thr Ala Leu Glu Ala Val
            340                 345                 350
Thr His Ala Phe Phe Asp Glu Leu Arg Asp Pro Asn Thr Arg Leu Pro
        355                 360                 365
Asn Gly Arg Val Leu Pro Pro Leu Phe Asn Phe Lys Ala His Glu Leu
    370                 375                 380
Lys Gly Val Ser Ala Glu Asn Leu Leu Lys Leu Val Pro Glu His Ala
385                 390                 395                 400
Arg Lys Gln Cys Pro Ser Leu Gly Leu
                405

<210> 29
<211> 1573
<212> DNA
<213> Triticum aestivum

<400> 29
gttggtgtgg tgcgtccttc ctcgcgcttt cagaacgaca cgagtactag tggtgatgcc 60
gaccgacttc cgaacgagat gggcaatatg agcataaggg atgacaggga ccctgaggat 120
atagtagtca acggcaatgg gacggaacca ggccatatta tagtcacaag cattgaggga 180
agaaatgggc aagcaaaaca gaccattagc tacatggctg agcgtgtggt tggtaatggg 240
tcatttggaa ctgttttcca ggctaagtgt cttgaaactg gcgagacggt ggctataaag 300
aaggttcttc aagacaagag atataagaac cgtgagctgc aaacgatgcg agttcttgac 360
cacccaaatg ttgtggcttt aaagcattgt tttttctcaa agactgagaa agaggagctt 420
tacctcaacc tggtgcttga gtatgtgccg gagactgctc atcgtgtcat taagcattat 480
aacaagatga accaacgcat gccattgata tatgcaaaac tgtacatgta tcagatatgt 540
agatctttgg catacattca caacagcatt ggagtatgcc acagagacat caagcctcaa 600
aatcttctgg tgaatccaca tacgcaccaa ttgaaattat gtgacttcgg aagtgcgaaa 660
gtgttggtaa aaggagaacc aaatatttcc tatatctgtt caaggtacta tagagcccca 720
gagctcatat ttggtgctac tgaatacaca acggcaattg acgtttggtc tgctggctgt 780
gttcttgctg aactccttct aggacagcct atattccctg gcgacagtgg tgttgatcag 840
cttgttgaaa tcatcaaggt tttaggtacc cctacaagag aagaaattaa gtgcatgaat 900
ccaaattata cggagtttaa attcccacaa atcaaagctc acccatggca caagatcttc 960
cataaaagaa tgcctgctga agcagtagat cttgtctcca gactcttgca atattcacca 1020
agcctgcgtt caactgcttt ggaagcatta attcatccat tcttcgatga actccgggac 1080
ccaaacaccc gtttgccgaa cggccgtttt cttcctcccc tctttaactt taagccccat 1140
gagttgaagg gtgtgccgat ggacatcctg gtgaagctca tccctgaaca tgctcggaag 1200
aactgtgcct ttgtaggatg gtgatccgcc agacggctgc ttgaagttta gttcagaaca 1260
aatccagttg ttgtctacta gaaaccccag gagtttgaga ttgtctgcag ccacacggga 1320
tataggcgat gacacatgtg attattattc cttttctcgt ccgagacctc gatgccatgt 1380
attctttccc cctactgccg atgtaacaaa ccacccatga tactgtaagt agatgagaag 1440
tgtttcgacc gttttcccct gagctcatgt gctatgcaat gaaggatgca ccctatgtac 1500
cgccaatatt tggtccagta tttgttcatg gatcgaggcc cccaaaaaaa aaaaaaaaaa 1560
aaaaaaaaaa aaa 1573

<210> 30
<211> 381
<212> PRT
<213> Triticum aestivum

<400> 30
Met Gly Asn Met Ser Ile Arg Asp Asp Arg Asp Pro Glu Asp Ile Val
1               5                   10                  15
Val Asn Gly Asn Gly Thr Glu Pro Gly His Ile Ile Val Thr Ser Ile
            20                  25                  30
Glu Gly Arg Asn Gly Gln Ala Lys Gln Thr Ile Ser Tyr Met Ala Glu
        35                  40                  45
Arg Val Val Gly Asn Gly Ser Phe Gly Thr Val Phe Gln Ala Lys Cys
    50                  55                  60
Leu Glu Thr Gly Glu Thr Val Ala Ile Lys Lys Val Leu Gln Asp Lys
65                  70                  75                  80
Arg Tyr Lys Asn Arg Glu Leu Gln Thr Met Arg Val Leu Asp His Pro
                85                  90                  95
Asn Val Val Ala Leu Lys His Cys Phe Phe Ser Lys Thr Glu Lys Glu
            100                 105                 110
Glu Leu Tyr Leu Asn Leu Val Leu Glu Tyr Val Pro Glu Thr Ala His
        115                 120                 125
Arg Val Ile Lys His Tyr Asn Lys Met Asn Gln Arg Met Pro Leu Ile
    130                 135                 140

Tyr Ala Lys Leu Tyr Met Tyr Gln Ile Cys Arg Ser Leu Ala Tyr Ile
145                 150                 155                 160
His Asn Ser Ile Gly Val Cys His Arg Asp Ile Lys Pro Gln Asn Leu
                165                 170                 175
Leu Val Asn Pro His Thr His Gln Leu Lys Leu Cys Asp Phe Gly Ser
            180                 185                 190
Ala Lys Val Leu Val Lys Gly Glu Pro Asn Ile Ser Tyr Ile Cys Ser
        195                 200                 205
Arg Tyr Tyr Arg Ala Pro Glu Leu Ile Phe Gly Ala Thr Glu Tyr Thr
    210                 215                 220
Thr Ala Ile Asp Val Trp Ser Ala Gly Cys Val Leu Ala Glu Leu Leu
225                 230                 235                 240
Leu Gly Gln Pro Ile Phe Pro Gly Asp Ser Gly Val Asp Gln Leu Val
                245                 250                 255
Glu Ile Ile Lys Val Leu Gly Thr Pro Thr Arg Glu Glu Ile Lys Cys
            260                 265                 270
Met Asn Pro Asn Tyr Thr Glu Phe Lys Phe Pro Gln Ile Lys Ala His
        275                 280                 285
Pro Trp His Lys Ile Phe His Lys Arg Met Pro Ala Glu Ala Val Asp
    290                 295                 300
Leu Val Ser Arg Leu Leu Gln Tyr Ser Pro Ser Leu Arg Ser Thr Ala
305                 310                 315                 320
Leu Glu Ala Leu Ile His Pro Phe Phe Asp Glu Leu Arg Asp Pro Asn
                325                 330                 335
Thr Arg Leu Pro Asn Gly Arg Phe Leu Pro Pro Leu Phe Asn Phe Lys
            340                 345                 350
Pro His Glu Leu Lys Gly Val Pro Met Asp Ile Leu Val Lys Leu Ile
        355                 360                 365
Pro Glu His Ala Arg Lys Asn Cys Ala Phe Val Gly Trp
    370                 375                 380

<210>  31
<211>  1581
<212>  DNA
<213>  Petunia hybrida

<400>  31
tctctcaggt ttagggtttc gtattgtccg atcgctatac tttgaagctc tttccgatca      60
ctgttttgtg tttttgtaaa atgtgagctg ttctttagct acaggtttga agctgttaat     120
ctgtcatttg agttgtgccc attactcagt agtataagta agtttatttt tgttgctgat     180
atggcgtctg gtataatgcc ttcggctggt ggaaaacatc gaactgatgc catgcttgtt     240
gacaaacttc ccgaagaaat aaatgaaatg aagatcagag atgataaagc agaaaaggaa     300
atggaagcag ctgtagtgga tggaaatgga actgaaaaag gccacatcat cgtgacaact     360
attgggggca aaaatggtga gcctaagcag accattagtt acatggccga gcgtgttgtt     420
ggacagggtt cgtttggaat agtgttccag gccaaatgcc ttgaaactgg agaaactgtt     480
gcaataaaaa aggttttaca ggataagaga tacaagaatc gggaattgca aacaatacgc     540
cttctagatc atcctaatgt tgttgcactg aggcactgct tcttttcaac cacagaaaag     600
gatgagcttt atctgaattt ggtccttgaa tatgtaccag agactgtcta ccgtgtcttg     660
agacattaca gcaaagcaaa ccaacagatg cctatgattt atgtcaagct ctacacatat     720
cagattttca gagctttggc ctacatacac ggcataggag tctgccacag ggacatcaag     780
cctcagaatc tactggtcaa cccccacacc caccagctta agctctgcga ctttgggagt     840
gcaaaagttc tggtcaaagg cgaaccaaat atttcatata tttgttctcg ttactatcgt     900
gcacccgaac ttatattcgg agcaactgaa tacacttttg caattgacat ttggtctgtg     960
ggttgcgtcc ttgccgaact gcttctgggg cagcccctct ttcctggtga gagtggagtt    1020
gatcagcttg ttgaaataat caaggttctt ggaacaccaa ctcgggagga aatcaagagt    1080
atgaatccaa attacactga gttcaaattc ccacaaatca aagctcaccc ttggcacaaa    1140
atttttcata agcggatgcc tccagaagct gtggaccttg tgtcaaggct tctccaatat    1200
tctccaaatt tgaggtccac tgcgttggag gcttgcactc acactttctt tgatgaactc    1260
cgtgatccta agactcgcct ccctaatggt cggccattgc cacctctttt caacttcagg    1320

cctcaagagc tgaaaggagc gtcggcagac ctcttaaaca agctgatacc agaacatgct   1380
aagaagcagt gtacctttct tggtgtctag gtttatgaat gttgtgtatc tattgtatac   1440
ttgaaatatt ttgcaaccac tggttaattc tctactttgg ctaaacctgt ttgacatgtc   1500
ttcctttcca tgaaaactta taactgaaac cacattgtgt aagttgttcc tagtagtgca   1560
aatgctattt acttgaactt g                                              1581

<210>  32
<211>  409
<212>  PRT
<213>  Petunia hybrida

<400>  32
Met Ala Ser Gly Ile Met Pro Ser Ala Gly Gly Lys His Arg Thr Asp
1               5                   10                  15
Ala Met Leu Val Asp Lys Leu Pro Glu Glu Ile Asn Glu Met Lys Ile
            20                  25                  30
Arg Asp Asp Lys Ala Glu Lys Glu Met Glu Ala Ala Val Val Asp Gly
        35                  40                  45
Asn Gly Thr Glu Lys Gly His Ile Ile Val Thr Thr Ile Gly Gly Lys
    50                  55                  60
Asn Gly Glu Pro Lys Gln Thr Ile Ser Tyr Met Ala Glu Arg Val Val
65                  70                  75                  80
Gly Gln Gly Ser Phe Gly Ile Val Phe Gln Ala Lys Cys Leu Glu Thr
                85                  90                  95
Gly Glu Thr Val Ala Ile Lys Lys Val Leu Gln Asp Lys Arg Tyr Lys
            100                 105                 110
Asn Arg Glu Leu Gln Thr Ile Arg Leu Leu Asp His Pro Asn Val Val
        115                 120                 125
Ala Leu Arg His Cys Phe Phe Ser Thr Thr Glu Lys Asp Glu Leu Tyr
    130                 135                 140
Leu Asn Leu Val Leu Glu Tyr Val Pro Glu Thr Val Tyr Arg Val Leu
145                 150                 155                 160
Arg His Tyr Ser Lys Ala Asn Gln Gln Met Pro Met Ile Tyr Val Lys
                165                 170                 175
Leu Tyr Thr Tyr Gln Ile Phe Arg Ala Leu Ala Tyr Ile His Gly Ile
            180                 185                 190
Gly Val Cys His Arg Asp Ile Lys Pro Gln Asn Leu Leu Val Asn Pro
        195                 200                 205
His Thr His Gln Leu Lys Leu Cys Asp Phe Gly Ser Ala Lys Val Leu
    210                 215                 220
Val Lys Gly Glu Pro Asn Ile Ser Tyr Ile Cys Ser Arg Tyr Tyr Arg
225                 230                 235                 240
Ala Pro Glu Leu Ile Phe Gly Ala Thr Glu Tyr Thr Phe Ala Ile Asp
                245                 250                 255
Ile Trp Ser Val Gly Cys Val Leu Ala Glu Leu Leu Leu Gly Gln Pro
            260                 265                 270
Leu Phe Pro Gly Glu Ser Gly Val Asp Gln Leu Val Glu Ile Ile Lys
        275                 280                 285
Val Leu Gly Thr Pro Thr Arg Glu Glu Ile Lys Ser Met Asn Pro Asn
    290                 295                 300
Tyr Thr Glu Phe Lys Phe Pro Gln Ile Lys Ala His Pro Trp His Lys
305                 310                 315                 320
Ile Phe His Lys Arg Met Pro Pro Glu Ala Val Asp Leu Val Ser Arg
                325                 330                 335
Leu Leu Gln Tyr Ser Pro Asn Leu Arg Ser Thr Ala Leu Glu Ala Cys
            340                 345                 350
Thr His Thr Phe Phe Asp Glu Leu Arg Asp Pro Lys Thr Arg Leu Pro
        355                 360                 365
Asn Gly Arg Pro Leu Pro Pro Leu Phe Asn Phe Arg Pro Gln Glu Leu

370                 375                 380
Lys Gly Ala Ser Ala Asp Leu Leu Asn Lys Leu Ile Pro Glu His Ala
385                 390                 395                 400
Lys Lys Gln Cys Thr Phe Leu Gly Val
                405

<210>  33
<211>  2193
<212>  DNA
<213>  Oryza sativa

<400>  33
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag atttttttta aaaaaataga     360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc ttttttcgat tagatgcaag     540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctcctcctc ccatctataa attcctcccc ccttttcccc tctctatata    1020
ggaggcatcc aagccaagaa gagggagagc accaaggaca cgcgactagc agaagccgag    1080
cgaccgcctt cttcgatcca tatcttccgg tcgagttctt ggtcgatctc ttccctcctc    1140
cacctcctcc tcacagggta tgtgcccttc ggttgttctt ggatttattg ttctaggttg    1200
tgtagtacgg gcgttgatgt taggaaaggg gatctgtatc tgtgatgatt cctgttcttg    1260
gatttgggat agaggggttc ttgatgttgc atgttatcgg ttcggtttga ttagtagtat    1320
ggttttcaat cgtctggaga gctctatgga atgaaatggg tttagggtac ggaatcttgc    1380
gattttgtga gtaccttttg tttgaggtaa aatcagagca ccggtgattt tgcttggtgt    1440
aataaaagta cggttgtttg gtcctcgatt ctggtagtga tgcttctcga tttgacgaag    1500
ctatcctttg tttattccct attgaacaaa aataatccaa ctttgaagac ggtcccgttg    1560
atgagattga atgattgatt cttaagcctg tccaaaattt cgcagctggc ttgtttagat    1620
acagtagtcc ccatcacgaa attcatggaa acagttataa tcctcaggaa caggggattc    1680
cctgttcttc cgatttgctt tagtcccaga attttttttc ccaaatatct taaaaagtca    1740
ctttctggtt cagttcaatg aattgattgc tacaaataat gcttttatag cgttatccta    1800
gctgtagttc agttaatagg taatacccct atagtttagt caggagaaga acttatccga    1860
tttctgatct ccatttttaa ttatatgaaa tgaactgtag cataagcagt attcatttgg    1920
attatttttt ttattagctc tcaccccttc attattctga gctgaaagtc tggcatgaac    1980
tgtcctcaat tttgttttca aattcacatc gattatctat gcattatcct cttgtatcta    2040
cctgtagaag tttctttttg gttattcctt gactgcttga ttacagaaag aaatttatga    2100
agctgtaatc gggatagtta tactgcttgt tcttatgatt catttccttt gtgcagttct    2160
tggtgtagct tgccactttc accagcaaag ttc                                 2193

<210>  34
<211>  54
<212>  DNA
<213>  Artificial sequence

<220>
<223>  primer: prm5797

<400>  34

ggggacaagt ttgtacaaaa aagcaggctt aaacaatggg ttcagtaggg gttg          54

<210>  35
<211>  50
<212>  DNA
<213>  Artificial sequence

<220>
<223>  primer: prm5798

<400>  35
ggggaccact ttgtacaaga aagctgggtg aagctgtctc atactcctgc               50

<210>  36
<211>  1243
<212>  DNA
<213>  Oryza sativa

<400>  36
aaaaccaccg agggacctga tctgcaccgg ttttgatagt tgagggaccc gttgtgtctg     60
gttttccgat cgagggacga aaatcggatt cggtgtaaag ttaagggacc tcagatgaac    120
ttattccgga gcatgattgg gaagggagga cataaggccc atgtcgcatg tgtttggacg    180
gtccagatct ccagatcact cagcaggatc ggccgcgttc gcgtagcacc cgcggtttga    240
ttcggcttcc cgcaaggcgg cggccggtgg ccgtgccgcc gtagcttccg ccggaagcga    300
gcacgccgcc gccgccgacc cggctctgcg tttgcaccgc cttgcacgcg atacatcggg    360
atagatagct actactctct ccgtttcaca atgtaaatca ttctactatt ttccacattc    420
atattgatgt taatgaatat agacatatat atctatttag attcattaac atcaatatga    480
atgtaggaaa tgctagaatg acttacattg tgaattgtga aatggacgaa gtacctacga    540
tggatggatg caggatcatg aaagaattaa tgcaagatcg tatctgccgc atgcaaaatc    600
ttactaattg cgctgcatat atgcatgaca gcctgcatgc gggcgtgtaa gcgtgttcat    660
ccattaggaa gtaaccttgt cattacttat accagtacta catactatat agtattgatt    720
tcatgagcaa atctacaaaa ctggaaagca ataaggaata cgggactgga aaagactcaa    780
cattaatcac caaatatttc gccttctcca gcagaatata tatctctcca tcttgatcac    840
tgtacacact gacagtgtac gcataaacgc agcagccagc ttaactgtcg tctcaccgtc    900
gcacactggc cttccatctc aggctagctt tctcagccac ccatcgtaca tgtcaactcg    960
gcgcgcgcac aggcacaaat tacgtacaaa acgcatgacc aaatcaaaac caccggagaa   1020
gaatcgctcc cgcgcgcggc ggcggcgcgc acgtacgaat gcacgcacgc acgcccaacc   1080
ccacgacacg atcgcgcgcg acgccggcga caccggccat ccacccgcgc cctcacctcg   1140
ccgactataa atacgtaggc atctgcttga tcttgtcatc catctcacca ccaaaaaaaa   1200
aggaaaaaaa aacaaaacac accaagccaa ataaaagcga caa                     1243

<210>  37
<211>  5
<212>  PRT
<213>  Artificial sequence

<220>
<223>  motif I

<220>
<221>  VARIANT
<222>  (1)..(1)
<223>  / replace = "His" / replace = "Val" / replace = "Asn" / replace =
       "Gln

<220>
<221>  VARIANT
<222>  (2)..(2)
<223>  / replace = "Gly"

<220>
<221>  VARIANT
<222>  (5)..(5)
<223>  / replace = "Asn"

<400>  37
Arg Glu Leu Lys Gly
1               5

<210>  38
<211>  7
<212>  PRT
<213>  Artificial equence

<220>
<223>  motif II

<220>
<221>  VARIANT
<222>  (2)..(2)
<223>  / replace = "Asn"

<220>
<221>  UNSURE
<222>  (4)..(6)
<223>  Xaa can be any naturally occurring amino acid

<220>
<221>  VARIANT
<222>  (7)..(7)
<223>  / replace = "Ala" / replace = "Ser"

<400>  38
Lys Gln Cys Xaa Xaa Xaa Gly
1               5